# EUROPEAN PATENT APPLICATION

(11) **EP 3 994 978 A1**
(43) Date of publication of application: **11.05.2022**
(21) Application number: 21200639.9
(22) Date of filing: 22.05.2015
(51) Int. Cl.: A01H 3/00

(54) **INTEGRATED PLANT BREEDING METHODS FOR COMPLEMENTARY PAIRINGS OF PLANTS AND MICROBIAL CONSORTIA**

(30) Priority: 23.05.2014 US 201462002646 P; 20.08.2014 US 201462039634 P
(62) Divisional of application: 15795842.2
(71) Applicant: BioConsortia, Inc., Davis, CA 95618 (US)
(72) Inventor: MEADOWS-SMITH, Marcus, Davis, 95618 (US); WIGLEY, Peter, Auckland 1052 (NZ); TURNER, Susan, Davis, 95618 (US)
(74) Representative: HGF

(57) **Abstract**

The disclosure relates to improving plant breeding methods by controlling for microbial diversity present in the plant breeding process. The present disclosure addresses a great need in the art, by providing for improved plant breeding methods that do not suffer from many of the drawbacks inherent with current methodologies. For instance, the methods of the present disclosure are able to capture and harness a previously untapped resource, the microbiome, and utilize such to improve traditional plant breeding methods. That is, the methods taught herein are able to control for, and beneficially harness, the microbial communities present in the plant breeding process.

## Description

### CROSS REFERENCE TO RELATED APPLICATIONS

The present Application is a PCT international filing claiming the benefit of priority to U.S. Provisional Application No. 62/039,634, filed on August 20, 2014, and U.S. Provisional Application No. 62/002,646, filed on May 23, 2014, each of which is hereby incorporated by reference in its entirety for all purposes.

### FIELD

The present disclosure teaches improved methods of plant breeding. The methods of plant breeding taught herein account for, and control, microbial variability in a plant breeding scheme.

In particular aspects, the present disclosure provides for methods of developing microbial consortia through directed evolution and accelerated microbial selection. The microbial consortia developed by the methods of the present disclosure are capable of producing desirable plant phenotypic responses in conjunction with plant breeding efforts.

### BACKGROUND

Known processes of imparting beneficial properties to plants, such as selective breeding schemes, suffer from a number of drawbacks. For example, traditional selective breeding methodologies can be: extremely costly, slow, and limited in scope.

Furthermore, traditional selective breeding approaches have not been able to account for the substantial amount of heterogeneity witnessed from within similar replicated lines during the plant breeding process. That is, during traditional selective plant breeding programs, breeders are well aware of the tremendous amount of intra line variability in plant health and growth response. This variability is often attributed to the microenvironment, thus preventing breeders from effectively harnessing the cause of the increased plant vigor witnessed from these experiments, and passing such traits on to subsequent lines.

Despite the past few decades witnessing an explosion in the area of creating successful and highly-productive transgenic crops, there has been relatively little research devoted to substantially improving the effectiveness of traditional plant breeding methodologies. To date, plant breeders are still unable to control, or harness, the tremendous amount of environmental variability associated with traditional plant breeding programs.

This inability to control or harness environmental variability represents a tremendous lost opportunity for plant breeders to capture the heterogeneous plant vigor that is witnessed in many breeding programs.

Thus, there is a great need in the art for the development of improved plant breeding methodologies that do not suffer from the drawbacks exhibited by present plant breeding methods.

### SUMMARY OF THE DISCLOSURE

The present disclosure addresses a great need in the art, by providing for improved plant breeding methods that do not suffer from many of the drawbacks inherent with current methodologies. For instance, the methods of the present disclosure are able to capture and harness a previously untapped resource, the microbiome, and utilize such to improve traditional plant breeding methods. That is, the methods taught herein are able to control for, and beneficially harness, the microbial communities present in the plant breeding process.

New DNA-sequencing techniques, similar to those used to analyze the human gut microbiome, are being used to analyze entire microbiomes of major crops. We liken these crop microbiomes to 'second genomes' that interact with the crop genome, and like the microbes in the gut, can result in benign or beneficial, to even harmful effects. One goal of the present disclosure is to identify and harness beneficial interactions, and utilize genetic techniques to identify microbially-mediated plant genes associated with specific crop traits, providing a way to merge crop genetic technologies with beneficial plant-microbial genetic variability.

By incorporating the microbiome into a traditional plant breeding scheme, the present disclosure provides for improved plant breeding methods that are faster and more robust than traditional plant breeding methods.

For instance, past plant breeding schemes would ascribe the tremendous amount of heterogeneity witnessed amongst plants of a crossed line to merely microenvironment variability. However, according to the methods taught herein, the microbial environment associated with a plant that exhibits increased vigor from within a crossed line can now be harnessed and utilized to cultivate plants of a subsequent cross, thereby capturing an important component of the microenvironment and utilizing such to impart the increased plant vigor witnessed in the parent plant onto its progeny.

Further, embodiments of the present disclosure are able to reduce the environmental heterogeneity that is present in the methods currently practiced by plant breeders.

For instance, in embodiments of the present disclosure, a uniform microbial consortium or community of microbes is provided, within which the plants of the breeding scheme are grown. Thus, the plants are exposed to a controlled microbial community, which allows the breeder to effectively control for an environmental variable that has heretofore not been addressed by traditional plant breeding methods.

Also provided herein, are methods of breeding plants with better phenotypes by utilizing the collective genotypes of the plant and its symbiotic microflora. The present disclosure refers to this unifying concept of a plant's genes and the genes of the microflora inhabiting the plant (e.g. endophytes) and its environment (e.g. microbes inhabiting the growth medium of the plant) as the "holobiome."

Methods of the present disclosure are therefore effective at controlling, and accounting for, the plant-associated microbial component of environmental variability, which has previously gone unaddressed in the plant breeding community.

In some aspects, the microbial community utilized to control for the microbial diversity present in a plant breeding environment is derived from an accelerated microbial selection process, which will be elaborated upon below.

However, in other embodiments, the microbial community utilized to control for the microbial diversity present in a plant breeding environment is not derived from an accelerated microbial selection process.

Therefore, in certain aspects, the particular source of the microbes utilized in a plant breeding method is not of paramount importance. Rather, in these aspects, the fact that the microbial community is identified and controlled for throughout the plant breeding program is the consideration of importance.

In some aspects, a microbial community utilized in embodiments of the disclosure is chosen from amongst members of microbes present in a database. In particular aspects, the microbial community utilized in embodiments of the disclosure is chosen from microbes present in a database based upon particular characteristics of said microbes. In other embodiments, the particular characteristics of the chosen microbes in not known. In still further aspects, the specific taxonomic identity of the utilized microbes is not known. In some aspects, commercially available microbes are utilized in the taught plant breeding schemes. Regardless of the microbes' source, an underlying feature of many of the methods taught herein is the ability to account for and control the microbiome associated with plants undergoing a breeding process.

According to the present disclosure, the relevant environments include the soil microbiome which can vary both on the macroscale and microscale. By controlling the plant-associated microbial component of environmental variability, such that a co-selected microbial consortium is optimized by accelerated microbial selection (AMS), new products tailored for specific crop cultivars can be produced. Thus, according to the present disclosure, the genotype of the plants and the genotype of the symbiont microflora *(i.e.,* the holobiome) are viewed as a collective genotype or genotypic system.

According to the present disclosure, microbial consortium are selected to be optimized by AMS as the source of new products tailored to specific crop cultivars, including such specific crop cultivars developed for specific cropping systems and crop environments.

In one embodiment of the present disclosure, the disclosed compositions and methods provide a uniform background microbiome derived from an initial set of AMS-identified microbes (using plant parental lines) for each breeding cycle. According to one embodiment of the present disclosure, the use of AMS-derived microbes aids in the reduction of "noise" in plant phenotypic expression due to a variable microbial background.

In other embodiments, the uniform background microbiome is not derived from an AMS procedure.

In some aspects, the uniform background microbiome is preselected based upon choosing microbes from a database, e.g. an annotated microbial database.

In some aspects, the uniform background microbiome is not preselected from a database, but rather is randomly or haphazardly chosen from any given source (e.g. soil in a location of interest).

In one embodiment of the present disclosure, the microbiome is supplied in the form of seed coatings for each of the initial plant populations and each of the subsequent selections.

In one embodiment of the present disclosure, the microbiome is supplied in the form of granules, or plug, or soil drench that is applied to the plant growth media. In other embodiments, the microbiome is supplied in the form of a foliar application, such as a foliar spray or liquid composition. The foliar spray or liquid application may be applied to a growing plant or to a growth media, e.g. soil.

In some embodiments, the compositions of the disclosure are administered to a plant or growth media as a topical application and/or drench application to improve crop growth, yield, and quality.

In embodiments, the compositions of the disclosure can be formulated as: (1) solutions; (2) wettable powders; (3) dusting powders; (4) soluble powders; (5) emulsions or suspension concentrates; (6) seed dressings, (7) tablets; (8) water-dispersible granules; (9) water soluble granules (slow or fast release); (10) microencapsulated granules or suspensions; and (11) as irrigation components, among others. In certain aspects, the compositions may be diluted in an aqueous medium prior to conventional spray application. The compositions of the present disclosure can be applied to the soil, plant, seed, rhizosphere, rhizosheath, or other area to which it would be beneficial to apply the microbial compositions. Further still, ballistic methods can be utilized as a means for introducing endophytic microbes.

In aspects, the compositions are applied to the foliage of plants. The compositions may be applied to the foliage of plants in the form of an emulsion or suspension concentrate, liquid solution, or foliar spray. The application of the compositions may occur in a laboratory, growth chamber, greenhouse, or in the field. The application of the compositions may occur via a spray, or via a direct inoculation of microbes onto the developing seed, thereby facilitating vertical transmission of epiphytes and/or endophytes. The application process could be undertaken during cross-pollination, thereby introducing the microbes as a component of the cross. Notably, the application of microbes could occur as a co-inoculation with pollen. Pollen is often the vehicle of pathogen transmission. The ability to modify the pollen microbiome, e.g. by including microbes that compete with or inhibit pollen-associated pathogens, could reduce disease transmission. It is also possible that microbial symbionts present in pollen contribute to early embryonic development and therefore could influence productivity.

In one embodiment of the present disclosure, the microbiomes from the best-performing plants selected in each step of a given breeding cycle are used as the source microbiomes for the next plant selection round. According to this embodiment, line breeding can be conducted using the microbiome alongside the plants. In this context, the microbiome may be endophytic, epiphytic or rhizospheric microbes.

In one embodiment of the present disclosure, the AMS process is conducted on each step of the plant breeding process using the best-performing plants as parental material for the next breeding cycle. According to this embodiment, AMS is utilized to identify and select consortia for "priming" plant phenotypic expression prior to selecting the plants.

In one embodiment of the present disclosure, the different processes described herein are combined in various ways, methods and systems. For example, coating seed in an initial plant population (e.g., about 100,000 or more segregating plants) with an AMS-derived uniform background microbial consortium is used to reduce natural microbial variability. Other embodiments do not utilize AMS-derived microbial consortia; but rather utilize: (1) microbial consortia selected *a priori* from a database based upon known characteristics of the microbes, or (2) selected haphazardly or randomly from areas of interest, in which the characteristics of the microbes comprising the consortia are not known, or (3) commercially available microbes or microbial products.

As a further example, when the breeding work has reduced the lines best expressing the desired genetic trait to about 20 or so different plant genotypes, further AMS is conducted on the pooled root/stem microbial consortia. For example, the best 2 or 3 plant lines are chosen on the basis of best plant lines with the best background microbes.

In another embodiment, the present disclosure further provides the plant/microbe kits or systems selected by such processes. Thus, according to the present disclosure, a preselected combination of a plant genotype is paired with a microbial consortium as a kit, system or product to be delivered to an agricultural production system, such as an agronomical, forestry or horticultural production operation.

In another embodiment of the present disclosure, AMS or components of the AMS process (e.g., microbe capture) are used to identify and select diverse microbial consortia that replicate and/or simulate the effects of field variability, such as for pre-field screening of hybrid performance.

In another embodiment, the present disclosure includes "bottom-up breeding" *(i.e.,* breeding the microbiome, then the plant). For example, the AMS process is used on the parental plant material to optimize the microbiome so as to generate the best possible plant; and, then the plant breeding program is initiated to improve the plant using the selected microbiome. This strategy can also be used to select for plants that tolerate extreme environments, such as salty soils, acidic soils, dry, soils, etc. While not wishing to be bound by a specific theory, these types of extreme environments will have a microbial flora that is vastly different from that found in normal plant growth media used in plant breeding. Therefore, according to one embodiment of the present disclosure, the breeding process is initiated by ensuring that the plants have a microflora that reflects their ultimate growing environment.

In some embodiments of the present disclosure, any of the approaches disclosed herein can be combined with hydroponic or other soil-free systems for manipulating the microbiome.

In certain embodiments, the disclosure provides a method for controlling the microbial variability associated with selective plant breeding, comprising:
a) subjecting one or more plants to a growth medium in the presence of a first set of one or more microorganisms;
b) selecting one or more plants and/or growth medium following step a);
c) acquiring a second set of one or more microorganisms from said one or more plants and/or growth medium selected in step b);
d) repeating steps a) to c) one or more times, wherein the second set of one or more microorganisms acquired in step c) is used as the first set of microorganisms in step a) of any successive repeat;
e) selecting one or more microorganisms that is associated with imparting a beneficial property to a plant; and
f) providing the selected one or more microorganisms to a plant undergoing a selective plant breeding program or a growth medium used to grow said plant during the selective plant breeding program.

However, in some embodiments, a plant breeding program is carried out as is standard in the art, with the additional step of identifying and controlling for the microbial community present in the growth medium of the plants throughout the duration of the breeding program. The microbial community can be controlled for by utilizing microbes derived from the AMS process, commercial microbes, randomly collected microbes, or any other source of microbes. In these embodiments, a key feature of the improved plant breeding methods taught herein is the fact that the microbial community associated with the plants undergoing the breeding program is accounted for and controlled. The microbiome associated with a plant breeding program may be found on the plants undergoing the breeding process (or a plant part, e.g. root), the container containing the plants, or growth medium (e.g. soil) containing the plants. The microbiome associated with these areas can be identified and controlled during the breeding operation. That is, the microbiome of the surface of the plant, as well as the microbiome on the growth media can be controlled.

In some aspects, the selected one or more microorganisms is provided as a seed coating to said plant undergoing a selective plant breeding program.

In an aspect, the selected one or more microorganisms is provided in the form of a granule, plug, or liquid drench.

In particular embodiments, the one or more microorganisms is provided to the growth medium used to grow said plant undergoing a selective plant breeding program and wherein said provided one or more microorganisms account for approximately: 0.01% to 0.10%, or 0.10% to 0.25%, or 0.25% to 0.50%, or 0.50% to 1%, or 1% to 10%, or greater of the total microbial diversity present in said growth medium.

In some embodiments, the one or more microorganisms is provided to the growth medium used to grow said plant undergoing a selective plant breeding program and wherein said provided one or more microorganisms account for approximately: 1% to 99%, or 5% to 99%, or 10% to 99%, or 20% to 99%, or 30% to 99%, or 40% to 99%, or 50% to 99%, or 60% to 99%, or 70% to 99%, or 80% to 99%, or 90% to 99%, or 1%, or 5%, or 10%, or 20%, or 30%, or 40%, or 50%, or 60%, or 70%, or 80%, or 90%, or 99%, or greater of the total microbial diversity present in said growth medium.

In some embodiments, the one or more microorganisms is provided as a seed treatment to a seed placed into a non-sterilized growth medium (e.g. soil) that already contains an endogenous and heterogeneous microbial population. In these instances, the microbial diversity accounted for by the introduced one or more microorganisms may be small in relation to the total microbial diversity present in the growth medium. However, in these embodiments, the one or more microorganisms have a relatively large effect on the treated seeds environment, as the introduced microbes are spatially close to the seed and will be the first microbial elements encountered in the growth of the seed.

Consequently, the mere fact that the introduced one or more microorganisms of the disclosure may not comprise a large percentage of the total microbial population found in a growth medium does not negate the fact that the introduced one or more microorganisms may have a disproportionate influence on the growth characteristics of the plant. By being incorporated as a seed treatment and consequently forming a microbial population that is spatially the first microbial elements encountered by the growing seedling, the compositions of the disclosure are able to reduce the microbial variability present on a microscale relative to the growing seedlings environment.

The reduction of microbial microscale variability, relative to a growing seed, may be accomplished by utilizing the compositions of the disclosure as a seed treatment. Alternatively, the same result can be achieved by a direct application of the composition next to a growing seed after the seed has been planted. For example, if a seed is being grown in a laboratory setting in a small container (e.g. a petri dish with growth medium) then the compositions of the disclosure can be directly injected next to the seed.

In other embodiments, the one or more microorganisms is provided to the growth medium used to grow said plant undergoing a selective plant breeding program and wherein said provided one or more microorganisms account for approximately 95% or greater of the total microbial diversity present in said growth medium and wherein said microbial diversity present in the growth medium is maintained from an F1 generation through each successive selective generation.

In particular embodiments, the one or more microorganisms is provided to the growth medium used to grow said plant undergoing a selective plant breeding program and wherein said provided one or more microorganisms account for approximately 95% or greater of the total microbial diversity present in said growth medium and wherein said microbial diversity present in the growth medium is maintained from an F1 generation through each successive selective generation such that upon reaching at least an F4 generation the microbial diversity in said plant growth medium is at least 90% similar to the microbial diversity found in the growth medium of the F1 generation.

Aspects of the disclosure include the aforementioned method that further comprises:
g) selecting a plant based upon a desired phenotypic or genotypic trait during the course of the selective plant breeding program and simultaneously collecting the microorganisms associated with said plant or plant growth medium.

Aspects of the disclosure include the aforementioned method that further comprises:
g) selecting a plant based upon a desired phenotypic or genotypic trait during the course of the selective plant breeding program and simultaneously collecting the microorganisms associated with said plant or plant growth medium; and
h) providing the microorganisms collected from step g) to a plant or plant growth medium utilized in the next subsequent generation of the selective plant breeding program.

In particular embodiments, a selective pressure is applied in step a).

In yet another particular embodiment, a selective pressure is applied in step a) and wherein the selective pressure is biotic and includes exposing the one or more plants to an organism selected from the group consisting of: fungi, bacteria, viruses, insects, mites, nematodes, and combinations thereof.

Also provided herein are embodiments in which a selective pressure is applied in step a) and wherein the selective pressure is abiotic and includes exposing the one or more plants to an abiotic pressure selected from the group consisting of: salt concentration, temperature, pH, water, minerals, organic nutrients, inorganic nutrients, organic toxins, inorganic toxins, metals, and combinations thereof.

A particular embodiment provides that the selective plant breeding program is conducted in a soil-free or hydroponic system.

Also provided herein is a method for conducting holobiome plant breeding, comprising:
a) subjecting one or more plants to a growth medium in the presence of a first set of one or more microorganisms;
b) selecting one or more plants and/or growth medium following step a);
c) acquiring a second set of one or more microorganisms from said one or more plants and/or growth medium selected in step b);
d) repeating steps a) to c) one or more times, wherein the second set of one or more microorganisms acquired in step c) is used as the first set of microorganisms in step a) of any successive repeat;
e) selecting one or more microorganisms that is associated with imparting a beneficial property to a plant;
f) providing the selected one or more microorganisms to a plant undergoing a selective plant breeding program or a growth medium used to grow said plant during the selective plant breeding program;
g) selecting a plant based upon a desired phenotypic or genotypic trait during the course of the selective plant breeding program and simultaneously collecting the microorganisms associated with said plant or plant growth medium; and
h) providing the microorganisms collected from step g) to a plant or plant growth medium utilized in the next subsequent generation of the selective plant breeding program.

Another aspect of the disclosure provides for a method for conducting holobiome plant breeding, comprising:
a) crossing two plant cultivars to produce F1 hybrid plants;
b) selfing the F1 hybrid plants to produce F2 seed;
c) planting the F2 seed in soil collected from a region exhibiting a desired environmental property, wherein said desired environmental property represents an environmental property for which the successive cohort plants of the selective plant breeding process are selected to tolerate;
d) growing the F2 seed under environmental conditions that approximate the desired environmental property;
e) selecting F2 plants that exhibit the best phenotypic response to said environmental property and allowing said selected F2 plants to reach maturity and set F3 seed;
f) harvesting F3 seed from the selected F2 plants and simultaneously harvesting a microbial community associated with the F2 plants and/or the soil utilized to grow said F2 plants;
g) planting the F3 seed in soil from step c) that has been inoculated with the microbial community collected in step f); and
h) repeating steps d) to g) one or more times.

Some embodiments provide that the soil utilized in step g), and any successive repeats of the plant selection process, is autoclaved before being inoculated with the microbial community collected in the preceding step.

Some embodiments provide that the desired environmental property is selected from the group consisting of: cold temperature, high temperature, high humidity, drought, salinity, low nitrogen, low phosphorous, low photosynthetically active radiation, high elemental metal concentrations, high soil acidity, and combinations thereof.

In an aspect, the soil from step c) is inoculated by applying to said soil a granule, plug, or liquid drench, comprising the harvested microbial community.

In particular embodiments, the plant selection process is repeated through the production of F4 seed, or F5 seed, or F6 seed, or F7 seed.

An aspect of the method is provided that further comprises: maintaining parental lines as controls through each successive plant selection cycle and said parental lines are grown in the soil from step c), but said soil is not inoculated with a harvested microbial community during successive plant selection cycles.

An aspect of the method is provided that further comprises: maintaining parental lines as controls through each successive plant selection cycle and said parental lines are grown in the soil from step c), but said soil is not inoculated with a harvested microbial community during successive plant selection cycles; and wherein the plant selection process is repeated through the production of F5 seed; and wherein the selected F4 plants that produced the F5 seed demonstrate an increased desired phenotypic response to said environmental property, as compared to the parental line plants.

An aspect of the method is provided that further comprises:
h) repeating steps d) to g) through the production of F5 seed;
i) planting the harvested F5 seed, and the microbial community harvested in association with the F4 plants and/or the soil utilized to grow said F4 plants that produced the F5 seed, in a replicated field trial; and
j) selecting the best performing F5 plants.

### BRIEF DESCRIPTION OF THE FIGURES

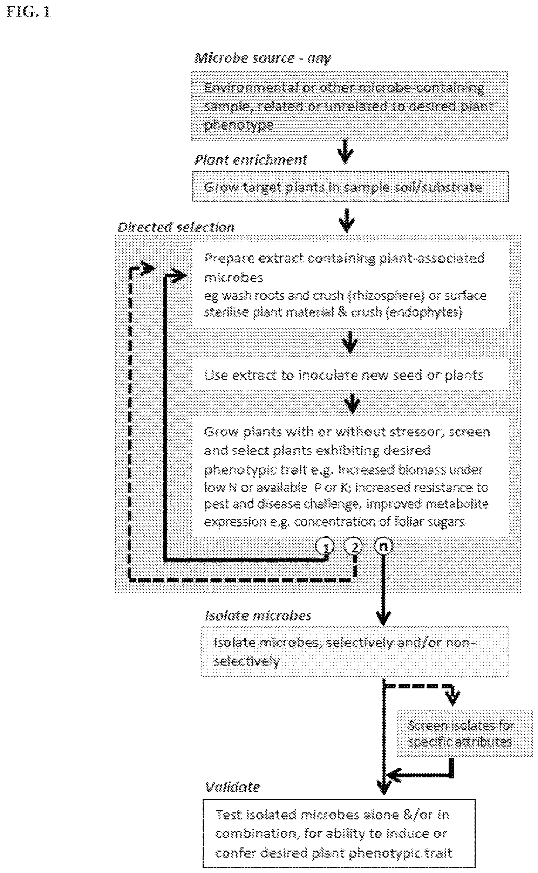

**FIG. 1** shows a generalized process schematic of a disclosed method of accelerated microbial selection, also referred to herein as directed microbial selection. When the process is viewed in the context of a microbial consortium, the schematic is illustrative of a process of directed evolution of a microbial consortium.
**FIG. 2** shows a generalized process flow chart of an embodiment of the taught methods.

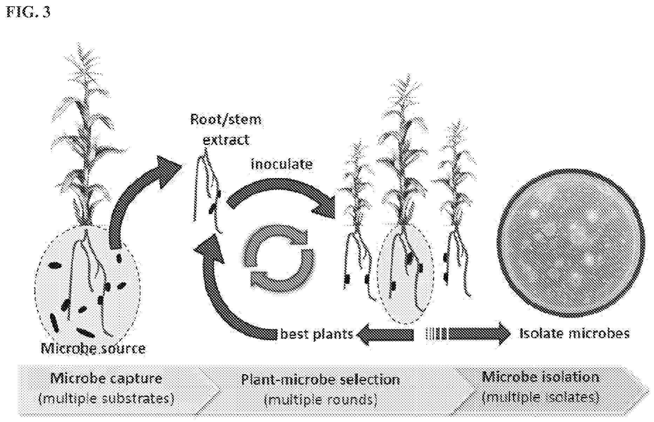

**FIG. 3** shows a graphic representation and associated flow chart of an embodiment of the disclosed methods.

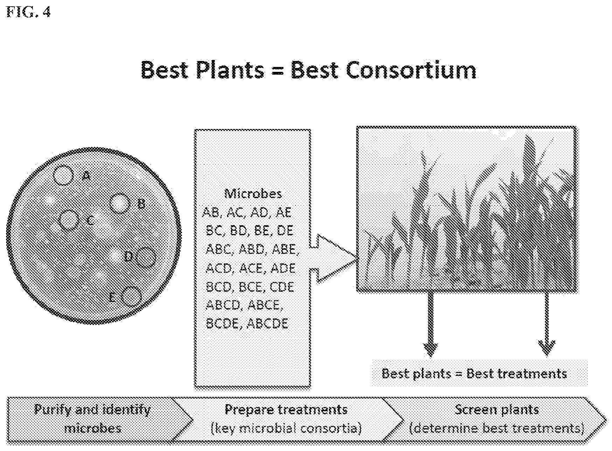

**FIG. 4** shows a graphic representation and associated flow chart of an embodiment of the disclosed methods. The figure illustrates the ability to evolve microbial consortia for imparting a desirable phenotypic trait in a plant.

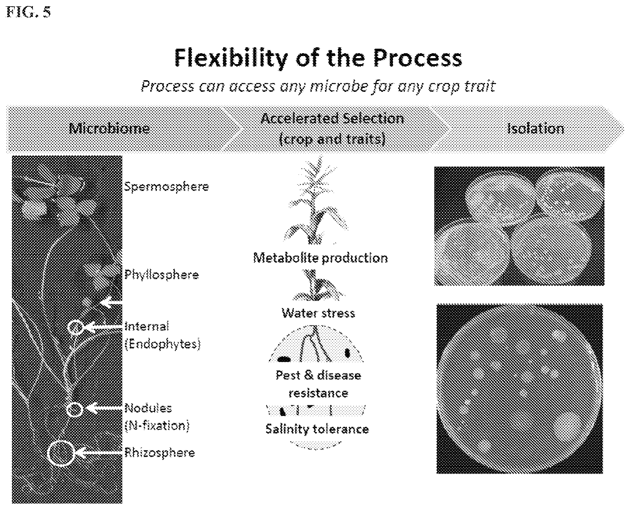

**FIG. 5** shows a graphic representation and associated flow chart of an embodiment of the disclosed methods and illustrates that the methods can utilize microbes from a variety of sources (including multiple locations from a single plant) and can select microbes that help develop a myriad of plant phenotypic traits, e.g. salinity tolerance, pest and disease resistance, water stress, and metabolite production.

### DETAILED DESCRIPTION

### Definitions

While the following terms are believed to be well understood by one of ordinary skill in the art, the following definitions are set forth to facilitate explanation of the presently disclosed subject matter

The term "a" or "an" refers to one or more of that entity; for example, "a gene" refers to one or more genes or at least one gene. As such, the terms "a" (or "an"), "one or more" and "at least one" are used interchangeably herein. In addition, reference to "an element" by the indefinite article "a" or "an" does not exclude the possibility that more than one of the elements is present, unless the context clearly requires that there is one and only one of the elements.

As used herein, the verb "comprise" as is used in this description and in the claims and its conjugations are used in its non-limiting sense to mean that items following the word are included, but items not specifically mentioned are not excluded.

As used herein the terms "microorganism" or "microbe" should be taken broadly. These terms, used interchangeably, include but are not limited to the two prokaryotic domains, Bacteria and Archaea, as well as eukaryotic fungi and protists.

The term "microbial consortia" refers to a subset of a microbial community of individual microbial species or strains of a species that can be described as carrying out a common function, or can be described as participating in, or leading to, or correlating with, a recognizable parameter or plant phenotypic trait. The community may comprise two or more species or strains of a species of microbes. In some instances, the microbes coexist within the community symbiotically.

The term "microbial community" means a group of microbes comprising two or more species or strains.

The term "directed evolution" is used in the broadest sense of the word "evolve" and does not necessarily refer to Mendelian inheritance. Thus, to "evolve" means to change. This change can be brought about by various parameters. In the examples that follow, a microbial community is evolved, *i.e.* the microbial community changes, over iterative selection steps according to the taught methods. In some embodiments, after several iterative rounds of accelerated microbial selection, the microbial community that results is drastically different from the microbial community present at the start of the method. Thus, in some embodiments, the methods take a random and heterogeneous microbial community, said members not necessarily working toward a desired function, but over the course of the iterative selection steps of the taught methods, a microbial community begins to emerge, wherein microbial species participate/correlate to a desired function, e.g. increasing a plant phenotypic trait of interest.

The term "accelerated microbial selection" or "AMS" is used interchangeably with the term "directed microbial selection" or "DMS" and refers to the iterative selection methodology elaborated upon in the disclosure.

As used herein, the term "genotype" refers to the genetic makeup of an individual cell, cell culture, tissue, organism (e.g., a plant), or group of organisms.

As used herein, the term "allele(s)" means any of one or more alternative forms of a gene, all of which alleles relate to at least one trait or characteristic. In a diploid cell, the two alleles of a given gene occupy corresponding loci on a pair of homologous chromosomes. Since the present disclosure, in embodiments, relates to QTLs, *i.e.* genomic regions that may comprise one or more genes or regulatory sequences, it is in some instances more accurate to refer to "haplotype" (i.e. an allele of a chromosomal segment) instead of "allele", however, in those instances, the term "allele" should be understood to comprise the term "haplotype". Alleles are considered identical when they express a similar phenotype. Differences in sequence are possible but not important as long as they do not influence phenotype.

As used herein, the term "locus" (loci plural) means a specific place or places or a site on a chromosome where for example a gene or genetic marker is found.

As used herein, the term "genetically linked" refers to two or more traits that are coinherited at a high rate during breeding such that they are difficult to separate through crossing.

A "recombination" or "recombination event" as used herein refers to a chromosomal crossing over or independent assortment. The term "recombinant" refers to a plant having a new genetic make up arising as a result of recombination event.

As used herein, the term "molecular marker" or "genetic marker" refers to an indicator that is used in methods for visualizing differences in characteristics of nucleic acid sequences. Examples of such indicators are restriction fragment length polymorphism (RFLP) markers, amplified fragment length polymorphism (AFLP) markers, single nucleotide polymorphisms (SNPs), insertion mutations, microsatellite markers (SSRs), sequence- characterized amplified regions (SCARs), cleaved amplified polymorphic sequence (CAPS) markers or isozyme markers or combinations of the markers described herein which defines a specific genetic and chromosomal location. Mapping of molecular markers in the vicinity of an allele is a procedure which can be performed quite easily by the average person skilled in molecular-biological techniques which techniques are for instance described in Lefebvre and Chevre, 1995; Lorez and Wenzel, 2007, Srivastava and Narula, 2004, Meksem and Kahl, 2005, Phillips and Vasil, 2001. General information concerning AFLP technology can be found in Vos et al.(1995, AFLP: a new technique for DNA fingerprinting, Nucleic Acids Res. 1995 November 11; 23(21): 4407-4414). Each of these references is hereby incorporated by reference in their entirety.

As used herein, the term "trait" refers to a characteristic or phenotype. For example, in the context of some embodiments of the present disclosure, yield of a crop relates to the amount of marketable biomass produced by a plant (e.g., fruit, fiber, grain). Desirable traits may also include other plant characteristics, including but not limited to: water use efficiency, nutrient use efficiency, production, mechanical harvestability, fruit maturity and shelf life, pest/disease resistance, early plant maturity, tolerance to stresses, etc. A trait may be inherited in a dominant or recessive manner, or in a partial or incomplete-dominant manner. A trait may be monogenic (i.e. determined by a single locus) or polygenic (i.e. determined by more than one locus) or may also result from the interaction of one or more genes with the environment.

A dominant trait results in a complete phenotypic manifestation at heterozygous or homozygous state; a recessive trait manifests itself only when present at homozygous state.

In the context of this disclosure, traits may also result from the interaction of one or more plant genes and one or more microorganism genes. Thus, in embodiments, the disclosure refers to a "holobiome," which refers to the entirety of genetic variability that is present within a plant undergoing a breeding selection process and also the genetic variability associated with one or more microorganisms inhabiting the growth medium or otherwise associated with the plant. Thus, as a simple example, consider a single plant being grown in a conventional nursery pot in a greenhouse with soil media, said single plant and nursery pot forming a single replicate from within a larger multi-replicate traditional breeding scheme. The genes associated with the plant, along with the genes associated with one or more microorganisms inhabiting the soil of the nursery pot, along with those microorganisms inhabiting the soil surface, or those microorganisms that inhabit the plant itself, would constitute the "holobiome."

As used herein, the term "traditional plant breeding" refers to methods of plant husbandry in which plants are crossed with each other to produce genetically and (potentially) phenotypically distinct plants. The term traditional plant breeding describes not only the historical plant breeding methods of cross-pollination employed for thousands of years, but also newer marker assisted, or double haploid plant breeding technologies. Traditional plant breeding therefore refers to all manner of plant breeding that existed before the present disclosure. The present disclosure teaches new methods of plant breeding, which control for microbial variability associated with the plants undergoing the breeding process. Thus, the disclosure improves upon the plant breeding methods that existed before *(i.e.* traditional plant breeding) the current methodology.

As used herein, the term "homozygous" means a genetic condition existing when two identical alleles reside at a specific locus, but are positioned individually on corresponding pairs of homologous chromosomes in the cell of a diploid organism. Conversely, as used herein, the term "heterozygous" means a genetic condition existing when two different alleles reside at a specific locus, but are positioned individually on corresponding pairs of homologous chromosomes in the cell of a diploid organism.

As used herein, the term "plant" includes the whole plant or any parts or derivatives thereof, such as plant cells, plant protoplasts, plant cell tissue cultures from which plants can be regenerated, plant calli, embryos, pollen, ovules, fruit, flowers, leaves, seeds, roots, root tips and the like.

As used herein, the term "phenotype" refers to the observable characteristics of an individual cell, cell culture, organism (e.g., a plant), or group of organisms which results from the interaction between that individual's genetic makeup (i.e., genotype) and the environment.

As used herein, the term "derived from" refers to the origin or source, and may include naturally occurring, recombinant, unpurified, or purified molecules. A nucleic acid or an amino acid derived from an origin or source may have all kinds of nucleotide changes or protein modification as defined elsewhere herein.

As used herein, the term "offspring" refers to any plant resulting as progeny from a vegetative or sexual reproduction from one or more parent plants or descendants thereof. For instance an offspring plant may be obtained by cloning or selfing of a parent plant or by crossing two parents plants and include selfings as well as the F1 or F2 or still further generations. An F1 is a first-generation offspring produced from parents at least one of which is used for the first time as donor of a trait, while offspring of second generation (F2) or subsequent generations (F3, F4, etc.) are specimens produced from selfings of F1's, F2's etc. An F1 may thus be (and usually is) a hybrid resulting from a cross between two true breeding parents (true-breeding is homozygous for a trait), while an F2 may be (and usually is) an offspring resulting from self-pollination of said F1 hybrids.

As used herein, the term "cross", "crossing", "cross pollination" or "cross-breeding" refer to the process by which the pollen of one flower on one plant is applied (artificially or naturally) to the ovule (stigma) of a flower on another plant.

As used herein, the term "cultivar" refers to a variety, strain or race of plant that has been produced by horticultural or agronomic techniques and is not normally found in wild populations.

As used herein, the terms "dicotyledon," "dicot" and "dicotyledonous" refer to a flowering plant having an embryo containing two seed halves or cotyledons. Examples include tobacco; tomato; the legumes, including peas, alfalfa, clover and soybeans; oaks; maples; roses; mints; squashes; daisies; walnuts; cacti; violets and buttercups.

As used herein, the term "monocotyledon" or "monocot" refer to any of a subclass (Monocotyledoneae) of flowering plants having an embryo containing only one seed leaf and usually having parallel-veined leaves, flower parts in multiples of three, and no secondary growth in stems and roots. Examples include lilies; orchids; rice; corn, grasses, such as tall fescue, goat grass, and Kentucky bluegrass; grains, such as wheat, oats and barley; irises; onions and palms.

As used herein, "improved" should be taken broadly to encompass improvement of a characteristic of a plant which may already exist in a plant or plants prior to application of the disclosure, or the presence of a characteristic which did not exist in a plant or plants prior to application of the disclosure. By way of example, "improved" growth should be taken to include growth of a plant where the plant was not previously known to grow under the relevant conditions.

As used herein, "inhibiting and suppressing" and like terms should be taken broadly and should not be construed to require complete inhibition or suppression, although this may be desired in some embodiments.

As used herein, "isolate", "isolated" and like terms should be taken broadly. These terms are intended to mean that the one or more microorganism(s) has been separated at least partially from at least one of the materials with which it is associated in a particular environment (for example soil, water, plant tissue). "Isolate", "isolated" and like terms should not be taken to indicate the extent to which the microorganism(s) has been purified.

As used herein, "individual isolates" should be taken to mean a composition or culture comprising a predominance of a single genera, species or strain of microorganism, following separation from one or more other microorganisms. The phrase should not be taken to indicate the extent to which the microorganism has been isolated or purified. However, "individual isolates" preferably comprise substantially only one genus, species or strain of microorganism.

As used herein, the term "chimeric" or "recombinant" when describing a nucleic acid sequence or a protein sequence refers to a nucleic acid or a protein sequence that links at least two heterologous polynucleotides or two heterologous polypeptides into a single macromolecule, or that re-arranges one or more elements of at least one natural nucleic acid or protein sequence. For example, the term "recombinant" can refer to an artificial combination of two otherwise separated segments of sequence, e.g., by chemical synthesis or by the manipulation of isolated segments of nucleic acids by genetic engineering techniques.

The term "recombinant" in reference to a plant or other organism refers to an organism that has been genetically altered through plant transformation. Thus, in some contexts, the terms transgenic and recombinant are interchangeably used in this application.

As used herein, a "synthetic nucleotide sequence" or "synthetic polynucleotide sequence" is a nucleotide sequence that is not known to occur in nature or that is not naturally occurring. Generally, such a synthetic nucleotide sequence will comprise at least one nucleotide difference when compared to any other naturally occurring nucleotide sequence. It is recognized that a genetic regulatory element of the present disclosure comprises a synthetic nucleotide sequence. In some embodiments, the synthetic nucleotide sequence shares little or no extended homology to natural sequences. Extended homology in this context generally refers to 100% sequence identity extending beyond about 25 nucleotides of contiguous sequence. A synthetic genetic regulatory element of the present disclosure comprises a synthetic nucleotide sequence.

As used herein, the term "nucleic acid" refers to a polymeric form of nucleotides of any length, either ribonucleotides or deoxyribonucleotides, or analogs thereof. This term refers to the primary structure of the molecule, and thus includes double- and single-stranded DNA, as well as double- and single-stranded RNA. It also includes modified nucleic acids such as methylated and/or capped nucleic acids, nucleic acids containing modified bases, backbone modifications, and the like. The terms "nucleic acid" and "nucleotide sequence" are used interchangeably.

As used herein, the term "gene" refers to any segment of DNA associated with a biological function. Thus, genes include, but are not limited to, coding sequences and/or the regulatory sequences required for their expression. Genes can also include nonexpressed DNA segments that, for example, form recognition sequences for other proteins. Genes can be obtained from a variety of sources, including cloning from a source of interest or synthesizing from known or predicted sequence information, and may include sequences designed to have desired parameters.

As used herein, the term "homologous" or "homologue" or "ortholog" is known in the art and refers to related sequences that share a common ancestor or family member and are determined based on the degree of sequence identity. The terms "homology", "homologous", "substantially similar" and "corresponding substantially" are used interchangeably herein. They refer to nucleic acid fragments wherein changes in one or more nucleotide bases do not affect the ability of the nucleic acid fragment to mediate gene expression or produce a certain phenotype. These terms also refer to modifications of the nucleic acid fragments of the instant disclosure such as deletion or insertion of one or more nucleotides that do not substantially alter the functional properties of the resulting nucleic acid fragment relative to the initial, unmodified fragment. It is therefore understood, as those skilled in the art will appreciate, that the disclosure encompasses more than the specific exemplary sequences. These terms describe the relationship between a gene found in one species, subspecies, variety, cultivar or strain and the corresponding or equivalent gene in another species, subspecies, variety, cultivar or strain. For purposes of this disclosure homologous sequences are compared. "Homologous sequences" or "homologues" or "orthologs" are thought, believed, or known to be functionally related. A functional relationship may be indicated in any one of a number of ways, including, but not limited to: (a) degree of sequence identity and/or (b) the same or similar biological function. Preferably, both (a) and (b) are indicated. Homology can be determined using software programs readily available in the art, such as those discussed in Current Protocols in Molecular Biology (F.M. Ausubel et al., eds., 1987) Supplement 30, section 7.718, Table 7.71. Some alignment programs are MacVector (Oxford Molecular Ltd, Oxford, U.K.), ALIGN Plus (Scientific and Educational Software, Pennsylvania) and AlignX (Vector NTI, Invitrogen, Carlsbad, CA). Another alignment program is Sequencher (Gene Codes, Ann Arbor, Michigan), using default parameters.

As used herein, the term "nucleotide change" refers to, e.g., nucleotide substitution, deletion, and/or insertion, as is well understood in the art. For example, mutations contain alterations that produce silent substitutions, additions, or deletions, but do not alter the properties or activities of the encoded protein or how the proteins are made.

As used herein, the term "protein modification" refers to, e.g., amino acid substitution, amino acid modification, deletion, and/or insertion, as is well understood in the art.

As used herein, the term "derived from" refers to the origin or source, and may include naturally occurring, recombinant, unpurified, or purified molecules. A nucleic acid or an amino acid derived from an origin or source may have all kinds of nucleotide changes or protein modification as defined elsewhere herein.

The disclosure provides agents to make and use the biological materials of the present disclosure. As used herein, the term "agent", as used herein, means a biological or chemical compound such as a simple or complex organic or inorganic molecule, a peptide, a protein or an oligonucleotide that modulates the function of a nucleic acid or polypeptide. A vast array of compounds can be synthesized, for example oligomers, such as oligopeptides and oligonucleotides, and synthetic organic and inorganic compounds based on various core structures, and these are also included in the term "agent". In addition, various natural sources can provide compounds for screening, such as plant or animal extracts, and the like. Compounds can be tested singly or in combination with one another.

As used herein, the term 'at least a portion" or "fragment" of a nucleic acid or polypeptide means a portion having the minimal size characteristics of such sequences, or any larger fragment of the full length molecule, up to and including the full length molecule. A fragment of a polynucleotide of the disclosure may encode a biologically active portion of a genetic regulatory element. A biologically active portion of a genetic regulatory element can be prepared by isolating a portion of one of the polynucleotides of the disclosure that comprises the genetic regulatory element and assessing activity as described herein. Similarly, a portion of a polypeptide may be 4 amino acids, 5 amino acids, 6 amino acids, 7 amino acids, and so on, going up to the full length polypeptide. The length of the portion to be used will depend on the particular application. A portion of a nucleic acid useful as hybridization probe may be as short as 12 nucleotides; in some embodiments, it is 20 nucleotides. A portion of a polypeptide useful as an epitope may be as short as 4 amino acids. A portion of a polypeptide that performs the function of the full-length polypeptide would generally be longer than 4 amino acids.

Variant polynucleotides also encompass sequences derived from a mutagenic and recombinogenic procedure such as DNA shuffling. Strategies for such DNA shuffling are known in the art. *See,* for example, Stemmer (1994) PNAS 91:10747-10751; Stemmer (1994) Nature 370:389-391; Crameri et al.(1997) Nature Biotech. 15:436-438; Moore et al.(1997) J. Mol. Biol. 272:336-347; Zhang et al.(1997) PNAS 94:4504-4509; Crameri et al.(1998) Nature 391:288-291; and U.S. Patent Nos. 5,605,793 and 5,837,458. For PCR amplifications of the polynucleotides disclosed herein, oligonucleotide primers can be designed for use in PCR reactions to amplify corresponding DNA sequences from cDNA or genomic DNA extracted from any plant of interest. Methods for designing PCR primers and PCR cloning are generally known in the art and are disclosed in Sambrook et al.(1989) Molecular Cloning: A Laboratory Manual (2nd ed., Cold Spring Harbor Laboratory Press, Plainview, New York). See also Innis et al., eds. (1990) PCR Protocols: A Guide to Methods and Applications (Academic Press, New York); Innis and Gelfand, eds. (1995) PCR Strategies (Academic Press, New York); and Innis and Gelfand, eds. (1999) PCR Methods Manual (Academic Press, New York). Known methods of PCR include, but are not limited to, methods using paired primers, nested primers, single specific primers, degenerate primers, gene-specific primers, vector-specific primers, partially-mismatched primers, and the like.

The term "primer" as used herein refers to an oligonucleotide which is capable of annealing to the amplification target allowing a DNA polymerase to attach, thereby serving as a point of initiation of DNA synthesis when placed under conditions in which synthesis of primer extension product is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxyribonucleotide. The primer must be sufficiently long to prime the synthesis of extension products in the presence of the agent for polymerization. The exact lengths of the primers will depend on many factors, including temperature and composition (A/T vs. G/C content) of primer. A pair of bi-directional primers consists of one forward and one reverse primer as commonly used in the art of DNA amplification such as in PCR amplification.

The terms "stringency" or "stringent hybridization conditions" refer to hybridization conditions that affect the stability of hybrids, e.g., temperature, salt concentration, pH, formamide concentration and the like. These conditions are empirically optimized to maximize specific binding and minimize non-specific binding of primer or probe to its target nucleic acid sequence. The terms as used include reference to conditions under which a probe or primer will hybridize to its target sequence, to a detectably greater degree than other sequences (e.g. at least 2-fold over background). Stringent conditions are sequence dependent and will be different in different circumstances. Longer sequences hybridize specifically at higher temperatures. Generally, stringent conditions are selected to be about 5° C lower than the thermal melting point (Tm) for the specific sequence at a defined ionic strength and pH. The Tm is the temperature (under defined ionic strength and pH) at which 50% of a complementary target sequence hybridizes to a perfectly matched probe or primer. Typically, stringent conditions will be those in which the salt concentration is less than about 1.0 M Na+ ion, typically about 0.01 to 1.0 M Na + ion concentration (or other salts) at pH 7.0 to 8.3 and the temperature is at least about 30° C for short probes or primers (e.g. 10 to 50 nucleotides) and at least about 60° C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37° C and a wash in 2×SSC at 40° C. Exemplary high stringency conditions include hybridization in 50% formamide, 1M NaCl, 1% SDS at 37° C, and a wash in 0.1×SSC at 60° C. Hybridization procedures are well known in the art and are described by e.g. Ausubel *et al.,* 1998 and Sambrook *et al.,* 2001. In some embodiments, stringent conditions are hybridization in 0.25 M Na2HPO4 buffer (pH 7.2) containing 1 mM Na2EDTA, 0.5-20% sodium dodecyl sulfate at 45°C, such as 0.5%, 1%, 2%, 3%, 4%, 5%, 6%, 7%, 8%, 9%, 10%, 11%, 12%, 13%, 14%, 15%, 16%, 17%, 18%, 19% or 20%, followed by a wash in 5×SSC, containing 0.1% (w/v) sodium dodecyl sulfate, at 55°C to 65°C.

As used herein, "promoter" refers to a DNA sequence capable of controlling the expression of a coding sequence or functional RNA. The promoter sequence consists of proximal and more distal upstream elements, the latter elements often referred to as enhancers. Accordingly, an "enhancer" is a DNA sequence that can stimulate promoter activity, and may be an innate element of the promoter or a heterologous element inserted to enhance the level or tissue specificity of a promoter. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity.

As used herein, a "plant promoter" is a promoter capable of initiating transcription in plant cells whether or not its origin is a plant cell, e.g. it is well known that Agrobacterium promoters are functional in plant cells. Thus, plant promoters include promoter DNA obtained from plants, plant viruses and bacteria such as Agrobacterium and Bradyrhizobium bacteria. A plant promoter can be a constitutive promoter or a non-constitutive promoter.

As used herein, a "constitutive promoter" is a promoter which is active under most conditions and/or during most development stages. There are several advantages to using constitutive promoters in expression vectors used in plant biotechnology, such as: high level of production of proteins used to select transgenic cells or plants; high level of expression of reporter proteins or scorable markers, allowing easy detection and quantification; high level of production of a transcription factor that is part of a regulatory transcription system; production of compounds that requires ubiquitous activity in the plant; and production of compounds that are required during all stages of plant development. Non-limiting exemplary constitutive promoters include, CaMV 35S promoter, opine promoters, ubiquitin promoter, alcohol dehydrogenase promoter, etc.

As used herein, a "non-constitutive promoter" is a promoter which is active under certain conditions, in certain types of cells, and/or during certain development stages. For example, tissue specific, tissue preferred, cell type specific, cell type preferred, inducible promoters, and promoters under development control are non-constitutive promoters. Examples of promoters under developmental control include promoters that preferentially initiate transcription in certain tissues, such as stems, leaves, roots, or seeds.

As used herein, "inducible" or "repressible" promoter is a promoter which is under chemical or environmental factors control. Examples of environmental conditions that may effect transcription by inducible promoters include anaerobic conditions, or certain chemicals, or the presence of light.

As used herein, a "tissue specific" promoter is a promoter that initiates transcription only in certain tissues. Unlike constitutive expression of genes, tissue-specific expression is the result of several interacting levels of gene regulation. As such, in the art sometimes it is preferable to use promoters from homologous or closely related plant species to achieve efficient and reliable expression of transgenes in particular tissues. This is one of the main reasons for the large amount of tissue-specific promoters isolated from particular plants and tissues found in both scientific and patent literature.

As used herein, a "tissue preferred" promoter is a promoter that initiates transcription mostly, but not necessarily entirely or solely in certain tissues.

As used herein, a "cell type specific" promoter is a promoter that primarily drives expression in certain cell types in one or more organs, for example, vascular cells in roots, leaves, stalk cells, and stem cells.

As used herein, a "cell type preferred" promoter is a promoter that primarily drives expression mostly, but not necessarily entirely or solely in certain cell types in one or more organs, for example, vascular cells in roots, leaves, stalk cells, and stem cells.

As used herein, "intron" is any nucleotide sequence within a gene that is removed by RNA splicing while the final mature RNA product of a gene is being generated. The term refers to both the DNA sequence within a gene, and the corresponding sequence in RNA transcripts.

As used herein, the "3' non-coding sequences" or "3' untranslated regions" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht, I. L., et al.(1989) Plant Cell 1:671-680.

As used herein, the term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., that the coding sequence is under the transcriptional control of the promoter). Coding sequences can be operably linked to regulatory sequences in a sense or antisense orientation. In another example, the complementary RNA regions of the disclosure can be operably linked, either directly or indirectly, 5' to the target mRNA, or 3' to the target mRNA, or within the target mRNA, or a first complementary region is 5' and its complement is 3' to the target mRNA.

As used herein, the phrases "recombinant construct", "expression construct", "chimeric construct", "construct", and "recombinant DNA construct" are used interchangeably herein. A recombinant construct comprises an artificial combination of nucleic acid fragments, e.g., regulatory and coding sequences that are not found together in nature. For example, a chimeric construct may comprise regulatory sequences and coding sequences that are derived from different sources, or regulatory sequences and coding sequences derived from the same source, but arranged in a manner different than that found in nature. Such construct may be used by itself or may be used in conjunction with a vector. If a vector is used then the choice of vector is dependent upon the method that will be used to transform host cells as is well known to those skilled in the art For example, a plasmid vector can be used. The skilled artisan is well aware of the genetic elements that must be present on the vector in order to successfully transform, select and propagate host cells comprising any of the isolated nucleic acid fragments of the disclosure. The skilled artisan will also recognize that different independent transformation events will result in different levels and patterns of expression (Jones et al., (1985) EMBO J. 4:2411-2418; De Almeida et al., (1989) Mol. Gen. Genetics 218:78-86), and thus that multiple events must be screened in order to obtain lines displaying the desired expression level and pattern. Such screening may be accomplished by Southern analysis of DNA, Northern analysis of mRNA expression, immunoblotting analysis of protein expression, or phenotypic analysis, among others. Vectors can be plasmids, viruses, bacteriophages, pro-viruses, phagemids, transposons, artificial chromosomes, and the like, that replicate autonomously or can integrate into a chromosome of a host cell. A vector can also be a naked RNA polynucleotide, a naked DNA polynucleotide, a polynucleotide composed of both DNA and RNA within the same strand, a poly-lysine-conjugated DNA or RNA, a peptide-conjugated DNA or RNA, a liposomeconjugated DNA, or the like, that is not autonomously replicating. As used herein, the term "expression" refers to the production of a functional end-product e.g., an mRNA or a protein (precursor or mature).

In some embodiments, the expression cassettes or recombinant constructs comprise at least one selectable or screenable marker. In some embodiments, the selectable or screenable marker is a plant selectable or screenable marker. As used herein, the phrase "plant selectable or screenable marker" refers to a genetic marker functional in a plant cell. A selectable marker allows cells containing and expressing that marker to grow under conditions unfavorable to growth of cells not expressing that marker. A screenable marker facilitates identification of cells which express that marker.

The disclosure provides inbred plants comprising recombinant sequences. As used herein, the term "inbred", "inbred plant" is used in the context of the present disclosure. This also includes any single gene conversions of that inbred. The term single allele converted plant as used herein refers to those plants which are developed by a plant breeding technique called backcrossing wherein essentially all of the desired morphological and physiological characteristics of an inbred are recovered in addition to the single allele transferred into the inbred via the backcrossing technique.

The disclosure provides samples comprising recombinant sequences. As used herein, the term "sample" includes a sample from a plant, a plant part, a plant cell, or from a transmission vector, or a soil, water or air sample.

The disclosure provides offsprings comprising recombinant sequences. As used herein, the term "offspring" refers to any plant resulting as progeny from a vegetative or sexual reproduction from one or more parent plants or descendants thereof. For instance an offspring plant may be obtained by cloning or selfing of a parent plant or by crossing two parent plants and include selfings as well as the F1 or F2 or still further generations. An F1 is a first-generation offspring produced from parents at least one of which is used for the first time as donor of a trait, while offspring of second generation (F2) or subsequent generations (F3, F4, etc.) are specimens produced from selfings of F1's, F2's etc. An F1 may thus be (and usually is) a hybrid resulting from a cross between two true breeding parents (true-breeding is homozygous for a trait), while an F2 may be (and usually is) an offspring resulting from self-pollination of said F1 hybrids.

The disclosure provides methods for crossing a first plant comprising recombinant sequences with a second plant. As used herein, the term "cross", "crossing", "cross pollination" or "cross-breeding" refer to the process by which the pollen of one flower on one plant is applied (artificially or naturally) to the ovule (stigma) of a flower on another plant.

The disclosure provides plant cultivars comprising recombinant sequences. As used herein, the term "cultivar" refers to a variety, strain or race of plant that has been produced by horticultural or agronomic techniques and is not normally found in wild populations.

In some embodiments, the present disclosure provides methods for obtaining plant genotypes comprising recombinant genes. As used herein, the term "genotype" refers to the genetic makeup of an individual cell, cell culture, tissue, organism (e.g., a plant), or group of organisms.

In some embodiments, the present disclosure provides homozygotes comprising recombinant genes. As used herein, the term "homozygote" refers to an individual cell or plant having the same alleles at one or more loci.

In some embodiments, the present disclosure provides homozygous plants comprising recombinant genes. As used herein, the term "homozygous" refers to the presence of identical alleles at one or more loci in homologous chromosomal segments.

In some embodiments, the transgenic cell or organism is hemizygous for the gene of interest which is under control of promoters of the present disclosure. As used herein, the term "hemizygous" refers to a cell, tissue or organism in which a gene is present only once in a genotype, as a gene in a haploid cell or organism, a sex-linked gene in the heterogametic sex, or a gene in a segment of chromosome in a diploid cell or organism where its partner segment has been deleted.

In some embodiments, the present disclosure provides heterozygotes comprising recombinant genes. As used herein, the terms "heterozygote" and "heterozygous" refer to a diploid or polyploid individual cell or plant having different alleles (forms of a given gene) present at least at one locus. In some embodiments, the cell or organism is heterozygous for the gene of interest which is under control of the synthetic regulatory element. As used herein, the terms "heterologous polynucleotide" or a "heterologous nucleic acid" or an "exogenous DNA segment" refer to a polynucleotide, nucleic acid or DNA segment that originates from a source foreign to the particular host cell, or, if from the same source, is modified from its original form. Thus, a heterologous gene in a host cell includes a gene that is endogenous to the particular host cell, but has been modified. Thus, the terms refer to a DNA segment which is foreign or heterologous to the cell, or homologous to the cell but in a position within the host cell nucleic acid in which the element is not ordinarily found. Exogenous DNA segments are expressed to yield exogenous polypeptides.

In some embodiments, the cell or organism has at least one heterologous trait. As used herein, the term "heterologous trait" refers to a phenotype imparted to a transformed host cell or transgenic organism by an exogenous DNA segment, heterologous polynucleotide or heterologous nucleic acid. Various changes in phenotype are of interest to the present disclosure, including but not limited to modifying the fatty acid composition in a plant, altering the amino acid content of a plant, altering a plant's pathogen defense mechanism, increasing a plant's yield of an economically important trait (e.g., grain yield, forage yield, etc.) and the like. These results can be achieved by providing expression of heterologous products or increased expression of endogenous products in plants using the methods and compositions of the present disclosure.

The disclosure provides methods for obtaining plant lines comprising recombinant genes. As used herein, the term "line" is used broadly to include, but is not limited to, a group of plants vegetatively propagated from a single parent plant, via tissue culture techniques or a group of inbred plants which are genetically very similar due to descent from a common parent(s). A plant is said to "belong" to a particular line if it (a) is a primary transformant (T0) plant regenerated from material of that line; (b) has a pedigree comprised of a TO plant of that line; or (c) is genetically very similar due to common ancestry (e.g., via inbreeding or selfing). In this context, the term "pedigree" denotes the lineage of a plant, e.g. in terms of the sexual crosses affected such that a gene or a combination of genes, in heterozygous (hemizygous) or homozygous condition, imparts a desired trait to the plant.

The disclosure provides open-pollinated populations comprising recombinant genes. As used herein, the terms "open-pollinated population" or "open-pollinated variety" refer to plants normally capable of at least some cross-fertilization, selected to a standard, that may show variation but that also have one or more genotypic or phenotypic characteristics by which the population or the variety can be differentiated from others. A hybrid, which has no barriers to cross-pollination, is an open-pollinated population or an open-pollinated variety.

The disclosure provides self-pollination populations comprising recombinant genes. As used herein, the term "self-crossing", "self pollinated" or "self-pollination" means the pollen of one flower on one plant is applied (artificially or naturally) to the ovule (stigma) of the same or a different flower on the same plant.

The disclosure provides ovules and pollens comprising recombinant genes. As used herein when discussing plants, the term "ovule" refers to the female gametophyte, whereas the term "pollen" means the male gametophyte.

In some embodiments, the transgenic plants comprising recombinant genes have one or more preferred phenotypes. As used herein, the term "phenotype" refers to the observable characters of an individual cell, cell culture, organism (e.g., a plant), or group of organisms which results from the interaction between that individual's genetic makeup (i.e., genotype) and the environment.

The disclosure provides plant tissue comprising recombinant genes. As used herein, the term "plant tissue" refers to any part of a plant. Examples of plant organs include, but are not limited to the leaf, stem, root, tuber, seed, branch, pubescence, nodule, leaf axil, flower, pollen, stamen, pistil, petal, peduncle, stalk, stigma, style, bract, fruit, trunk, carpel, sepal, anther, ovule, pedicel, needle, cone, rhizome, stolon, shoot, pericarp, endosperm, placenta, berry, stamen, and leaf sheath.

The disclosure provides methods for obtaining plants comprising recombinant genes through transformation. As used herein, the term "transformation" refers to the transfer of nucleic acid *(i.e.,* a nucleotide polymer) into a cell. As used herein, the term "genetic transformation" refers to the transfer and incorporation of DNA, especially recombinant DNA, into a cell.

The disclosure provides transformants comprising recombinant genes. As used herein, the term "transformant" refers to a cell, tissue or organism that has undergone transformation. The original transformant is designated as "T0" or "TO." Selfing the TO produces a first transformed generation designated as "T1" or "T1."

The present disclosure provides transgenes comprising recombinant promoters. As used herein, the term "transgene" refers to a nucleic acid that is inserted into an organism, host cell or vector in a manner that ensures its function.

The disclosure provides transgenic plants comprising recombinant promoters. As used herein, the term "transgenic" refers to cells, cell cultures, organisms (e.g., plants), and progeny which have received a foreign or modified gene by one of the various methods of transformation, wherein the foreign or modified gene is from the same or different species than the species of the organism receiving the foreign or modified gene.

The disclosure provides transgenic events comprising recombinant promoters. As used herein, the term "transposition event" refers to the movement of a transposon from a donor site to a target site.

In some embodiments, the present disclosure provides plant varieties comprising recombinant genes. As used herein, the term "variety" refers to a subdivision of a species, consisting of a group of individuals within the species that are distinct in form or function from other similar arrays of individuals.

In some embodiments, the present disclosure provides organisms recombinant genes. As used herein, an "organism" refers any life form that has genetic material comprising nucleic acids including, but not limited to, prokaryotes, eukaryotes, and viruses. Organisms of the present disclosure include, for example, plants, animals, fungi, bacteria, and viruses, and cells and parts thereof.

As used herein, "coding sequence" refers to a DNA sequence that codes for a specific amino acid sequence. By "gene of interest" is intended any nucleotide sequence that can be expressed when operably linked to a promoter. A gene of interest of the present disclosure may, but need not, encode a protein. Unless stated otherwise or readily apparent from the context, when a gene of interest of the present disclosure is said to be operably linked to a promoter of the disclosure, the gene of interest does not by itself comprise a functional promoter. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. As used herein, "regulatory sequences" may include, but are not limited to, promoters, translation leader sequences, introns, and polyadenylation recognition sequences. As used herein, the term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., that the coding sequence is under the transcriptional control of the promoter). Coding sequences can be operably linked to regulatory sequences in a sense or antisense orientation. In another example, the complementary RNA regions of the disclosure can be operably linked, either directly or indirectly, 5' to the target mRNA, or 3' to the target mRNA, or within the target mRNA, or a first complementary region is 5' and its complement is 3' to the target mRNA.

As used herein a "reporter" or a "reporter gene" refers to a nucleic acid molecule encoding a detectable marker. The reporter gene can be, for example, luciferase (e.g., firefly luciferase or Renilla luciferase), GUS (β-glucuronidase), β-galactosidase, chloramphenicol acetyl transferase (CAT), or a fluorescent protein (e.g., green fluorescent protein (GFP), red fluorescent protein (DsRed), yellow fluorescent protein, blue fluorescent protein, cyan fluorescent protein, or variants thereof. Reporter genes are detectable by a reporter assay. Reporter assays can measure the level of reporter gene expression or activity by any number of means, including, for example, measuring the level of reporter mRNA, the level of reporter protein, or the amount of reporter protein activity. Reporter assays are known in the art or otherwise disclosed herein.

### Transgenic Methods

Any transgenic plant incorporated with the expression cassette generated from the present disclosure can be used as a donor to produce more transgenic plants through plant breeding methods well known to those skilled in the art. Particular embodiments of plant breeding techniques are discussed later in the present Application.

The goal, in general, is to develop new, unique and superior varieties and hybrids. In some embodiments, selection methods, e.g., molecular marker assisted selection, can be combined with breeding methods to accelerate the process.

Additional breeding methods have been known to one of ordinary skill in the art, e.g., methods discussed in Chahal and Gosal (Principles and procedures of plant breeding: biotechnological and conventional approaches, CRC Press, 2002, ISBN 084931321X, 9780849313219), Taji et al.(In vitro plant breeding, Routledge, 2002, ISBN 156022908X, 9781560229087), Richards (Plant breeding systems, Taylor & Francis US, 1997, ISBN 0412574500, 9780412574504), Hayes (Methods of Plant Breeding, Publisher: READ BOOKS, 2007, ISBN1406737062, 9781406737066), each of which is incorporated by reference in its entirety.

In some embodiments, said method comprises (i) crossing any one of the plants of the present disclosure comprising the expression cassette as a donor to a recipient plant line to create a F1 population; (ii) selecting offsprings that have expression cassette. Optionally, the offsprings can be further selected by testing the expression of the gene of interest.

In some embodiments, complete chromosomes of the donor plant are transferred. For example, the transgenic plant with the expression cassette can serve as a male or female parent in a cross pollination to produce offspring plants, wherein by receiving the transgene from the donor plant, the offspring plants have the expression cassette.

### Protoplast Fusion

In a method for producing plants having the expression cassette, protoplast fusion can also be used for the transfer of the transgene from a donor plant to a recipient plant. Protoplast fusion is an induced or spontaneous union, such as a somatic hybridization, between two or more protoplasts (cells in which the cell walls are removed by enzymatic treatment) to produce a single bi- or multi-nucleate cell. The fused cell, which may be obtained with plant species that cannot be interbred in nature, is tissue cultured into a hybrid plant exhibiting the desirable combination of traits. More specifically, a first protoplast can be obtained from a plant having the expression cassette. A second protoplast can be obtained from a second plant line, optionally from another plant species or variety, preferably from the same plant species or variety, that comprises commercially desirable characteristics, such as, but not limited to disease resistance, insect resistance, valuable grain characteristics (e.g., increased seed number, see weight and/or seed size) etc. The protoplasts are then fused using traditional protoplast fusion procedures, which are known in the art to produce the cross.

### Embryo Rescue

Alternatively, embryo rescue may be employed in the transfer of the expression cassette from a donor plant to a recipient plant. Embryo rescue can be used as a procedure to isolate embryos from crosses wherein plants fail to produce viable seed. In this process, the fertilized ovary or immature seed of a plant is tissue cultured to create new plants (see Pierik, 1999, In vitro culture of higher plants, Springer, ISBN 079235267x, 9780792352679, which is incorporated herein by reference in its entirety).

In some embodiments, the recipient plant is an elite line having one or more certain agronomically important traits. Examples of agronomically important traits include but are not limited to those that result in increased biomass production, production of specific biofuels, increased food production, improved food quality, increased seed oil content, etc. Additional examples of agronomically important traits includes pest resistance, vigor, development time (time to harvest), enhanced nutrient content, novel growth patterns, flavors or colors, salt, heat, drought and cold tolerance, and the like. Agronomically important traits do not include selectable marker genes (e.g., genes encoding herbicide or antibiotic resistance used only to facilitate detection or selection of transformed cells), hormone biosynthesis genes leading to the production of a plant hormone (e.g., auxins, gibberellins, cytokinins, abscisic acid and ethylene that are used only for selection), or reporter genes (e.g. luciferase, β-glucuronidase, chloramphenicol acetyl transferase (CAT, etc.). For example, the recipient plant can be a plant with increased seed weight and/or seed size which is due to a trait not related to the expression cassette in the donor plant. The recipient plant can also be a plant with preferred carbohydrate composition, e.g., composition preferred for nutritional or industrial applications, especially those plants in which the preferred composition is present in seeds.

### Molecular Markers

In some embodiments, molecular markers are designed and made, based on the promoters or the genes of interest of the present application. In some embodiments, the molecular markers are selected from Isozyme Electrophoresis, Restriction Fragment Length Polymorphisms (RFLPs), Randomly Amplified Polymorphic DNAs (RAPDs), Arbitrarily Primed Polymerase Chain Reaction (AP-PCR), DNA Amplification Fingerprinting (DAF), Sequence Characterized Amplified Regions (SCARs). Amplified Fragment Length Polymorphisms (AFLPs), and Simple Sequence Repeats (SSRs) which are also referred to as Microsatellites, etc. Methods of developing molecular markers and their applications are described by Avise (Molecular markers, natural history, and evolution, Publisher: Sinauer Associates, 2004, ISBN 0878930418, 9780878930418), Srivastava et al.(Plant biotechnology and molecular markers, Publisher: Springer, 2004, ISBN1402019114, 9781402019111), and Vienne (Molecular markers in plant genetics and biotechnology, Publisher: Science Publishers, 2003), each of which is incorporated by reference in its entirety.

The molecular markers can be used in molecular marker assisted breeding. For example, the molecular markers can be utilized to monitor the transfer of the genetic material. In some embodiments, the transferred genetic material is a gene of interest, such as genes that contribute to one or more favorable agronomic phenotypes when expressed in a plant cell, a plant part, or a plant.

### Plant Transformation

The expression cassettes of the present disclosure can be transformed into a plant. The most common method for the introduction of new genetic material into a plant genome involves the use of living cells of the bacterial pathogen *Agrobacterium tumefaciens* to literally inject a piece of DNA, called transfer or T-DNA, into individual plant cells (usually following wounding of the tissue) where it is targeted to the plant nucleus for chromosomal integration.

*Agrobacterium tumefaciens* is a naturally occurring bacterium that is capable of inserting its DNA (genetic information) into plants, resulting in a type of injury to the plant known as crown gall. Most species of plants can now be transformed using this method, including cucurbitaceous species.

There are numerous patents governing *Agrobacterium* mediated transformation and particular DNA delivery plasmids designed specifically for use with *Agrobacterium---*for example, US4536475, EP0265556, EP0270822, WO8504899, WO8603516, US5591616, EP0604662, EP0672752, WO8603776, WO9209696, WO9419930, WO9967357, US4399216, WO8303259, US5731179, EP068730, WO9516031, US5693512, US6051757 and EP904362A1. *Agrobacterium-mediated* plant transformation involves as a first step the placement of DNA fragments cloned on plasmids into living *Agrobacterium* cells, which are then subsequently used for transformation into individual plant cells. *Agrobacterium-*mediated plant transformation is thus an indirect plant transformation method. Methods of *Agrobacterium-mediated* plant transformation that involve using vectors with no T-DNA are also well known to those skilled in the art and can have applicability in the present disclosure. *See,* for example, U.S. Patent No. 7,250,554, which utilizes P-DNA instead of T-DNA in the transformation vector.

A transgenic plant formed using *Agrobacterium* transformation methods typically contains a single gene on one chromosome, although multiple copies are possible. Such transgenic plants can be referred to as being hemizygous for the added gene. A more accurate name for such a plant is an independent segregant, because each transformed plant represents a unique T-DNA integration event (U.S. Patent No. 6,156,953). A transgene locus is generally characterized by the presence and/or absence of the transgene. A heterozygous genotype in which one allele corresponds to the absence of the transgene is also designated hemizygous (U.S. Patent No. 6,008,437).

General transformation methods, and specific methods for transforming certain plant species (e.g., maize) are described in U.S. Patent Nos.: 4940838, 5464763, 5149645, 5501967, 6265638, 4693976, 5635381, 5731179, 5693512, 6162965, 5693512, 5981840, 6420630, 6919494, 6329571, 6215051, 6369298, 5169770, 5376543, 5416011, 5569834, 5824877, 5959179, 5563055, and 5968830, each of which is incorporated herein by reference in its entirety.

In some embodiments, the expression cassettes can be introduced into an expression vector suitable for corn transformation, such as the vectors described by Sidorov and Duncan, 2008 (Agrobacterium-Mediated Maize Transformation: Immature Embryos Versus Callus, Methods in Molecular Biology, 526:47-58), Frame et al., 2002 (Agrobacterium tumefaciens-Mediated Transformation of Maize Embryos Using a Standard Binary Vector System, Plant Physiology, May 2002, Vol. 129, pp. 13-22), Ahmadabadi et al., 2007 (A leaf-based regeneration and transformation system for maize (Zea mays L.), TransgenicRes. 16, 437-448), U.S. Patent Nos. 6,420,630, 6,919,494 and 7,682,829, or similar experimental procedures well known to those skilled in the art. Each of the references above is incorporated herein by reference in its entirety.

### Direct Plant Transformation

Direct plant transformation methods using DNA have also been reported. The first of these to be reported historically is electroporation, which utilizes an electrical current applied to a solution containing plant cells (M. E. Fromm et al., Nature, 319, 791 (1986); H. Jones et al., Plant Mol. Biol., 13, 501 (1989) and H. Yang et al., Plant Cell Reports, 7, 421 (1988).

Another direct method, called "biolistic bombardment", uses ultrafine particles, usually tungsten or gold, that are coated with DNA and then sprayed onto the surface of a plant tissue with sufficient force to cause the particles to penetrate plant cells, including the thick cell wall, membrane and nuclear envelope, but without killing at least some of them (US 5,204,253, US 5,015,580).

A third direct method uses fibrous forms of metal or ceramic consisting of sharp, porous or hollow needle-like projections that literally impale the cells, and also the nuclear envelope of cells. Both silicon carbide and aluminum borate whiskers have been used for plant transformation (Mizuno *et al.,* 2004; Petolino *et al.,* 2000; US5302523 US Application 20040197909) and also for bacterial and animal transformation (Kaepler *et al.,* 1992; Raloff, 1990; Wang, 1995). There are other methods reported, and undoubtedly, additional methods will be developed.

However, the efficiencies of each of these indirect or direct methods in introducing foreign DNA into plant cells are invariably extremely low, making it necessary to use some method for selection of only those cells that have been transformed, and further, allowing growth and regeneration into plants of only those cells that have been transformed.

### Positive Transformed Plant Selection

For efficient plant transformation, a selection method must be employed such that whole plants are regenerated from a single transformed cell and every cell of the transformed plant carries the DNA of interest. These methods can employ positive selection, whereby a foreign gene is supplied to a plant cell that allows it to utilize a substrate present in the medium that it otherwise could not use, such as mannose or xylose (for example, refer US 5767378; US 5994629).

### Negative Transformed Plant Selection

More typically, however, negative selection is used because it is more efficient, utilizing selective agents such as herbicides or antibiotics that either kill or inhibit the growth of nontransformed plant cells and reducing the possibility of chimeras. Resistance genes that are effective against negative selective agents are provided on the introduced foreign DNA used for the plant transformation. For example, one of the most popular selective agents used is the antibiotic kanamycin, together with the resistance gene neomycin phosphotransferase (nptII), which confers resistance to kanamycin and related antibiotics (see, for example, Messing & Vierra, Gene 19: 259-268 (1982); Bevan et al., Nature 304:184-187 (1983)). However, many different antibiotics and antibiotic resistance genes can be used for transformation purposes (refer US 5034322, US 6174724 and US 6255560). In addition, several herbicides and herbicide resistance genes have been used for transformation purposes, including the bar gene, which confers resistance to the herbicide phosphinothricin (White et al., Nucl Acids Res 18: 1062 (1990), Spencer et al., Theor Appl Genet 79: 625-631(1990), US 4795855, US 5378824 and US 6107549). In addition, the dhfr gene, which confers resistance to the anticancer agent methotrexate, has been used for selection (Bourouis et al., EMBO J. 2(7): 1099-1104 (1983).

### Homologous Recombination

Genes can be introduced in a site directed fashion using homologous recombination. Homologous recombination permits site specific modifications in endogenous genes and thus inherited or acquired mutations may be corrected, and/or novel alterations may be engineered into the genome. Homologous recombination and site-directed integration in plants are discussed in, for example, U.S. Patent Nos. 5,451,513; 5,501,967 and 5,527,695.

Methods of producing transgenic plants are well known to those of ordinary skill in the art. Transgenic plants can now be produced by a variety of different transformation methods including, but not limited to, electroporation; microinjection; microprojectile bombardment, also known as particle acceleration or biolistic bombardment; viral-mediated transformation; and Agrobacterium-mediated transformation. *See,* for example, U.S. Patent Nos. 5,405,765; 5,472,869; 5,538,877; 5,538,880; 5,550,318; 5,641,664; 5,736,369 and 5,736,369; International Patent Application Publication Nos. WO2002/038779 and WO/2009/117555; Lu et al., (Plant Cell Reports, 2008, 27:273-278); Watson et al., Recombinant DNA, Scientific American Books (1992); Hinchee et al., Bio/Tech. 6:915-922 (1988); McCabe et al., Bio/Tech. 6:923-926 (1988); Toriyama et al., Bio/Tech. 6:1072-1074 (1988); Fromm et al., Bio/Tech. 8:833-839 (1990); Mullins et al., Bio/Tech. 8:833-839 (1990); Hiei et al., Plant Molecular Biology 35:205-218 (1997); Ishida et al., Nature Biotechnology 14:745-750 (1996); Zhang et al., Molecular Biotechnology 8:223-231 (1997); Ku et al., Nature Biotechnology 17:76-80 (1999); and, Raineri et al., Bio/Tech. 8:33-38 (1990)), each of which is expressly incorporated herein by reference in their entirety.

### Biolistic Bombardment

Microprojectile bombardment is also known as particle acceleration, biolistic bombardment, and the gene gun (Biolistic^{®} Gene Gun). The gene gun is used to shoot pellets that are coated with genes (e.g., for desired traits) into plant seeds or plant tissues in order to get the plant cells to then express the new genes. The gene gun uses an actual explosive (.22 caliber blank) to propel the material. Compressed air or steam may also be used as the propellant. The Biolistic^{®} Gene Gun was invented in 1983-1984 at Cornell University by John Sanford, Edward Wolf, and Nelson Allen. It and its registered trademark are now owned by E. I. du Pont de Nemours and Company. Most species of plants have been transformed using this method.

In one aspect, the disclosure relates to a method for identifying one or more microorganisms capable of imparting one or more "beneficial property to a plant." It should be appreciated that as referred to herein a "beneficial property to a plant" should be interpreted broadly to mean any property which is beneficial for any particular purpose including properties which may be beneficial to human beings, other animals, the environment, a habitat, an ecosystem, the economy, of commercial benefit, or of any other benefit to any entity or system. Accordingly, the term should be taken to include properties which may suppress, decrease or block one or more characteristic of a plant, including suppressing, decreasing or inhibiting the growth or growth rate of a plant. The disclosure may be described herein, by way of example only, in terms of identifying positive benefits to one or more plants or improving plants. However, it should be appreciated that the disclosure is equally applicable to identifying negative benefits that can be conferred to plants. Such beneficial properties include, but are not limited to, for example: improved growth, health and/or survival characteristics, resistance to pests and/or diseases, tolerance to growth in different geographical locations and/or different environmental biological and/or physical conditions, suitability or quality of a plant for a particular purpose, structure, color, chemical composition or profile, taste, smell, improved quality. By way of example, the disclosure may allow for the identification of microorganisms which allow a plant to grow in a variety of different temperatures (including extreme temperatures), pH, salt concentrations, mineral concentrations, in the presence of toxins, and/or to respond to a greater extent to the presence of organic and/or inorganic fertilizers.

In other embodiments, beneficial properties include, but are not limited to, for example; decreasing, suppressing, or inhibiting the growth of a plant identified to be a weed; constraining the height and width of a plant to a desirable ornamental size; limiting the height of plants used in ground cover applications such as motorway and roadside banks and erosion control projects; slowing the growth of plants used in turf applications such as lawns, bowling greens and golf courses to reduce the necessity of mowing; reducing ratio of foliage/flowers in ornamental flowering shrubs; regulate production of and/or response to plant pheromones (resulting in increased tannin production in surrounding plant community and decreased appeal to foraging species).

In certain embodiments, methods of the disclosure relate to selecting one or more microorganisms which are capable of imparting one or more beneficial property to a plant. As is further described herein, such microorganisms may be contained within a plant, on a plant, and/or within the plant rhizosphere. Accordingly, where reference is made herein to acquiring a second set of one or more microorganisms "from" a plant, unless the context requires otherwise, it should be taken to include reference to acquiring a second set of microorganisms contained within a plant, on a plant, within the plant rhizosphere, or from within the area from which the plant is growing, e.g. growth media, soil adjacent the plant, etc. For ease of reference, the wording "associated with" may be used synonymously to refer to microorganisms contained within a plant, on a plant, and/or within the plant rhizosphere.

### Microorganisms in Plant Breeding

The inventors have found that one can readily identify microorganisms capable of imparting one or more beneficial property to one or more plants through use of a method of the disclosure. The method is broadly based on the presence of variability (e.g., genetic variability, or variability in the phenotype) in the plants and microbial populations used. The inventors have identified that this variability can be used to support a directed process of selection of one or more microorganisms of use to a plant and for identifying particular plant/microbe combinations which are of benefit for a particular purpose, and which may never have been recognized using conventional techniques.

In some embodiments, the plant microbe combination of the present disclosure is a combination between a particular plant species and one, or a combination of microorganisms. In other embodiments the plant microbe combination of the present disclosure is a combination between a plant with a particular genotype and one, or a combination of microorganisms. In some embodiments, the benefits of a particular plant/microorganism combination may be specific to certain environmental conditions (e.g., drought conditions, or aluminum toxicity), or for specific desired phenotypes (e.g., fruit flavor). Thus in some embodiments of the present disclosure, a plant's phenotype is a consequence of a plant's genotype, environment, symbiont microflora, and synergistic effects therefrom.

In some embodiments, the methods of the disclosure may be used as a part of a plant breeding program. The methods may allow for, or at least assist with, the selection of plants which have a particular genotype/phenotype which is influenced by the microbial flora, in addition to identifying microorganisms and/or compositions that are capable of imparting one or more property to one or more plants.

In some embodiments, the present disclosure teaches methods of reducing the environmental variability in current plant breeding programs by providing a uniform microbial consortium. In some embodiments, a microbial consortium optimized for a particular species or environment is used during the breeding process of a new plant variety. In some embodiments, this strategy can be used to select for plants that tolerate extreme environments (e.g., salty, acid, dry, soils). This approach is based in part on the present discovery that certain environments have a microbial flora that is vastly different from that found in normal plant growth media used in plant breeding. Thus, the methods of the present disclosure improve the plant breeding process by conducting selections with a microflora that reflects their ultimate growing environment.

In other embodiments, the present disclosure teaches methods of incorporating microflora diversity into plant breeding methods to simulate expected field environmental variability. In some embodiments, expected field microbial combinations are identified and applied to plant breeding in order to replicate field conditions. In some embodiments this will lead to plant varieties with higher resistance to environmental variability for higher crop consistencies in pre-field screenings.

In other embodiments, the methods of the disclosure are useful for improving the efficiency of crop breeding programs through the use of directed selection of crop-associated microbes that influence phenotypic traits under the control of quantitative trait loci (QTLs). The methods may indirectly manipulate the expression of crop QTLs that control the heritable variability of the traits and physiological mechanisms underlying desirable traits such as biomass compartmentalization, abiotic stress tolerance, resistance to pest and diseases and nutrient assimilation.

Methods of the disclosure may be used to assist in improving plants by identifying microorganisms that optimize the expression of desirable plant genes or traits. In some embodiments, the methods of the present disclosure can be used to breed plants with better phenotypes utilizing the collective genotype of the plant and its symbiont microflora. That is, the present methods can utilize the concept of the holobiome to capture the entirety of genetic variability associated with a plant and its associated microbial community.

In some embodiments, desirable plant genotype-microbial genotype, i.e. "holobiome," combinations are achieved by selecting from variability in the plant (conventional plant breeding techniques) and variability in the microbiome impacting the plants' phenotype. In some embodiments, the breeding programs of the present disclosure combine traditional plant breeding and selection techniques with directed evolution and section of a plant's holobiome.

### Accelerated Microbial Selection

As aforementioned, the present disclosure provides a method by which to harness the genetic variability associated with the microbial communities associated with plants undergoing a breeding program.

The process by which the microbial communities are manipulated is termed "accelerated microbial selection." This iterative process is extremely effective at identifying and selecting for one or more microorganisms associated with imparting a desirable phenotypic trait upon a plant.

The accelerated microbial selection process is described in, for example: (1) International Application No. PCT/NZ2012/000041, filed on March 16, 2012, published as WO 2012/125050, on September 20, 2012, and claiming priority to New Zealand Application No. 588048, filed on March 17, 2011; and associated U.S. National Stage Application No. 14/005,383, filed on March 16, 2012; (2) International Application NO. PCT/NZ2013/000171, filed on September 19, 2013, published as WO 2014/046553, on March 27, 2014 , and claiming priority to New Zealand Application No. 602352, filed on September 19, 2012; (3) U.S. Application No. 14/218,920, filed on March 18, 2014, claiming priority as a Continuation-in-Part Application to International Application No. PCT/NZ2013/000171; (4) U.S. Application No. 14/050,788, filed on October 10, 2013, and claiming priority to U.S. Application No. 14/031,461, filed on September 19, 2013, claiming priority to New Zealand Application No. 602534, filed on September 19, 2012; (5) U.S. Application No. 14/050,876, filed on October 10, 2013, claiming priority to U.S. Application No. 14/031, 511, filed on September 19, 2013, claiming priority to New Zealand Application No. 602533, filed September 19, 2012; (6) International Application No. PCT/NZ2014/000044, filed on March 19, 2014; and (7) International Application No. PCT/NZ2014/000045, filed on March 19, 2014. Each of the aforementioned references is incorporated herein by reference in their entireties for all purposes.

In one embodiment, the accelerated microbial selection process involves: a) subjecting one or more plant (including for example seeds, seedlings, cuttings, and/or propagules thereof) to a growth medium in the presence of a first set of one or more microorganisms; b) selecting one or more plant following step a); c) acquiring a second set of one or more microorganisms associated with said one or more plant selected in step b) or plant growth media; d) repeating steps a) to c) one or more times, wherein the second set of one or more microorganisms acquired in step c) is used as the first set of microorganisms in step a) of any successive repeat.

In one embodiment, the one or more plant is selected (step b) on the basis of one or more selection criterion.

In one embodiment, the one or more plant is selected on the basis of one or more phenotypic trait. In one embodiment, the one or more plant is selected based on the presence of a desirable phenotypic trait. In one embodiment, the phenotypic trait is one of those detailed herein after.

In one embodiment, the one or more plant is selected on the basis of one or more genotypic trait. In one embodiment, the one or more plant is selected based on the presence of a desirable genotypic trait

In one embodiment, the one or more plant is selected based on a combination of one or more genotypic and one or more phenotypic traits. In one embodiment, different selection criteria may be used in different iterations of a method of the disclosure.

In one embodiment, the second set of one or more microorganisms (step c) are isolated from the root, stem and/or foliar (including reproductive) tissue of the one or more plants selected. Alternatively, the second set of one or more microorganisms are isolated from whole plant tissue of the one or more plants selected. In another embodiment, the plant tissues may be surface sterilized and then one or more microorganisms isolated from any tissue of the one or more plants. This embodiment allows for the targeted selection of endophytic microorganisms. In another embodiment, the second set of one or more microorganisms may be isolated from the growth medium surrounding selected plants. In another embodiment, the second set of one or more microorganisms are acquired in crude form.

In one embodiment, the one or more microorganisms are acquired in step c) any time after germination.

In one embodiment, where two or more microorganisms are acquired in step c), the method further comprises the steps of separating the two or more microorganisms into individual isolates, selecting two or more individual isolates, and then combining the selected two or more isolates.

In another embodiment, the method further comprises repeating steps a) to c) one or more times, wherein where two or more microorganisms are acquired in step c), the two or more microorganisms are separated into individual isolates, two or more individual isolates are selected and then combined, and the combined isolates are used as the first set of one or more microorganism in step a) of the successive repeat. Accordingly, where reference is made to using the one or more microorganisms acquired in step c) in step a) of the method, it should be taken to include using the combined isolates of this embodiment of the disclosure.

In another embodiment, two or more methods of the disclosure may be performed separately and the second set of one or more microorganisms acquired in step c) of each separate method combined. In one embodiment, the combined microorganisms are used as the first set of one or more microorganisms in step a) of any successive repeat of the method of the disclosure.

In one embodiment, the methods of the first aspect of the disclosure may also be useful in identifying and/or selecting one or more endophytic microorganism capable of imparting one or more beneficial property to a plant.

In one embodiment, plant material (including for example seeds, seedlings, cuttings, and/or propagules thereof) may be used as the source of microorganisms for step a). In an embodiment, the plant material used as a source for microorganisms in step a) is seed material. The plant material may be surface sterilized.

In one embodiment of the present disclosure, the disclosed compositions and methods provide a uniform background microbiome derived from an initial set of AMS-identified microbes (using plant parental lines) for each breeding cycle. In other embodiments, two or more initial sets of microbes can be combined or tested separately during the breeding methods of the present disclosure.

In some embodiments, the initial set of microorganisms is obtained from a previously conducted AMS procedure. For example, in some embodiments, the disclosure teaches that microorganisms can undergo one or more rounds of selection using a standardized plant variety in order to develop an initial set of microorganisms for breeding.

In other embodiments, the initial set of microorganisms is obtained from the soil of a field, pond, beach, garden, or other arable land source. In some embodiments, the present disclosure teaches that the initial set of microorganisms does not include pathogenic soil. For example, in some embodiments, the present disclosure does not utilize soil infested with *Fusarium oxysporum, Fusarium solani, Aphanomyces, Pythium ultimum, Macrophomina phaseolina, Rhizoctonia solani, Sclerotinia sclerotiorum,* or *Sclerotium rolfsii.*

In some embodiments, the microorganisms used for the breeding methods of the present disclosure are commercial microbial products. For example, in some embodiments, the present disclosure teaches the use of one or more commercially available microbial products to provide the uniform microbiome found in the plant breeding methods taught herein. A non-exclusive list of the microbial products compatible with the present disclosure include: Nutri-Life 4/20^{™}, Nutri-Life Bio-N^{™}, Nutri-Life Bio-Plex^{™}, Nutri-Life Platform^{™}, Nutri-Life Sudo-Shield^{™}, Nutri-Life B Sub^{™}, Nutri-Life Bio-P^{™}, Nutri-Life Bio-P^{™}, Nutri-Life Myco-Force^{™}, Nutri-Root-Guard^{™}, Nutri-Life Tricho-Shield^{™}, Ag + Humus^{™} inoculant, Ag Select^{™} inoculant, Lawn and Garden^{™} inoculant, Turf^{™} inoculant, Water Doctor^{™} inoculant, SCD Probiotics Soil Enrichment^{™}, All Seasons Bokashi^{™}, SCD Bio Ag^{®}, ProBio Balance^{™}, and VOTiVO^{™}. In some embodiments, a person skilled in the art will recognize that many other commercial microbial products can be used to provide for, and control, the microbial variability during the improved plant breeding methods taught herein.

In some embodiments, the present disclosure teaches that the initial microbes for the plant breeding methods described herein exclude biopestides, microbial control agents, or other disease control microbes.

A non-exclusive list of the microbial products which are not used with the present disclosure, in certain embodiments, include: Bactur^{®}, Bactospeine^{®}, Bioworm^{®}, Caterpillar Killer^{®}, Dipel^{®}, Futura^{®}, Javelin^{®}, SOKBt^{®}, Thuricide^{®}, Topside^{®}, Tribactur^{®}, Worthy Attack^{®}, Aquabee^{®}, Bactimos^{®}, Gnatrol^{®}, LarvX^{®}, Mosquito Attack^{®}, Skeetal^{®}, Teknar^{®}, Vectobac^{®}, Foil^{®} M-One^{®} M-Track^{®}, Novardo^{®}, Trident^{®}, Certan^{®}, Doom^{®}, Japidemic^{®}, Grub Attack^{®}, Vectolex CG^{®}, Vectolex WDG^{®}, Botanigard^{®}, Mycotrol^{®}, Naturalis^{®}, Laginex^{®}, NOLO Bait^{®}, Grasshopper Attack^{®}, Gypchek^{®} virus, TM Biocontrol-1^{®}, Neochek-S^{®}, Biosafe^{®}, Ecomask^{®}, Scanmask^{®}, Vector^{®}, Nematac^{®}.

In another embodiment, the methods of the disclosure may be useful in identifying and/or selecting one or more unculturable microorganisms capable of imparting one or more beneficial property to a plant. In this embodiment, plant material (including for example seeds, seedlings, cuttings, and/or propagules thereof) may be used as the source of microorganisms for step a). In an embodiment, the plant material used as a source for microorganisms in step a) is explant material (for example, plant cuttings). The plant material may be surface sterilized.

In some embodiments, the accelerated microbial selection methods involve a selective pressure step.

Thus, in an embodiment, the accelerated microbial selection process involves: a) subjecting one or more plant (including for example seeds, seedlings, cuttings, and/or propagules thereof) to a growth medium in the presence of a first set of one or more microorganisms; b) applying one or more selective pressures during step a); c) selecting one or more plant following step b); d) acquiring a second set of one or more microorganisms associated with said one or more plant selected in step c); e) repeating steps a) to d) one or more times, wherein the second set of one or more microorganisms acquired in step d) is used as the first set of microorganisms in step a) of any successive repeat.

In one embodiment, the one or more selective pressures applied in successive repeats of steps a) to d) is different In another embodiment, the one or more selective pressures applied in successive repeats of steps a) to d) is the same.

In one embodiment, one selective pressure is applied in step b). In another embodiment two or more selective pressures are applied in step b).

In one embodiment, the selective pressure is biotic and includes, but is not limited to, exposure to one or more organisms that are detrimental to the plant. In one embodiment, the organisms include fungi, bacteria, viruses, insects, mites and nematodes.

In another embodiment, the selective pressure is abiotic. Abiotic selective pressures include, but are not limited to, exposure to or changes in the level of salt concentration, temperature, pH, water, minerals, organic nutrients, inorganic nutrients, organic toxins, inorganic toxins, and metals.

Other abiotic pressures include active chemical agents. In specific embodiments, the abiotic pressure includes active agricultural chemical agents.

In one embodiment, the selective pressure is applied during substantially the whole time during which the one or more plant is subjected to the growth medium and one or more microorganisms. In one embodiment, the selective pressure is applied during substantially the whole growth period of the one or more plant. Alternatively, the selective pressure is applied at a discrete time point.

In some embodiments, the improved plant breeding methods of the present disclosure include an optional step of identifying the consortia of microorganisms associated with any plant. For example, in some embodiments, the present disclosure teaches the identification of microbial consortia that are associated with a specific plant phenotype identified at any of the plant breeding steps described herein (e.g. F1, F2, F3, etc). In other embodiments, the present disclosure teaches the identification of microbial consortia identified at the end of the breeding program. In some embodiments, only the best performing consortia are analysed. In other embodiments, medium or low performing consortia are also analysed.

In some embodiments, the present disclosure teaches a variety of molecular methods of identifying microbial consortia. For example, in some embodiments, the present disclosure teaches the use of in-situ detection techniques such as fluorescence in situ hybridization (FISH), antibody fluorescence, and/or microscopy (fluorescence, laser confocal, light, SEM or TEM). In other embodiments, the present disclosure teaches the detection of consortia through culturing techniques including selective media, or differential media techniques which identify microorganisms based on their growth properties on various substances.

In other embodiments, the present disclosure teaches methods of identifying microorganisms based on their DNA, RNA, or protein compositions. For example, in some embodiments, the present disclosure teaches the identification of microorganisms through PCR-based detection techniques including PCR, qPCR, RT PCR, and RT qPCR. Thus, for example, microbes can be identified based on the reactivity of DNA or RNA samples to selected primer sets. In other embodiments, the present disclosure teaches the identification or microorganisms through DNA sequencing. Mixed DNA extractions, sequencing, and identifications are possible through next generation sequencing techniques such as Solexa, Roche 454, or Illumina sequencers which allow for large scale sequencing and genome assembly. In some embodiments the present disclosure teaches the identification of microbial consortia based on 16s ribosomal RNA sequence comparisons *(see* Woo et al., 2008 Clinical Microbiology and Infection Oct (10) pgs 908-934). In some embodiments a person having skill in the art will recognize that the present disclosure is compatible with many other DNA and RNA sequencing and analysis technologies.

In some embodiments the DNA analysis of the present disclosure does not require a culturing step, but instead relies on DNA/RNA extracted directly from soil samples *(see* Yeates et al., 1998 Biological Procedures Online Vol 1 1 pgs 40-47; also commercial solutions such as PowerSoil^{™} DNA isolation kit, Norgen Soil DNA Isolation Kit^{™}, Sureprep^{™} Soil DNA isolation kit).

In some embodiments, the present disclosure teaches methods of identifying microorganisms using protein compositions. For example, microbial consortia can be identified through antibody-based protein detection techniques such as Western Blot analysis, or enzyme-linked immunosorbent assay (ELISA). In other embodiments, the proteins of microbial corsortia can be analysed via 1D or 2D gel separations followed by protein staining or immune detection. In some embodiments, the present disclosure teaches the identification of microbial proteins through Matrix-assisted laser desorption ionization time of flight mass spectrometry (MALDI-TOF), which allows for the rapid identification of various proteins unique to a particular organism *(see* Dingle and Butler-WU, 2013 Clinics in Laboratory Medicine 3 pgs 589-609).

Thus, any method of identifying the microbial community can be employed. The microbial community can be identified directly or it can be identified indirectly by ascertainment of certain protein or exudate signatures. Regardless of the identification method, the present disclosure accounts for, and controls, the microbial variability present in the plant breeding process.

### Microorganisms

As used herein the term "microorganism" should be taken broadly. It includes but is not limited to the two prokaryotic domains, Bacteria and Archaea, as well as eukaryotic fungi and protists. By way of example, the microorganisms may include Proteobacteria (such as *Pseudomonas, Enterobacter, Stenotrophomonas, Burkholderia, Rhizobium, Herbaspirillum, Pantoea, Serratia, Rahnella, Azospirillum, Azorhizobium, Azotobacter, Duganella, Delftia, Bradyrhizobiun, Sinorhizobium* and *Halomonas),* Firmicutes (such as *Bacillus, Paenibacillus, Lactobacillus, Mycoplasma,* and *Acetobacterium* ), Actinobacteria (such as *Streptomyces, Rhodococcus, Microbacterium,* and *Curtobacterium),* and the fungi Ascomycota (such as *Trichoderma, Ampelomyces, Coniothyrium, Paecoelomyces, Penicillium, Cladosporium, Hypocrea, Beauveria, Metarhizium, Verticullium, Cordyceps, Pichea,* and *Candida,* Basidiomycota (such as *Coprinus, Corticium,* and *Agaricus)* and Oomycota (such as *Pythium, Mucor,* and *Mortierella).*

In a particular embodiment, the microorganism is an endophyte or an epiphyte or a microorganism inhabiting the plant rhizosphere or rhizosheath. That is, the microorganism may be found present in the soil material adhered to the roots of a plant or in the area immediately adjacent a plant's roots. In one embodiment, the microorganism is a seed-borne endophyte.

In certain embodiments, the microorganism is unculturable. This should be taken to mean that the microorganism is not known to be culturable or is difficult to culture using methods known to one skilled in the art.

Microorganisms of use in the methods of the present disclosure (for example, as the first set of one or more microorganisms) may be collected or obtained from any source or contained within and/or associated with material collected from any source.

In one embodiment, the first set of one or more microorganisms are obtained from any general terrestrial environment, including its soils, plants, fungi, animals (including invertebrates) and other biota, including the sediments, water and biota of lakes and rivers; from the marine environment, its biota and sediments (for example sea water, marine muds, marine plants, marine invertebrates (for example sponges), marine vertebrates (for example, fish)); the terrestrial and marine geosphere (regolith and rock, for example crushed subterranean rocks, sand and clays); the cryosphere and its meltwater; the atmosphere (for example, filtered aerial dusts, cloud and rain droplets); urban, industrial and other man-made environments (for example, accumulated organic and mineral matter on concrete, roadside gutters, roof surfaces, road surfaces).

In another embodiment the first set of one or more microorganisms are collected from a source likely to favor the selection of appropriate microorganisms. By way of example, the source may be a particular environment in which it is desirable for other plants to grow, or which is thought to be associated with terroir. In another example, the source may be a plant having one or more desirable traits, for example a plant which naturally grows in a particular environment or under certain conditions of interest. By way of example, a certain plant may naturally grow in sandy soil or sand of high salinity, or under extreme temperatures, or with little water, or it may be resistant to certain pests or disease present in the environment, and it may be desirable for a commercial crop to be grown in such conditions, particularly if they are, for example, the only conditions available in a particular geographic location. By way of further example, the microorganisms may be collected from commercial crops grown in such environments, or more specifically from individual crop plants best displaying a trait of interest amongst a crop grown in any specific environment, for example the fastest-growing plants amongst a crop grown in saline-limiting soils, or the least damaged plants in crops exposed to severe insect damage or disease epidemic, or plants having desired quantities of certain metabolites and other compounds, including fiber content, oil content, and the like, or plants displaying desirable *colors,* taste or smell. The microorganisms may be collected from a plant of interest or any material occurring in the environment of interest, including fungi and other animal and plant biota, soil, water, sediments, and other elements of the environment as referred to previously.

In certain embodiments, the microorganisms are sourced from previously performed methods of the disclosure (for example, the microorganisms acquired from prior selections, or collected from various loci/environmental conditions), including combinations of individual isolates separated different environments or combinations of microorganisms resulting from two or more separately performed methods of the disclosure.

While the disclosure obviates the need for pre-existing knowledge about a microorganism's desirable properties with respect to a particular plant species, in one embodiment a microorganism or a combination of microorganisms of use in the methods of the disclosure may be selected from a pre-existing collection of individual microbial species or strains based on some knowledge of their likely or predicted benefit to a plant. For example, the microorganism may be predicted to: improve nitrogen fixation; release phosphate from the soil organic matter; release phosphate from the inorganic forms of phosphate (e.g. rock phosphate); "fix carbon" in the root microsphere; live in the rhizosphere of the plant thereby assisting the plant in absorbing nutrients from the surrounding soil and then providing these more readily to the plant; increase the number of nodules on the plant roots and thereby increase the number of symbiotic nitrogen fixing bacteria (e.g. *Rhizobium* species) per plant and the amount of nitrogen fixed by the plant; elicit plant defensive responses such as ISR (induced systemic resistance) or SAR (systemic acquired resistance) which help the plant resist the invasion and spread of pathogenic microorganisms; compete with microorganisms deleterious to plant growth or health by antagonism, or competitive utilization of resources such as nutrients or space; change the *color* of one or more part of the plant, or change the chemical profile of the plant, its smell, taste or one or more other quality.

In one embodiment a microorganism or combination of microorganisms (the first set of one or more microorganisms) is selected from a pre-existing collection of individual microbial species or strains that provides no knowledge of their likely or predicted benefit to a plant. For example, a collection of unidentified microorganisms isolated from plant tissues without any knowledge of their ability to improve plant growth or health, or a collection of microorganisms collected to explore their potential for producing compounds that could lead to the development of pharmaceutical drugs.

In one embodiment, the microorganisms are acquired from the source material (for example, soil, rock, water, air, dust, plant or other organism) in which they naturally reside. The microorganisms may be provided in any appropriate form, having regard to its intended use in the methods of the disclosure. However, by way of example only, the microorganisms may be provided as an aqueous suspension, gel, homogenate, granule, powder, slurry, live organism or dried material. The microorganisms may be isolated in substantially pure or mixed cultures. They may be concentrated, diluted or provided in the natural concentrations in which they are found in the source material. For example, microorganisms from saline sediments may be isolated for use in this disclosure by suspending the sediment in fresh water and allowing the sediment to fall to the bottom. The water containing the bulk of the microorganisms may be removed by decantation after a suitable period of settling and either applied directly to the plant growth medium, or concentrated by filtering or centrifugation, diluted to an appropriate concentration and applied to the plant growth medium with the bulk of the salt removed. By way of further example, microorganisms from mineralized or toxic sources may be similarly treated to recover the microbes for application to the plant growth material to minimize the potential for damage to the plant.

In another embodiment, the microorganisms are used in a crude form, in which they are not isolated from the source material in which they naturally reside. For example, the microorganisms are provided in combination with the source material in which they reside; for example, as soil, or the roots, seed or foliage of a plant. In this embodiment, the source material may include one or more species of microorganisms.

In some embodiments, a mixed population of microorganisms is used in the methods of the disclosure.

In embodiments of the disclosure where the microorganisms are isolated from a source material (for example, the material in which they naturally reside), any one or a combination of a number of standard techniques which will be readily known to skilled persons may be used.

However, by way of example, these in general employ processes by which a solid or liquid culture of a single microorganism can be obtained in a substantially pure form, usually by physical separation on the surface of a solid microbial growth medium or by volumetric dilutive isolation into a liquid microbial growth medium. These processes may include isolation from dry material, liquid suspension, slurries or homogenates in which the material is spread in a thin layer over an appropriate solid gel growth medium, or serial dilutions of the material made into a sterile medium and inoculated into liquid or solid culture media.

Whilst not essential, in one embodiment, the material containing the microorganisms may be pre-treated prior to the isolation process in order to either multiply all microorganisms in the material, or select portions of the microbial population, either by enriching the material with microbial nutrients (for example, nitrates, sugars, or vegetable, microbial or animal extracts), or by applying a means of ensuring the selective survival of only a portion of the microbial diversity within the material (for example, by pasteurizing the sample at 60°C-80°C for 10 -20 minutes to select for microorganisms resistant to heat exposure (for example, bacilli), or by exposing the sample to low concentrations of an organic solvent or sterilant (for example, 25% ethanol for 10 minutes) to enhance the survival of actinomycetes and spore-forming or solvent-resistant microorganisms). Microorganisms can then be isolated from the enriched materials or materials treated for selective survival, as above.

In an embodiment of the disclosure endophytic or epiphytic microorganisms are isolated from plant material. Any number of standard techniques known in the art may be used and the microorganisms may be isolated from any appropriate tissue in the plant, including for example root, stem and leaves, and plant reproductive tissues. By way of example, conventional methods for isolation from plants typically include the sterile excision of the plant material of interest (e.g. root or stem lengths, leaves), surface sterilization with an appropriate solution (e.g. 2% sodium hypochlorite), after which the plant material is placed on nutrient medium for microbial growth (see, for example, Strobel G and Daisy B (2003) Microbiology and Molecular Biology Reviews 67 (4): 491-502; Zinniel DK et al.(2002) Applied and Environmental Microbiology 68 (5): 2198-2208).

In one embodiment of the disclosure, the microorganisms are isolated from root tissue. Further methodology for isolating microorganisms from plant material are detailed hereinafter.

In one embodiment, the microbial population is exposed (prior to the method or at any stage of the method) to a selective pressure to enhance the probability that the eventually selected plants will have microbial assemblages likely to have desired properties. For example, exposure of the microorganisms to pasteurisation before their addition to a plant growth medium (preferably sterile) is likely to enhance the probability that the plants selected for a desired trait will be associated with spore-forming microbes that can more easily survive in adverse conditions, in commercial storage, or if applied to seed as a coating, in an adverse environment.

In certain embodiments, as mentioned herein before, the microorganism(s) may be used in crude form and need not be isolated from a plant or a media. For example, plant material or growth media which includes the microorganisms identified to be of benefit to a selected plant may be obtained and used as a crude source of microorganisms for the next round of the method or as a crude source of microorganisms at the conclusion of the method. For example, whole plant material could be obtained and optionally processed, such as mulched or crushed. Alternatively, individual tissues or parts of selected plants (such as leaves, stems, roots, and seeds) may be separated from the plant and optionally processed, such as mulched or crushed. In certain embodiments, one or more part of a plant which is associated with the second set of one or more microorganisms may be removed from one or more selected plants and, where any successive repeat of the method is to be conducted, grafted on to one or more plant used in any step of the plant breeding methods.

In some aspects, the present methods do not utilize root nodulating bacteria. In some aspects, the methods do not utilize rhizobia. In some aspects, the present methods do not utilize *Rhizobium spp.*

### Plants

Any number of a variety of different plants, including mosses and lichens and algae, may be used in the methods of the disclosure. In embodiments, the plants have economic, social and/or environmental value. For example, the plants may include those of use: as food crops; as fiber crops; as oil crops; in the forestry industry; in the pulp and paper industry; as a feedstock for biofuel production; and/or, as ornamental plants. In other embodiments, the plants may be economically, socially and/or environmentally undesirable, such as weeds. The following is a list of non-limiting examples of the types of plants the methods of the disclosure may be applied to:
**Food crops:**
   *Cereals* (maize, rice, wheat, barley, sorghum, millet, oats, rye, triticale, buckwheat);
   *leafy vegetables* (brassicaceous plants such as cabbages, broccoli, bok choy, rocket; salad greens such as spinach, cress, lettuce);
   *fruiting and flowering vegetables* (e.g. avocado, sweet corn, artichokes, curcubits e.g. squash, cucumbers, melons, courgettes, pumpkins; solanaceous vegetables/fruits e.g. tomatoes, eggplant, capsicums);
   *legumes* (groundnuts, peanuts, peas, soybeans, beans, lentils, chickpea, okra);
   *bulbed and stem vegetables* (asparagus, celery, *Allium* crops e.g garlic, onions, leeks);
   *roots and tuberous vegetables* (carrots, beet, bamboo shoots, cassava, yams, ginger, Jerusalem artichoke, parsnips, radishes, potatoes, sweet potatoes, taro, turnip, wasabi);
   sugar crops including sugar beet *(Beta vulgaris),* sugar cane *(Saccharum officinarum*);
   crops grown for the production of non-alcoholic beverages and stimulants (coffee, black, herbal and green teas, cocoa, tobacco);
   *fruit crops* such as true berry fruits (e.g. kiwifruit, grape, currants, gooseberry, guava, feijoa, pomegranate), citrus fruits (e.g. oranges, lemons, limes, grapefruit), epigynous fruits (e.g. bananas, cranberries, blueberries), aggregate fruit (blackberry, raspberry, boysenberry), multiple fruits (e.g. pineapple, fig), stone fruit crops (e.g. apricot, peach, cherry, plum), pipfruit (e.g. apples, pears) and others such as strawberries, sunflower seeds;
   *culinary and medicinal herbs* e.g. rosemary, basil, bay laurel, coriander, mint, dill, *Hypericum,* foxglove, alovera, rosehips);
   *crop plants producing spices* e.g. black pepper, cumin cinnamon, nutmeg, ginger, cloves, saffron, cardamom, mace, paprika, masalas, star anise;
   *crops grown for the production of nuts* e.g. almonds and walnuts, Brazil nut, cashew nuts, coconuts, chestnut, macadamia nut, pistachio nuts; peanuts, pecan nuts;
   crops grown for production of beers, wines and other alcoholic beverages e.g grapes, hops;
   *oilseed crops* e.g. soybean, peanuts, cotton, olives, sunflower, sesame, lupin species and brassicaeous crops (e.g. canola/oilseed rape); and, *edible fungi* e.g. white mushrooms, Shiitake and oyster mushrooms;
**Plants used in pastoral agriculture:**
   *legumes: Trifolium* species, *Medicago* species, and *Lotus* species; White clover *(T.repens);* Red clover *(T. pratense);* Caucasian clover *(T. ambigum);* subterranean clover *(T.subterraneum);* Alfalfa/Lucerne *(Medicago sativum);* annual medics; barrel medic; black medic; Sainfoin *(Onobrychis viciifolia);* Birdsfoot trefoil *(Lotus corniculatus);* Greater Birdsfoot trefoil *(Lotus pedunculatus);*
   seed legumes/pulses including Peas *(Pisum sativum),* Common bean *(Phaseolus vulgaris),* Broad beans *(Viciafaba),* Mung bean *(Vigna radiata),* Cowpea (*Vigna unguiculata*), Chick pea *(Cicer arietum),* Lupins *(Lupinus species);* Cereals including Maize/corn *(Zea mays),* Sorghum *(Sorghum spp.),* Millet *(Panicum miliaceum, P. sumatrense*), Rice *(Oryza sativa indica, Oryza sativa japonica),* Wheat *(Triticum sativa),* Barley *(Hordeum vulgare),* Rye *(Secale cereale),* Triticale *(Triticum X Secale),* Oats *(Avena fatua);*
   Forage and Amenity grasses: Temperate grasses such as *Lolium* species; *Festuca* species; *Agrostis* spp., Perennial ryegrass *(Lolium perenne);* hybrid ryegrass *(Lolium hybridum*); annual ryegrass *(Lolium multiflorum),* tall fescue *(Festuca arundinacea);* meadow fescue *(Festuca pratensis);* red fescue *(Festuca rubra); Festuca ovina; Festuloliums (Lolium X Festuca crosses);* Cocksfoot *(Dactylis glomerata*); Kentucky bluegrass *Poa pratensis; Poa palustris; Poa nemoralis; Poa trivialis; Poa compresa*; *Bromus* species; *Phalaris (Phleum* species); *Arrhenatherum elatius; Agropyron* species; *Avena strigosa; Setaria italic;*
   *Tropical grasses* such as: *Phalaris* species; *Brachiaria* species; *Eragrostis* species; *Panicum* species; Bahai grass *(Paspalum notatum); Brachypodium* species; and, grasses used for biofuel production such as Switchgrass *(Panicum virgatum)* and *Miscanthus* species;
**Fiber crops:**
   cotton, hemp, jute, coconut, sisal, flax *(Linum spp.),* New Zealand flax *(Phormium spp.*); plantation and natural forest species harvested for paper and engineered wood fiber products such as coniferous and broad leafed forest species;
**Tree and shrub species used in plantation forestry and bio-fuel crops:**
   Pine *(Pinus* species); Fir *(Pseudotsuga* species); Spruce *(Picea* species); Cypress *(Cupressus* species); Wattle *(Acacia* species); Alder *(Alnus* species); Oak species *(Quercus* species); Redwood *(Sequoiadendron* species); willow *(Salix* species); birch *(Betula* species); Cedar *(Cedrus* species); Ash *(Fraxinus* species); Larch *(Larix* species); *Eucalyptus* species; Bamboo (Bambuseae species) and Poplars *(Populus* species).
**Plants grown for conversion to energy, biofuels or industrial products by extractive. biological, physical or biochemical treatment:**
   Oil-producing plants such as oil palm, jatropha, soybean, cotton, linseed; Latexproducing plants such as the Para Rubber tree, *Hevea brasiliensis* and the Panama Rubber Tree *Castilla elastica;* plants used as direct or indirect feedstocks for the production of biofuels i.e. after chemical, physical (e.g. thermal or catalytic) or biochemical (e.g. enzymatic pre-treatment) or biological (e.g. microbial fermentation) transformation during the production of biofuels, industrial solvents or chemical products e.g. ethanol or butanol, propane dials, or other fuel or industrial material including sugar crops (e.g. beet, sugar cane), starch producing crops (e.g. C3 and C4 cereal crops and tuberous crops), cellulosic crops such as forest trees (e.g. Pines, Eucalypts) and Graminaceous and Poaceous plants such as bamboo, switch grass, miscanthus; crops used in energy, biofuel or industrial chemical production via gasification and/or microbial or catalytic conversion of the gas to biofuels or other industrial raw materials such as solvents or plastics, with or without the production of biochar (e.g. biomass crops such as coniferous, eucalypt, tropical or broadleaf forest trees, graminaceous and poaceous crops such as bamboo, switch grass, miscanthus, sugar cane, or hemp or softwoods such as poplars, willows; and, biomass crops used in the production of biochar;
**Crops producing natural products useful for the pharmaceutical, agricultural, and nutraceutical industries:**
   crops producing pharmaceutical precursors or compounds or nutraceutical and cosmeceutical compounds and materials for example, star anise (shikimic acid), Japanese knotweed (resveratrol), kiwifruit (soluble fiber, proteolytic enzymes);
**Floricultural, Ornamental and Amenity plants grown for their aesthetic or environmental properties:**
   Flowers such as roses, tulips, chrysanthemums;
   Ornamental shrubs such as Buxus, Hebe, Rosa, Rhododendron, Hedera
   Amenity plants such as Platanus, Choisya, Escallonia, Euphorbia, Carex
   Mosses such as sphagnum moss
**Plants grown for bioremediation:**
   Helianthus, Brassica, Salix, Populus, Eucalyptus
   It should be appreciated that a plant may be provided in the form of a seed, seedling, cutting, propagule, or any other plant material or tissue capable of growing. In one embodiment the seed may surface-sterilised with a material such as sodium hypochlorite or mercuric chloride to remove surface-contaminating microorganisms. In one embodiment, the propagule is grown in axenic culture before being placed in the plant growth medium, for example as sterile plantlets in tissue culture.

### Growth Medium

The term "growth medium" as used herein, should be taken broadly to mean any medium which is suitable to support growth of a plant. By way of example, the media may be natural or artificial including, but not limited to, soil, potting mixes, bark, vermiculite, hydroponic solutions alone and applied to solid plant support systems, and tissue culture gels. It should be appreciated that the media may be used alone or in combination with one or more other media. It may also be used with or without the addition of exogenous nutrients and physical support systems for roots and foliage.

In one embodiment, the growth medium is a naturally occurring medium such as soil, sand, mud, clay, humus, regolith, rock, or water. In another embodiment, the growth medium is artificial. Such an artificial growth medium may be constructed to mimic the conditions of a naturally occurring medium, however, this is not necessary. Artificial growth media can be made from one or more of any number and combination of materials including sand, minerals, glass, rock, water, metals, salts, nutrients, water. In one embodiment, the growth medium is sterile. In another embodiment, the growth medium is not sterile.

The medium may be amended or enriched with additional compounds or components, for example, a component which may assist in the interaction and/or selection of specific groups of microorganisms with the plant and each other. For example, antibiotics (such as penicillin) or sterilants (for example, quaternary ammonium salts and oxidizing agents) could be present and/or the physical conditions (such as salinity, plant nutrients (for example organic and inorganic minerals (such as phosphorus, nitrogenous salts, ammonia, potassium and micronutrients such as cobalt and magnesium), pH, and/or temperature) could be amended.

In certain embodiments of the disclosure, the growth medium may be pre-treated to assist in the survival and/or selection of certain microorganisms. For example, the medium may be pre-treated by incubating in an enrichment media to encourage the multiplication of endogenous microbes that may be present therein. By way of further example, the medium may be pre-treated by incubating in a selective medium to encourage the multiplication of specific groups of microorganisms. A further example includes the growth medium being pre-treated to exclude a specific element of the microbial assemblage therein; for example pasteurization (to remove spore-forming bacteria and fungi) or treatment with organic solvents such as various alcohols to remove microorganisms sensitive to these materials but allow the survival of actinomycetes and spore-forming bacteria, for example. Methods for pre-treating or enriching may be informed by culture independent microbial community profiling techniques that provide information on the identity of microbes or groups of microbes present. These methods may include, but are not limited to, sequencing techniques including high throughput sequencing and phylogenetic analysis, or microarray-based screening of nucleic acids coding for components of rRNA operons or other taxonomically informative loci.

### Growth Conditions

In accordance with the methods of the disclosure one or more plant is subjected to one or more microorganism and a growth medium. The plant is preferably grown or allowed to multiply in the presence of the one or more microorganisms and growth medium. The microorganisms may be present in the growth medium naturally without the addition of further microorganisms, for example in a natural soil. The growth medium, plant and microorganisms may be combined or exposed to one another in any appropriate order. In one embodiment, the plant, seed, seedling, cutting, propagule or the like is planted or sown into the growth medium which has been previously inoculated with the one or more microorganisms. Alternatively, the one or more microorganisms may be applied to the plant, seed, seedling, cutting, propagule or the like which is then planted or sown into the growth medium (which may or may not contain further microorganisms).

In another embodiment, the plant, seed, seedling, cutting, propagule or the like is first planted or sown into the growth medium, allowed to grow, and at a later time the one or more microorganisms are applied to the plant, seed, seedling, cutting, propagule or the like and/or the growth medium itself is inoculated with the one or more microorganisms. The microorganisms may be applied to the plant, seedling, cutting, propagule or the like and/or the growth medium using any appropriate techniques known in the art. However, by way of example, in one embodiment, the one or more microorganisms are applied to the plant, seedling, cutting, propagule or the like by spraying or dusting. In another embodiment, the microorganisms are applied directly to seeds (for example as a coating) prior to sowing. In a further embodiment, the microorganisms or spores from microorganisms are formulated into granules and are applied alongside seeds during.

In another embodiment, microorganisms may be inoculated into a plant by cutting the roots or stems and exposing the plant surface to the microorganisms by spraying, dipping or otherwise applying a liquid microbial suspension, or gel, or powder. In another embodiment the microorganism(s) may be injected directly into foliar or root tissue, or otherwise inoculated directly into or onto a foliar or root cut, or else into an excised embryo, or radicle or coleoptile. These inoculated plants may then be further exposed to a growth media containing further microorganisms, however, this is not necessary. In certain embodiments, the microorganisms are applied to the plant, seedling, cutting, propagule or the like and/or growth medium in association with plant material (for example, plant material with which the microorganisms are associated).

In other embodiments, particularly where the microorganisms are unculturable, the microorganisms may be transferred to a plant by any one or a combination of grafting, insertion of explants, aspiration, electroporation, wounding, root pruning, induction of stomatal opening, or any physical, chemical or biological treatment that provides the opportunity for microbes to enter plant cells or the intercellular space. Persons of skill in the art may readily appreciate a number of alternative techniques that may be used. It should be appreciated that such techniques are equally applicable to application of the initial flora of microorganisms as well as the final flora of microorganisms obtained from the breeding methods of the present disclosure.

In one embodiment the microorganisms infiltrate parts of the plant such as the roots, stems, leaves and/or reproductive plant parts (become endophytic), and/or grow upon the surface of roots, stems, leaves and/or reproductive plant parts (become epiphytic) and/or grow in the plant rhizosphere. In one embodiment microorganism(s) form a symbiotic relationship with the plant. The growth conditions used may be varied depending on the species of plant, as will be appreciated by persons skilled in the art. However, by way of example, for clover, in a growth room one would typically grow plants in a soil containing approximately one-third organic matter in the form of peat, one-third compost, and one-third screened pumice, supplemented by fertilizers typically containing nitrates, phosphates, potassium and magnesium salts and micronutrients and at a pH of between 6 and 7. The plants may be grown at a temperature between 22-24°C in an 16:8 period of daylight:darkness, and watered automatically.

### Selective Pressure

In certain aspects and embodiments of the disclosure, at a desired time during the period within which the plant is subjected to one or more microorganism and a growth medium, a selective pressure is applied. The selective pressure may be any biotic or abiotic factor or element which may have an impact on the health, growth, and/or survival of a particular plant, including environmental conditions and elements which plants may be exposed to in their natural environment or a commercial situation. Examples of biotic selective pressures include but are not limited to organisms that are detrimental to the plant, for example, fungi, bacteria, viruses, insects, mites, nematodes, animals. Abiotic selective pressures include for example any chemical and physical factors in the environment; for example, water availability, soil mineral composition, salt, temperature, alterations in light spectrum (e.g. increased UV light), pH, organic and inorganic toxins (for example, exposure to or changes in the level of toxins), metals, organic nutrients, inorganic nutrients, air quality, atmospheric gas composition, air flow, rain fall, and hail.

For example, the plant/microorganisms may be exposed to a change in or extreme salt concentrations, temperature, pH, higher than normal levels of atmospheric gases such as CO2, water levels (including drought conditions or flood conditions), low nitrogen levels, provision of phosphorus in a form only available to the plant after microbial degradation, exposure to or changes in the level of toxins in the environment, soils with nearly toxic levels of certain minerals such as aluminates, or high winds.

In one embodiment, the selective pressure is applied directly to the plant, the microorganisms and/or the growth medium. In another embodiment the selective pressure is applied indirectly to the plant, the microorganisms and/or the growth medium, via the surrounding environment; for example, a gaseous toxin in the air or a flying insect.

The selective pressure may be applied at any time, preferably during the time the plant is subjected to the one or more microorganism and growth medium. In one embodiment, the selective pressure is applied for substantially the whole time during which a plant is growing and/or multiplying. In another embodiment, the selective pressure is applied at a discrete time point during growth and/or multiplication. By way of example, the selective pressure may be applied at different growth phases of the one or more plants which simulate a potential stress on the plant that might occur in a natural or commercial setting.

For example, the inventor has observed that some pests attack plants only at specific stages of the plant's life. In addition, the inventor has observed that different populations of potentially beneficial microorganisms can associate with plants at different points in the plant's life. Simulating a pest attack on the plant at the relevant time point, may allow for the identification and isolation of microorganisms which may protect the plant from attack at that particular life stage. It should also be appreciated that the selective pressure may be present in the growth medium or in the general environment at the time the plant, seed, seedling, cutting, propagule or the like is planted or sown.

In one embodiment, the microbial population is exposed (prior to the method or at any stage of the method) to a selective pressure to enhance the probability that the eventually selected plants will have microbial assemblages likely to have desired properties. For example, exposure of the microorganisms to pasteurization before their addition to a plant growth medium (preferably sterile) is likely to enhance the probability that the plants selected for a desired trait will be associated with spore-forming microbes that can more easily survive in adverse conditions, in commercial storage, or if applied to seed as a coating, in an adverse environment.

The plants may be grown and subjected to the selective pressure for any appropriate length of time before they are selected and harvested. By way of example only, the plants and any microorganisms associated with them may be selected and harvested at any time during the growth period of a plant, in one embodiment, any time after germination of the plant. In an embodiment, the plants are grown or allowed to multiply for a period which allows one to distinguish between plants having desirable phenotypic features and those that do not. By way of general example wheat may be selected for improvements in the speed of foliar growth say after one month, but equally may be selected for superior grain yield on maturity of the seed head. The length of time a plant is grown depends on the timing required to express the plant trait that is desired to be improved by the disclosure, or the time required to express a trait correlated with the desired trait. For example, in the case of winter wheat varieties, mainly sown in the Northern Hemisphere, it may be important to select plants that display early tillering after exposure of seed to a growth medium containing microorganisms under conditions of light and temperature similar to those experienced by winter wheat seed in the Northern Hemisphere, since early tillering is a trait related to winter survival, growth and eventual grain yield in the summer.

Or, a tree species may be selected for improved growth and health at 4-6 months as these traits are related to the health and growth rate and size of trees of 10 years later, an impractical period product development using this disclosure. It should be appreciated that the methods of the disclosure may involve applying two or more selective pressures simultaneously or successively in step in between breeding cycles.

### Stacking

The inventors envisage advantages being obtained by stacking selective pressures in repeated rounds of the breeding methods of the disclosure. This may allow for acquiring a population of microorganisms that may assist a plant in surviving in a number of different environmental conditions, resisting a number of different diseases and attack by a number of different organisms, for example.

Similarly, the inventors envisage advantages being obtained by stacking the means of selection (or the selection criteria) of plants in repeated rounds of the breeding methods of the disclosure. This may allow for the acquiring a population of microorganisms that may assist a plant in having a number of different desirable traits, for example.

One could also stack both selective pressures and selection criteria in methods of the disclosure. In one embodiment of the disclosure the one or more microorganisms acquired from the one or more plants selected following exposure to a selective pressure, as previously described, is used in a second round or cycle of the method; *i.e.* the microorganisms from the selected plants are provided, along with one or more plants and a growth medium, a selective pressure is applied, plants are selected at a desired time and microorganisms are isolated from the selected plants. The microorganisms acquired from the second round of the method may then be used in a subsequent round, and so on and so on.

In one embodiment, the selective pressure applied in each repeat of the method is different. For example, in the first round the pressure may be a particular soil pH and in the second round the pressure may be nematode attack. However, in other embodiments of the disclosure, the selective pressure applied in each round may be the same. It could also be the same but applied at differing intensities with each round. For example, in the first round the selective pressure may be a particular concentration of salt present in the soil. In the second round, the selective pressure may be a higher concentration of salt present in the soil. In one embodiment, the selective pressure is increased in successive rounds in a pattern that may be linear, stepped or curvilinear. For example in round one of iterative selective process wheat plus microorganisms may be exposed to 100 mM NaCl, in the second to 110 mM salt, in the third to 120 mM salt, thus increasing the selective pressure on the plants as adaptation occurs via improved plant/microorganism associations.

Alternatively, it may be advantageous to maintain a selective pressure of 120mM for several rounds to allow for a slower adjustment in the microbial population balance underlying improvements in the ability of wheat to grow productively in a higher salt environment. In one embodiment, a selective pressure may be separated disjunctively from a specific step of the iterative process, particularly the first round of an iterative cycle. For example in round one the selective pressure may not be applied at all. But after the microorganisms have been isolated from the selected plants after exposure for a relevant period to a growth medium and microorganisms in round one, they are applied to the plant growth medium along with the plant, seed, seedling, cutting, propagule or the like for round two. After an appropriate time a selective pressure is applied in round two and in successive rounds. This type of selection may be especially relevant for selection factors that severely diminish the plant tissue that is the target of the selection. For example nematodes are especially destructive of root tissue and it may be advantageous to allow particular microbes to multiply to high levels on, in, or around the roots in round one to allow high concentrations of microorganisms from the roots of plants selected in round one to be applied to the growth medium in round two.

Where selection criteria are stacked, the one or more microorganisms acquired from the one or more plants selected, as previously described, is used in a second round or cycle of the method, where a different selection criterion is used. For example, in the first round, one or more plants may have been selected based on biomass. In the second round, one or more plants may be selected based on production of a particular compound. The microorganisms from the second round of the method may then be used in a subsequent round, and so on and so on. Any number of different selection criteria may be employed in successive rounds of the method, as desired or appropriate.

In one embodiment, the selection criteria applied in each repeat of the method is different. However, in other embodiments of the disclosure, the selection criteria applied in each round may be the same. It could also be the same but applied at differing intensities with each round. For example, the selection criteria may be fiber levels and level of fiber required for a plant to be selected may increase with successive rounds of the method. The selective criteria may increase or decrease in successive rounds in a pattern that may be linear, stepped or curvilinear

It should also be appreciated that in certain embodiments of the disclosure, where one or more microorganisms forms an endophytic or epiphytic relationship with a plant that allows vertical transmission from one generation or propagule to the next the microorganisms need not be isolated from the plant At the conclusion of a method of the disclosure, a target or selected plant itself may be multiplied by seed or vegetatively (along with the associated microorganisms) to confer the benefits to "daughter" plants of the next generation or multiplicative phase.

Similarly, where a successive repeat of the method is desired, plant material (whole plant, plant tissue, part of the plant) comprising the set of one or more microorganisms can be used to inoculate the plant of the successive breeding cycle (e.g., progeny plants). It should further be appreciated that two or more selective pressures and/or two or more selection criterion may be applied with each iteration of the breeding methods of the disclosure.

### Plant Breeding Methods

Selective plant breeding is a common approach to improving plants by imparting them with improved traits for growth in a particular area (breeding for a certain climate), or for a specific use (breeding for machine harvestability).

In some embodiments, selection methods, e.g., molecular marker assisted selection, can be combined with breeding methods to accelerate the process. In some embodiments of the present disclosure, selective breeding of plant genotypes is combined with the directed selection of microbial flora to improve traditional breeding schemes.

Choice of breeding or selection methods depends on the mode of plant reproduction, the heritability of the trait(s) being improved, and the type of cultivar used commercially (e.g., F1 hybrid cultivar, pure line cultivar, etc.). For highly heritable traits, a choice of superior individual plants evaluated at a single location will be effective, whereas for traits with low heritability, selection should be based on mean values obtained from replicated evaluations of families of related plants. Non-limiting breeding methods commonly include pedigree selection, modified pedigree selection, mass selection, recurrent selection, and backcross breeding.

The complexity of inheritance influences choice of the breeding method. Backcross breeding is used to transfer one or a few favorable genes for a heritable trait into a desirable cultivar. This approach has been used extensively for breeding disease-resistant cultivars, nevertheless, it is also suitable for the adjustment and selection of morphological characters, color characteristics and simply inherited quantitative characters.

Various recurrent selection techniques are used to improve quantitatively inherited traits controlled by numerous genes. The use of recurrent selection in self-pollinating crops depends on the ease of pollination, the frequency of successful hybrids from each pollination and the number of hybrid offspring from each successful cross.

Taught below are various plant breeding methods. The disclosure teaches that these plant breeding methods can be improved, by controlling for the microbial variability associated with the plants undergoing the breeding process.

### Plant Breeding Methods

### i. Open-Pollinated Populations

The improvement of open-pollinated populations of such crops as rye, many maizes and sugar beets, herbage grasses, legumes such as alfalfa and clover, and tropical tree crops such as cacao, coconuts, oil palm and some rubber, depends essentially upon changing genefrequencies towards fixation of favorable alleles while maintaining a high (but far from maximal) degree of heterozygosity.

Uniformity in such populations is impossible and trueness-to-type in an open-pollinated variety is a statistical feature of the population as a whole, not a characteristic of individual plants. Thus, the heterogeneity of open-pollinated populations contrasts with the homogeneity (or virtually so) of inbred lines, clones and hybrids.

Population improvement methods fall naturally into two groups, those based on purely phenotypic selection, normally called mass selection, and those based on selection with progeny testing. Interpopulation improvement utilizes the concept of open breeding populations; allowing genes to flow from one population to another. Plants in one population (cultivar, strain, ecotype, or any germplasm source) are crossed either naturally (e.g., by wind) or by hand or by bees (commonly *Apis mellifera* L. or *Megachile rotundata* F.) with plants from other populations. Selection is applied to improve one (or sometimes both) population(s) by isolating plants with desirable traits from both sources.

There are basically two primary methods of open-pollinated population improvement.

First, there is the situation in which a population is changed en masse by a chosen selection procedure. The outcome is an improved population that is indefinitely propagable by random-mating within itself in isolation.

Second, the synthetic variety attains the same end result as population improvement, but is not itself propagable as such; it has to be reconstructed from parental lines or clones. These plant breeding procedures for improving open-pollinated populations are well known to those skilled in the art and comprehensive reviews of breeding procedures routinely used for improving cross-pollinated plants are provided in numerous texts and articles, including: Allard, Principles of Plant Breeding, John Wiley & Sons, Inc. (1960); Simmonds, Principles of Crop Improvement, Longman Group Limited (1979); Hallauer and Miranda, Quantitative Genetics in Maize Breeding, Iowa State University Press (1981); and, Jensen, Plant Breeding Methodology, John Wiley & Sons, Inc. (1988).

### ii. Mass Selection

In mass selection, desirable individual plants are chosen, harvested, and the seed composited without progeny testing to produce the following generation. Since selection is based on the maternal parent only, and there is no control over pollination, mass selection amounts to a form of random mating with selection. As stated above, the purpose of mass selection is to increase the proportion of superior genotypes in the population.

### iii. Synthetics

A synthetic variety is produced by crossing inter se a number of genotypes selected for good combining ability in all possible hybrid combinations, with subsequent maintenance of the variety by open pollination. Whether parents are (more or less inbred) seed-propagated lines, as in some sugar beet and beans (Vicia) or clones, as in herbage grasses, clovers and alfalfa, makes no difference in principle. Parents are selected on general combining ability, sometimes by test crosses or topcrosses, more generally by polycrosses. Parental seed lines may be deliberately inbred (e.g. by selfing or sib crossing). However, even if the parents are not deliberately inbred, selection within lines during line maintenance will ensure that some inbreeding occurs. Clonal parents will, of course, remain unchanged and highly heterozygous.

Whether a synthetic can go straight from the parental seed production plot to the farmer or must first undergo one or more cycles of multiplication depends on seed production and the scale of demand for seed. In practice, grasses and clovers are generally multiplied once or twice and are thus considerably removed from the original synthetic.

While mass selection is sometimes used, progeny testing is generally preferred for polycrosses, because of their operational simplicity and obvious relevance to the objective, namely exploitation of general combining ability in a synthetic.

The number of parental lines or clones that enters a synthetic varies widely. In practice, numbers of parental lines range from 10 to several hundred, with 100-200 being the average. Broad based synthetics formed from 100 or more clones would be expected to be more stable during seed multiplication than narrow based synthetics.

### iv. Hybrids

A hybrid is an individual plant resulting from a cross between parents of differing genotypes. Commercial hybrids are now used extensively in many crops, including corn (maize), sorghum, sugarbeet, sunflower and broccoli. Hybrids can be formed in a number of different ways, including by crossing two parents directly (single cross hybrids), by crossing a single cross hybrid with another parent (three-way or triple cross hybrids), or by crossing two different hybrids (four-way or double cross hybrids).

Strictly speaking, most individuals in an out breeding (*i.e.*, open-pollinated) population are hybrids, but the term is usually reserved for cases in which the parents are individuals whose genomes are sufficiently distinct for them to be recognized as different species or subspecies. Hybrids may be fertile or sterile depending on qualitative and/or quantitative differences in the genomes of the two parents. Heterosis, or hybrid vigor, is usually associated with increased heterozygosity that results in increased vigor of growth, survival, and fertility of hybrids as compared with the parental lines that were used to form the hybrid. Maximum heterosis is usually achieved by crossing two genetically different, highly inbred lines.

The production of hybrids is a well-developed industry, involving the isolated production of both the parental lines and the hybrids which result from crossing those lines. For a detailed discussion of the hybrid production process, see, e.g., Wright, Commercial Hybrid Seed Production 8:161-176, In Hybridization of Crop Plants.

### v. Bulk Segregation Analysis (BSA)

BSA, a.k.a. bulked segregation analysis, or bulk segregant analysis, is a method described by Michelmore *et al.*(Michelmore et al., 1991, Identification of markers linked to disease-resistance genes by bulked segregant analysis: a rapid method to detect markers in specific genomic regions by using segregating populations. Proceedings of the National Academy of Sciences, USA, 99:9828-9832) and Quarrie *et al.*(Quarrie et al., 1999, Journal of Experimental Botany, 50(337):1299-1306).

For BSA of a trait of interest, parental lines with certain different phenotypes are chosen and crossed to generate F2, doubled haploid or recombinant inbred populations with QTL analysis. The population is then phenotyped to identify individual plants or lines having high or low expression of the trait. Two DNA bulks are prepared, one from the individuals having one phenotype (e.g., resistant to virus), and the other from the individuals having reversed phenotype (e.g., susceptible to virus), and analyzed for allele frequency with molecular markers. Only a few individuals are required in each bulk (e.g., 10 plants each) if the markers are dominant (e.g., RAPDs). More individuals are needed when markers are codominant (e.g., RFLPs). Markers linked to the phenotype can be identified and used for breeding or QTL mapping.

### vi. Hand-Pollination Method

Hand pollination describes the crossing of plants via the deliberate fertilization of female ovules with pollen from a desired male parent plant. In some embodiments the donor or recipient female parent and the donor or recipient male parent line are planted in the same field. The inbred male parent can be planted earlier than the female parent to ensure adequate pollen supply at the pollination time. The male parent and female parent can be planted at a ratio of 1 male parent to 4-10 female parents. The diploid male parent may be planted at the top of the field for efficient male flower collection during pollination. Pollination is started when the female parent flower is ready to be fertilized. Female flower buds that are ready to open in the following days are identified, covered with paper cups or small paper bags that prevent bee or any other insect from visiting the female flowers, and marked with any kind of material that can be easily seen the next morning. This process is best done in the afternoon. The male flowers of the diploid male parent are collected in the early morning before they are open and visited by pollinating insects. The covered female flowers of the female parent, which have opened, are un-covered and pollinated with the collected fresh male flowers of the diploid male parent, starting as soon as the male flower sheds pollen. The pollinated female flowers are again covered after pollination to prevent bees and any other insects visit. The pollinated female flowers are also marked. The marked fruits are harvested. In some embodiments, the male pollen used for fertilization has been previously collected and stored.

### vii. Bee-Pollination Method

Using the bee-pollination method, the parent plants are usually planted within close proximity. In some embodiments more female plants are planted to allow for a greater production of seed. Breeding of dioecious species can also be done by growing equal amount of each parent plant. Beehives are placed in the field for transfer of pollen by bees from the male parent to the female flowers of the female parent. In some embodiments, fruits set after the introduction of the beehives can be marked for later collection.

### viii. Targeting Induced Local Lesions in Genomes (TILLING)

Breeding schemes of the present application can include crosses with TILLING^{®} plant lines. TILLING^{®} is a method in molecular biology that allows directed identification of mutations in a specific gene. TILLING^{®} was introduced in 2000, using the model plant *Arabidopsis thaliana.* TILLING^{®} has since been used as a reverse genetics method in other organisms such as zebrafish, corn, wheat, rice, soybean, tomato and lettuce.

The method combines a standard and efficient technique of mutagenesis with a chemical mutagen (e.g., Ethyl methanesulfonate (EMS)) with a sensitive DNA screeningtechnique that identifies single base mutations (also called point mutations) in a target gene. EcoTILLING is a method that uses TILLING^{®} techniques to look for natural mutations in individuals, usually for population genetics analysis *(see* Comai, et al., 2003 The Plant Journal 37, 778-786; Gilchrist et al.2006 Mol. Ecol. 15, 1367-1378; Mejlhede et al.2006 Plant Breeding 125, 461-467; Nieto et a/.2007 BMC Plant Biology 7, 34-42, each of which is incorporated by reference hereby for all purposes). DEcoTILLING is a modification of TILLING^{®} and EcoTILLING which uses an inexpensive method to identify fragments (Garvin et al., 2007, DEco-TILLING: An inexpensive method for SNP discovery that reduces ascertainment bias. Molecular Ecology Notes 7, 735-746).

The TILLING^{®} method relies on the formation of heteroduplexes that are formed when multiple alleles (which could be from a heterozygote or a pool of multiple homozygotes and heterozygotes) are amplified in a PCR, heated, and then slowly cooled. A "bubble" forms at the mismatch of the two DNA strands (the induced mutation in TILLING^{®} or the natural mutation or SNP in EcoTILLING), which is then cleaved by single stranded nucleases. The products are then separated by size on several different platforms.

Several TILLING^{®} centers exists over the world that focus on agriculturally important species: UC Davis (USA), focusing on Rice; Purdue University (USA), focusing on Maize; University of British Columbia (CA), focusing on *Brassica napus;* John Innes Centre (UK), focusing on *Brassica rapa*; Fred Hutchinson Cancer Research, focusing on Arabidopsis; Southern Illinois University (USA), focusing on Soybean; John Innes Centre (UK), focusing on Lotus and Medicago; and INRA (France), focusing on Pea and Tomato.

More detailed description on methods and compositions on TILLING^{®} can be found in US 5994075, US 2004/0053236 A1, WO 2005/055704, and WO 2005/048692, each of which is hereby incorporated by reference for all purposes.

Thus in some embodiments, the breeding methods of the present disclosure include breeding with one or more TILLING plant lines with one or more identified mutations.

### ix. Double Haploids and Chromosome Doubling

One way to obtain homozygous plants without the need to cross two parental lines followed by a long selection of the segregating progeny, and/or multiple back-crossings is to produce haploids and then double the chromosomes to form doubled haploids. Haploid plants can occur spontaneously, or may be artificially induced via chemical treatments or by crossing plants with inducer lines (Seymour et al.2012, PNAS vol 109, pg 4227-4232; Zhang et al., 2008 Plant Cell Rep. Dec 27(12) 1851-60). The production of haploid progeny can occur via a variety of mechanisms which can affect the distribution of chromosomes during gamete formation. The chromosome complements of haploids sometimes double spontaneously to produce homozygous doubled haploids (DHs). Mixoploids, which are plants which contain cells having different ploidies, can sometimes arise and may represent plants that are undergoing chromosome doubling so as to spontaneously produce doubled haploid tissues, organs, shoots, floral parts or plants. Another common technique is to induce the formation of double haploid plants with a chromosome doubling treatment such as colchicine (El-Hennawy *et al.*, 2011 Vol 56, issue 2 pg 63-72; Doubled Haploid Production in Crop Plants 2003 edited by Maluszynski ISBN 1-4020-1544-5). The production of doubled haploid plants yields highly uniform inbred lines and is especially desirable as an alternative to sexual inbreeding of longer-generation crops. By producing doubled haploid progeny, the number of possible gene combinations for inherited traits is more manageable. Thus, an efficient doubled haploid technology can significantly reduce the time and the cost of inbred and cultivar development.

### x. Protoplast Fusion

In another method for breeding plants, protoplast fusion can also be used for the transfer of trait-conferring genomic material from a donor plant to a recipient plant. Protoplast fusion is an induced or spontaneous union, such as a somatic hybridization, between two or more protoplasts (cells of which the cell walls are removed by enzymatic treatment) to produce a single bi- or multi-nucleate cell. The fused cell that may even be obtained with plant species that cannot be interbred in nature is tissue cultured into a hybrid plant exhibiting the desirable combination of traits.

### xi. Embryo Rescue

Alternatively, embryo rescue may be employed in the transfer of resistance-conferring genomic material from a donor plant to a recipient plant. Embryo rescue can be used as a procedure to isolate embryo's from crosses wherein plants fail to produce viable seed. In this process, the fertilized ovary or immature seed of a plant is tissue cultured to create new plants (see Pierik, 1999, In vitro culture of higher plants, Springer, ISBN 079235267x, 9780792352679, which is incorporated herein by reference in its entirety).

### xii. Pedigreed varieties

A pedigreed variety is a superior genotype developed from selection of individual plants out of a segregating population followed by propagation and seed increase of self pollinated offspring and careful testing of the genotype over several generations. This is an open pollinated method that works well with naturally self pollinating species. This method can be used in combination with mass selection in variety development. Variations in pedigree and mass selection in combination are the most common methods for generating varieties in self pollinated crops.

### xiii. Gene editing technologies

Breeding and selection schemes of the present application can include crosses with plant lines that have undergone genome editing. In some embodiments, the breeding and selection methods of the present disclosure are compatible with plants that have been modified using any genome editing tool, including, but not limited to: ZFNs, TALENS, CRISPR, and Mega nuclease technologies. In some embodiments, persons having skill in the art will recognize that the AMS and breeding methods of the present disclosure are compatible with many other gene editing technologies.

In some embodiments, the gene editing tools of the present disclosure comprise proteins or polynucleotides which have been custom designed to target and cut at specific deoxyribonucleic acid (DNA) sequences. In some embodiments, gene editing proteins are capable of directly recognizing and binding to selected DNA sequences. In other embodiments, the gene editing tools of the present disclosure form complexes, wherein nuclease components rely on nucleic acid molecules for binding and recruiting the complex to the target DNA sequence.

In some embodiments, the single component gene editing tools comprise a binding domain capable of recognizing specific DNA sequences in the genome of the plant and a nuclease that cuts double-stranded DNA. The rationale for the development of gene editing technology for plant breeding is the creation of a tool that allows the introduction of site-specific mutations in the plant genome or the site-specific integration of genes.

Many methods are available for delivering genes into plant cells, e.g. transfection, electroporation, viral vectors and *Agrobacterium* mediated transfer. Genes can be expressed transiently from a plasmid vector. Once expressed, the genes generate the targeted mutation that will be stably inherited, even after the degradation of the plasmid containing the gene.

In some embodiments, the breeding and selection methods of the present disclosure are compatible with plants that have been modified through Zinc Finger Nucleases. Three variants of the ZFN technology are recognized in plant breeding (with applications ranging from producing single mutations or short deletions/insertions in the case of ZFN-1 and -2 techniques up to targeted introduction of new genes in the case of the ZFN-3 technique):

**ZFN-1:** Genes encoding ZFNs are delivered to plant cells without a repair template. The ZFNs bind to the plant DNA and generate site specific double-strand breaks (DSBs). The natural DNA-repair process (which occurs through nonhomologous end-joining, NHEJ) leads to site specific mutations, in one or only a few base pairs, or to short deletions or insertions.

**ZFN-2:** Genes encoding ZFNs are delivered to plant cells along with a repair template homologous to the targeted area, spanning a few kilo base pairs. The ZFNs bind to the plant DNA and generate site-specific DSBs. Natural gene repair mechanisms generate site-specific point mutations e.g. changes to one or a few base pairs through homologous recombination and the copying of the repair template.

**ZFN-3:** Genes encoding ZFNs are delivered to plant cells along with a stretch of DNA which can be several kilo base pairs long and the ends of which are homologous to the DNA sequences flanking the cleavage site. As a result, the DNA stretch is inserted into the plant genome in a site specific manner.

In some embodiments, the breeding and selection methods of the present disclosure are compatible with plants that have been modified through Transcription activator-like (TAL) effector nucleases (TALENs). TALENS are polypeptides with repeat polypeptide arms capable of recognizing and binding to specific nucleic acid regions. By engineering the polypeptide arms to recognize selected target sequences, the TAL nucleases can be use to direct double stranded DNA breaks to specific genomic regions. These breaks can then be repaired via recombination to edit, delete, insert, or otherwise modify the DNA of a host organism. In some embodiments, TALENSs are used alone for gene editing (e.g., for the deletion or disruption of a gene). In other embodiments, TALs are used in conjunction with donor sequences and/or other recombination factor proteins that will assist in the Nonhomologous end joining (NHEJ) process to replace the targeted DNA region. For more information on the TAL-mediated gene editing compositions and methods of the present disclosure, *see* US Patent Nos. 8,440,432; 8,440,432; US 8,450,471; US 8,586,526; US 8,586,363; US 8,592,645; US 8,697,853; 8,704,041; 8,921,112; and 8,912,138, each of which is hereby incorporated in its entirety for all purposes.

In some embodiments, the breeding and selection methods of the present disclosure are compatible with plants that have been modified through Clustered Regularly Interspaced Short Palindromic Repeats (CRISPR) or CRISPR-associated (Cas) gene editing tools. CRISPR proteins were originally discovered as bacterial adaptive immunity systems which protected bacteria against viral and plasmid invasion.

There are at least three main CRISPR system types (Type I, II, and III) and at least 10 distinct subtypes (Makarova, K.S., et.al., Nat Rev Microbiol. 2011 May 9;9(6):467-477). Type I and III systems use Cas protein complexes and short guide polynucleotide sequences to target selected DNA regions. Type II systems rely on a single protein (e.g. Cas9) and the targeting guide polynucleotide, where a portion of the 5' end of a guide sequence is complementary to a target nucleic acid. For more information on the CRISPR gene editing compositions and methods of the present disclosure, *see* US Patent Nos. 8,697,359; 8,889,418; 8,771,945; and 8,871,445, each of which is hereby incorporated in its entirety for all purposes.

In some embodiments, the breeding and selection methods of the present disclosure are compatible with plants that have been modified through meganucleases. In some embodiments, meganucleases are engineered endonucleases capable of targeting selected DNA sequences and inducing DNA breaks. In some embodiments, new meganucleases targeting specific regions are developed through recombinant techniques which combine the DNA binding motifs from various other identified nucleases. In other embodiments, new meganucleases are created through semi-rational mutational analysis, which attempts to modify the structure of existing binding domains to obtain specificity for additional sequences. For more information on the use of meganucleases for genome editing, *see* Silva et al., 2011 Current Gene Therapy 11 pg 11-27; and Stoddard et al., 2014 Mobile DNA 5 pg 7, each of which is hereby incorporated in its entirety for all purposes.

### xiv. Oligonucleotide directed mutagenesis (ODM)

ODM is another tool for targeted mutagenesis in plant breeding. ODM is based on the use of oligonucleotides for the induction of targeted mutations in the plant genome, usually of one or a few adjacent nucleotides. The genetic changes that can be obtained using ODM include the introduction of a new mutation (replacement of one or a few base pairs), the reversal of an existing mutation or the induction of short deletions.

ODM is also known as oligonucleotide-mediated gene modification, targeted gene correction, targeted gene repair, RNA-mediated DNA modification, RNA-templated DNA repair, induced targeted mutagenesis, targeted nucleotide exchange, chimeraplasty, genoplasty, oligonucleotide mediated gene editing, chimeric oligonucleotide dependent mismatch repair, oligonucleotide-mediated gene repair, triplex-forming oligonucleotides induced recombination, oligodeoxynucleotide-directed gene modification, and therapeutic nucleic acid repair approach.

The oligonucleotides usually employed are approximately 20 to 100 nucleotides long and are chemically synthesized in order to share homology with the target sequence in the host genome, but not with the nucleotide(s) to be modified. Oligonucleotides such as chimeric oligonucleotides, consisting of mixed DNA and RNA bases, and single-stranded DNA oligonucleotides can be deployed for ODM.

Oligonucleotides can be delivered to the plant cells by methods suitable for the different cell types, including electroporation and polyethylene glycol (PEG) mediated transfection. The specific methods used for plants are usually particle bombardment of plant tissue or electroporation of protoplasts.

Oligonucleotides target the homologous sequence in the genome and create one or more mismatched base pairs corresponding to the noncomplementary nucleotides. The cell's own gene repair mechanism is believed to recognize these mismatches and induce their correction. The oligonucleotides are expected to be degraded in the cell but the induced mutations will be stably inherited.

### xv. Cisgenesis and Intragenesis

As opposed to transgenesis which can be used to insert genes from any organism, both eukaryotic and prokaryotic, into plant genomes, cisgenesis and intragenesis are terms recently created by scientists to describe the restriction of transgenesis to DNA fragments from the species itself or from a cross-compatible species. In the case of cisgenesis, the inserted genes, associated introns and regulatory elements are contiguous and unchanged. In the case of intragenesis, the inserted DNA can be a new combination of DNA fragments from the species itself or from a crosscompatible species.

Both approaches aim to confer a new property to the modified plant. However, by definition, only cisgenics could achieve results also possible by traditional breeding methods (but in a much shorter time frame).

Intragenesis offers considerably more options for modifying gene expression and trait development than cisgenesis, by allowing combinations of genes with different promoters and regulatory elements. Intragenesis can also include the use of silencing approaches, e.g. RNA interference (RNAi), by introducing inverted DNA repeats.

Cisgenic and intragenic plants are produced by the same transformation techniques as transgenic plants. The currently most investigated cisgenic plants are potato and apple, and *Agrobacterium-mediated* transformation is most frequently used. However, biolistic approaches are also suitable on a case-by-case basis.

### xvi. RNA-dependent DNA methylation (RdDM)

RdDM allows breeders to produce plants that do not contain foreign DNA sequences and in which no changes or mutations are made in the nucleotide sequence, but in which gene expression is modified due to epigenetics.

RdDM induces the transcriptional gene silencing (TGS) of targeted genes via the methylation of promoter sequences. In order to obtain targeted RdDM, genes encoding RNAs which are homologous to promoter regions are delivered to the plant cells by suitable methods of transformation. This involves, at some stage, the production of a transgenic plant. These genes, once transcribed, give rise to double stranded RNAs (dsRNAs) which, after processing by specific enzymes, induce methylation of the target promoter sequences thereby inhibiting the transcription of the target gene.

In plants, methylation patterns are meiotically stable. The change in the methylation pattern of the promoter, and therefore the desired trait, will be inherited by the following generation. The progeny will include plant lines which, due to segregation in the breeding population, do not contain the inserted genes but retain the desired trait. The methylated status can continue for a number of generations following the elimination of the inserted genes.

The epigenetic effect is assumed to decrease through subsequent generations and to eventually fade out, but this point needs further investigation.

### xvii. Grafting (on GM rootstock)

Grafting is a method whereby the aboveground vegetative component of one plant (also known as the scion), is attached to a rooted lower component (also known as the rootstock) of another plant to produce a chimeric organism with improved cultivation characteristics.

Transgenesis, cisgenesis, and a range of other techniques can be used to transform the rootstock and/or scion. If a GM scion is grafted onto a non-GM rootstock, then stems, leaves, flowers, seeds, and fruits will be transgenic.

When a non-GM scion is grafted onto a GM rootstock, leaves, stems, flowers, seeds and fruits would not carry the genetic modification with respect to changes in genomic DNA sequences.

Transformation of the rootstock can be obtained using traditional techniques for plant transformation, e.g. *Agrobacterium-mediated* transformation and biolistic approaches. Using genetic modification, characteristics of a rootstock including rooting capacity or resistance to soil borne diseases, can be improved, resulting in a substantial increase in the yield of harvestable components such as fruit.

If gene silencing in rootstocks is an objective this can also be obtained through RNA interference (RNAi), a system of gene silencing that employs small RNA molecules. In grafted plants, the small RNAs can also move through the graft so that the silencing signal can affect gene expression in the scion. RNAi rootstocks may therefore be used to study the effects of transmissible RNAi-mediated control of gene expression.

### xviii. Reverse Breeding

Reverse breeding is a method in which the order of events leading to the production of a hybrid plant variety is reversed. It facilitates the production of homozygous parental lines that, once hybridized, reconstitute the genetic composition of an elite heterozygous plant, without the need for back-crossing and selection.

The method of reverse breeding includes the following steps: Selection of an elite heterozygous line that has to be reproduced; Suppression of meiotic recombination in the elite heterozygous line through silencing of genes such as *dmc1* and *spo11* following plant transformation with transgenes encoding RNA interference (RNAi) sequences; Production of haploid microspores (immature pollen grains) from flowers of the resulting transgenic elite heterozygous line; Use of doubled haploid (DH) technology to double the genome of the haploid microspores and to obtain homozygous cells; Culture of the microspores in order to obtain homozygous diploid plants; Selection of plant pairs (called parental lines) that do not contain the transgene and whose hybridization would reconstitute the elite heterozygous line.

The reverse breeding technique makes use of transgenesis to suppress meiotic recombination. In subsequent steps, only non-transgenic plants are selected. Therefore, the offspring of the selected parental lines would genotypically reproduce the elite heterozygous plant and would not carry any additional genomic change.

In addition to the producing of homozygous lines from heterozygous plants, reverse breeding offers further possible applications in plant breeding, e.g. the production of socalled chromosome substitution lines.

### xix. Agro-infiltration (agro-infiltration "sensu stricto", agro-inoculation, floral dip)

Plant tissues, mostly leaves, are infiltrated with a liquid suspension of *Agrobacterium* sp. containing the desired gene(s) to be expressed in the plant. The genes are locally and transiently expressed at high levels.

The technique is often used in a research context: e.g. to study plant-pathogen interaction in living tissues (leaves) or to test the functionality of regulatory elements in gene constructs.

However the technique has also been developed as a production platform for high value recombinant proteins due to the flexibility of the system and the high yields of the recombinant proteins obtained. In all cases, the plant of interest is the agro-infiltrated plant and not the progeny.

Agro-infiltration can be used to screen for plants with valuable phenotypes that can then be used in breeding programs.

For instance, agro-infiltration with specific genes from pathogens can be used to evaluate plant resistance. The resistant plants identified in the agro-infiltration test might then be used directly as parents for breeding. The progenies obtained will not be transgenic as no genes are inserted into the genome of the germline cells of the agro-infiltrated plant.

Alternatively, other stored plants which are genetically identical to the identified candidate plant may be used as parents.

Depending on the tissues and the type of gene constructs infiltrated, three types of agro-infiltration can be distinguished:
**1. Agro-infiltration sensu stricto:** Nongermline tissue (typically leaf tissue) is infiltrated with non-replicative constructs in order to obtain localized expression in the infiltrated area.
**2. Agro-inoculation or agro-infection:** Non-germline tissue (typically leaf tissue) is infiltrated with a construct containing the foreign gene in a full-length virus vector in order to obtain expression in the entire plant.
**3. Floral dip:** Germline tissue (typically flowers) is immersed into a suspension of *Agrobacterium* carrying a DNA-construct in order to obtain transformation of some embryos that can be selected at the germination stage. The aim is to obtain stably transformed plants. Therefore, the resulting plants are GMOs that do not differ from GM plants obtained by other transformation methods.

### xx. Synthetic Genomics

Synthetic genomics has been defined as "the engineering of biological components and systems that do not exist in nature and the re-engineering of existing biological elements; it is determined on the intentional design of artificial biological systems, rather than on the understanding of natural biology." (Synbiology, 2006).

Thanks to the technological level reached by genetic engineering and the current knowledge regarding complete genomes' sequences, large functional DNA molecules can now be synthesised efficiently and quickly without using any natural template.

Recently the genome of *Mycoplasma genitalium,* the smallest known bacterial genome, was assembled from commercially synthesized pieces.

Synthetic genomics not only provides the possibility to reproduce existing organisms in vitro, but the synthesis of building blocks enables the creation of modified natural or even completely artificial organisms.

One of the goals of synthetic genomics is the preparation of viable minimal genomes which will function as platforms for the biochemical production of chemicals with economic relevance.

The production of biofuels, pharmaceuticals, and the bioremediation of environmental pollution are expected to constitute the first commercial applications of this new technique.

However, presently there is no research relevant to the use of synthetic genomics in plant breeding. This is expected to change in the future as the field progresses.

### Breeding Evaluation

Each breeding program can include a periodic, objective evaluation of the efficiency of the breeding procedure. Evaluation criteria vary depending on the goal and objectives, but should include gain from selection per year based on comparisons to an appropriate standard, overall value of the advanced breeding lines, and number of successful cultivars produced per unit of input (e.g., per year, per dollar expended, etc.).

In some embodiments, the present disclosure teaches the evaluation of both the plant breeding, and microbial flora. In some embodiments, the evaluation of the plants and microflora is conducted in parallel (e.g., separately evaluating the benefits through the effects of the improved microflora and the latest plant progeny). In other embodiments, the present disclosure teaches methods of evaluating the synergistic effects of plant progeny-microflora combinations.

Promising advanced breeding lines are thoroughly tested per se and in hybrid combination and compared to appropriate standards in environments representative of the commercial target area(s). Similarly, promising microflora can be thoroughly tested with the same or different plants, alone, or in combination with other identified microflora. In some embodiments the "other" microflora are identified through the present breeding process. In other embodiments, the "other" microflora is single microorganisms or combinations of microorganisms which have been previously identified through methods of the present disclosure, or other methods known in the art. This testing can in some cases continue for three or more years. The best lines are candidates for use as parents in new commercial cultivars; those still deficient in a few traits may be used as parents to produce new populations for further selection.

Typically, following growth of the one or more plants in the presence of one or more microorganisms, and in certain embodiments following exposure to a selective pressure, one or more plant is selected based on one or more selection criterion.

In one embodiment, the plants are selected on the basis of one or more phenotypic traits. Skilled persons will readily appreciate that such traits include any observable characteristic of the plant, including for example growth rate, height, weight, color, taste, smell, changes in the production of one or more compounds by the plant (including for example, metabolites, proteins, drugs, carbohydrates, oils, and any other compounds).

Selecting plants based on genotypic information is also envisaged (for example, including the pattern of plant gene expression in response to the microorganisms, genotype, presence of genetic markers).

It should be appreciated that in certain embodiments, plants may be selected based on the absence, suppression or inhibition of a certain feature or trait (such as an undesirable feature or trait) as opposed to the presence of a certain feature or trait (such as a desirable feature or trait).

Where the presence of one or more genetic marker is assessed, the one or more marker may already be known and/or associated with a particular characteristic of a plant; for example, a marker or markers may be associated with an increased growth rate or metabolite profile. This information could be used in combination with assessment based on other characteristics in a method of the disclosure to select for a combination of different plant characteristics that may be desirable. Such techniques may be used to identify novel quatitative trait loci (QTLs) which link desirable plant traits with a specific microbial flora - for example matching plant genotype to the microbiome type. By way of example, plants may be selected based on growth rate, size (including but not limited to weight, height, leaf size, stem size, branching pattern, or the size of any part of the plant), general health, survival, tolerance to adverse physical environments and/or any other characteristic, as described herein before.

Further non-limiting examples include selecting plants based on: speed of seed germination; quantity of biomass produced; increased root, and/or leaf/shoot growth that leads to an increased yield (herbage or grain or fiber or oil) or biomass production; effects on plant growth that results in an increased seed yield for a crop, which may be particularly relevant in cereal crops such as wheat, barley, oats, rye, maize, rice, sorghum, oilseed crops such as soybean, canola, cotton, sunflower, and seed legumes such as peas, beans; effects on plant growth that result in an increased oil yield, which may be particularly relevant in oil seed crops such as soybean, canola, cotton, jatropha and sunflower; effects on plant growth that result in an increased fiber yield (e.g. in cotton, flax and linseed) or for effects that result in an increased tuber yield in crops such as potatoes and sugar beet; effects on plant growth that result in an increased digestibility of the biomass which may be particularly relevant in forage crops such as forage legumes (alfalfa, clovers, medics), forage grasses *Lolium* species; *Festuca* species; *Paspalum* species; *Brachiaria* species; *Eragrostis* species), forage crops grown for silage such as maize and forage cereals (wheat, barley, oats); effects on plant growth which result in an increased fruit yield which may be particularly relevant to pip fruit trees (such as apples, pears, etc), berry fruits (such as strawberries, raspberries, cranberries), stone fruit (such as nectarines, apricots), and citrus fruit, grapes, figs, nut trees; effects on plant growth that lead to an increased resistance or tolerance disease including fungal, viral or bacterial diseases or to pests such as insects, mites or nematodes in which damage is measured by decreased foliar symptoms such as the incidence of bacterial or fungal lesions, or area of damaged foliage or reduction in the numbers of nematode cysts or galls on plant roots, or improvements in plant yield in the presence of such plant pests and diseases; effects on plant growth that lead to increased metabolite yields, for example in plants grown for pharmaceutical, nutraceutical or cosmeceutical purposes which may be particularly relevant for plants such as star anise grown for the production of shikimic acid critical for the production of anti-influenza drug oseltamivir, or the production of Japanese knotweed for the extraction of resveratrol, or the production of soluble fiber and dietary enzyme products from kiwifruit, or for example increased yields of "condensed tannins" or other metabolites useful for inhibiting the production of greenhouse gases such as methane in grazing animals; effects on plant growth that lead to improved aesthetic appeal which may be particularly important in plants grown for their form, color or taste, for example the color intensity and form of ornamental flowers, the taste of fruit or vegetable, or the taste of wine from grapevines treated with microorganisms; and, effects on plant growth that lead to improved concentrations of toxic compounds taken up or detoxified by plants grown for the purposes of bioremediation.

### Molecular Breeding Evaluation Techniques

Selection of plants based on phenotypic or genotypic information may be performed using techniques such as, but not limited to: high through-put screening of chemical components of plant origin, sequencing techniques including high through-put sequencing of genetic material, differential display techniques (including DDRT-PCR, and DD-PCR), nucleic acid microarray techniques, RNA-seq (Whole Transcriptome Shotgun Sequencing), qRTPCR (quantitative real time PCR).

In one embodiment, the evaluating step of a plant breeding program involves the identification of desirable traits in progeny plants. Progeny plants can be grown in, or exposed to conditions designed to emphasize a particular trait (e.g. drought conditions for drought tolerance, lower temperatures for freezing tolerant traits). Progeny plants with the highest scores for a particular trait may be used for subsequent breeding steps.

In some embodiments, plants selected from the evaluation step can exhibit a 1%, 5%, 10%, 15%, 20%, 25%, 30%, 35%, 40%, 45%, 50%, 55%, 60%, 65%, 70%, 75%, 80%, 85%, 90%, 95%, 100%, 120% or more improvement in a particular plant trait compared to a control plant.

In other embodiments, the evaluating step of plant breeding comprises one or more molecular biological tests for genes or other markers. For example, the molecular biological test can involve probe hybridization and/or amplification of nucleic acid (e.g., measuring nucleic acid density by Northern or Southern hybridization, PCR) and/or immunological detection (e.g., measuring protein density, such as precipitation and agglutination tests, ELISA (e.g., Lateral Flow test or DAS-ELISA), Western blot, RIA, immune labeling, immunosorbent electron microscopy (ISEM), and/or dot blot).

The procedure to perform a nucleic acid hybridization, an amplification of nucleic acid (e.g., RT-PCR) or an immunological detection (e.g., precipitation and agglutination tests, ELISA (e.g., Lateral Flow test or DAS-ELISA), Western blot, RIA, immunogold or immunofluorescent labeling, immunosorbent electron microscopy (ISEM), and/or dot blot tests) are performed as described elsewhere herein and well-known by one skilled in the art.

In one embodiment, the evaluating step comprises PCR (semi-quantitative or quantitative), wherein primers are used to amplify one or more nucleic acid sequences of a desirable gene, or a nucleic acid associated with said gene or a desirable trait (e.g., a cosegregating nucleic acid, or other marker).

In another embodiment, the evaluating step comprises immunological detection (e.g., precipitation and agglutination tests, ELISA (e.g., Lateral Flow test or DAS-ELISA), Western blot, RIA, immuno labeling (gold, fluorescent, or other detectable marker), immunosorbent electron microscopy (ISEM), and/or dot blot), wherein one or more gene or marker-specific antibodies are used to detect one or more desirable proteins. In one embodiment, said specific antibody is selected from the group consisting of polyclonal antibodies, monoclonal antibodies, antibody fragments, and combination thereof.

Reverse Transcription Polymerase Chain Reaction (RT-PCR) can be utilized in the present disclosure to determine expression of a gene to assist during the selection step of a breeding scheme. It is a variant of polymerase chain reaction (PCR), a laboratory technique commonly used in molecular biology to generate many copies of a DNA sequence, a process termed "amplification". In RT-PCR, however, RNA strand is first reverse transcribed into its DNA complement (complementary DNA, or cDNA) using the enzyme reverse transcriptase, and the resulting cDNA is amplified using traditional or real-time PCR.

RT-PCR utilizes a pair of primers, which are complementary to a defined sequence on each of the two strands of the cDNA. These primers are then extended by a DNA polymerase and a copy of the strand is made after each cycle, leading to logarithmic amplification.

RT-PCR includes three major steps. The first step is the reverse transcription (RT) where RNA is reverse transcribed to cDNA using a reverse transcriptase and primers. This step is very important in order to allow the performance of PCR since DNA polymerase can act only on DNA templates. The RT step can be performed either in the same tube with PCR (one-step PCR) or in a separate one (two-step PCR) using a temperature between 40°C and 50°C, depending on the properties of the reverse transcriptase used.

The next step involves the denaturation of the dsDNA at 95°C, so that the two strands separate and the primers can bind again at lower temperatures and begin a new chain reaction. Then, the temperature is decreased until it reaches the annealing temperature which can vary depending on the set of primers used, their concentration, the probe and its concentration (if used), and the cations concentration. The main consideration, of course, when choosing the optimal annealing temperature is the melting temperature (Tm) of the primers and probes (if used). The annealing temperature chosen for a PCR depends directly on length and composition of the primers. This is the result of the difference of hydrogen bonds between A-T (2 bonds) and G-C (3 bonds). An annealing temperature about 5 degrees below the lowest Tm of the pair of primers is usually used.

The final step of PCR amplification is the DNA extension from the primers which is done by the thermostable Taq DNA polymerase usually at 72°C, which is the optimal temperature for the polymerase to work. The length of the incubation at each temperature, the temperature alterations and the number of cycles are controlled by a programmable thermal cycler. The analysis of the PCR products depends on the type of PCR applied. If a conventional PCR is used, the PCR product is detected using agarose gel electrophoresis and ethidium bromide (or other nucleic acid staining).

Conventional RT-PCR is a time-consuming technique with important limitations when compared to real time PCR techniques. This, combined with the fact that ethidium bromide has low sensitivity, yields results that are not always reliable. Moreover, there is an increased cross-contamination risk of the samples since detection of the PCR product requires the post-amplification processing of the samples. Furthermore, the specificity of the assay is mainly determined by the primers, which can give false-positive results. However, the most important issue concerning conventional RT-PCR is the fact that it is a semi or even a low quantitative technique, where the amplicon can be visualized only after the amplification ends.

Real time RT-PCR provides a method where the amplicons can be visualized as the amplification progresses using a fluorescent reporter molecule. There are three major kinds of fluorescent reporters used in real time RT-PCR, general non specific DNA Binding Dyes such as SYBR Green I, TaqMan Probes and Molecular Beacons (including Scorpions).

The real time PCR thermal cycler has a fluorescence detection threshold, below which it cannot discriminate the difference between amplification generated signal and background noise. On the other hand, the fluorescence increases as the amplification progresses and the instrument performs data acquisition during the annealing step of each cycle. The number of amplicons will reach the detection baseline after a specific cycle, which depends on the initial concentration of the target DNA sequence. The cycle at which the instrument can discriminate the amplification generated fluorescence from the background noise is called the threshold cycle (Ct). The higher is the initial DNA concentration, the lower its Ct will be.

Other forms of nucleic acid detection can include next generation sequencing methods such as DNA SEQ or RNA SEQ using any known sequencing platform including, but not limited to: Roche 454, Solexa Genome Analyzer, AB SOLiD, Illumina GA/HiSeq, Ion PGM, Mi Seq, among others (Liu et al,. 2012 Journal of Biomedicine and Biotechnology Volume 2012 ID 251364; Franca et al., 2002 Quarterly Reviews of Biophysics 35 pg 169-200; Mardis 2008 Genomics and Human Genetics vol 9 pg 387-402).

In other embodiments, nucleic acids may be detected with other high throughput hybridization technologies including microarrays, gene chips, LNA probes, nanoStrings, and fluorescence polarization detection among others.

In some embodiments, detection of markers can be achieved at an early stage of plant growth by harvesting a small tissue sample (e.g., branch, or leaf disk). This approach is preferable when working with large populations as it allows breeders to weed out undesirable progeny at an early stage and conserve growth space and resources for progeny which show more promise. In some embodiments the detection of markers is automated, such that the detection and storage of marker data is handled by a machine. Recent advances in robotics have also led to full service analysis tools capable of handling nucleic acid/protein marker extractions, detection, storage and analysis.

### Quantitative Trait Loci

Breeding schemes of the present application can include crosses between donor and recipient plants. In some embodiments said donor plants contain a gene or genes of interest which may confer the plant with a desirable phenotype. The recipient line can be an elite line having certain favorite traits such for commercial production. In one embodiment, the elite line may contain other genes that also impart said line with the desired phenotype. When crossed together, the donor and recipient plant may create a progeny plant with combined desirable loci which may provide quantitatively additive effect of a particular characteristic. In that case, QTL mapping can be involved to facilitate the breeding process.

A QTL (quantitative trait locus) mapping can be applied to determine the parts of the donor plant's genome conferring the desirable phenotype, and facilitate the breeding methods. Inheritance of quantitative traits or polygenic inheritance refers to the inheritance of a phenotypic characteristic that varies in degree and can be attributed to the interactions between two or more genes and their environment. Though not necessarily genes themselves, quantitative trait loci (QTLs) are stretches of DNA that are closely linked to the genes that underlie the trait in question. QTLs can be molecularly identified to help map regions of the genome that contain genes involved in specifying a quantitative trait. This can be an early step in identifying and sequencing these genes.

Typically, QTLs underlie continuous traits (those traits that vary continuously, e.g. yield, height, level of resistance to virus, etc.) as opposed to discrete traits (traits that have two or several character values, e.g. smooth vs. wrinkled peas used by Mendel in his experiments). Moreover, a single phenotypic trait is usually determined by many genes. Consequently, many QTLs are associated with a single trait.

A quantitative trait locus (QTL) is a region of DNA that is associated with a particular phenotypic trait - these QTLs are often found on different chromosomes. Knowing the number of QTLs that explains variation in the phenotypic trait tells about the genetic architecture of a trait. It may tell that a trait is controlled by many genes of small effect, or by a few genes of large effect.

Another use of QTLs is to identify candidate genes underlying a trait Once a region of DNA is identified as contributing to a phenotype, it can be sequenced. The DNA sequence of any genes in this region can then be compared to a database of DNA for genes whose function is already known.

In a recent development, classical QTL analyses are combined with gene expression profiling *i.e.* by DNA microarrays. Such expression QTLs (e-QTLs) describes cis- and transcontrolling elements for the expression of often disease-associated genes. Observed epistatic effects have been found beneficial to identify the gene responsible by a cross-validation of genes within the interacting loci with metabolic pathway- and scientific literature databases.

QTL mapping is the statistical study of the alleles that occur in a locus and the phenotypes (physical forms or traits) that they produce (see, Meksem and Kahl, The handbook of plant genome mapping: genetic and physical mapping, 2005, Wiley-VCH, ISBN 3527311165, 9783527311163). Because most traits of interest are governed by more than one gene, defining and studying the entire locus of genes related to a trait gives hope of understanding what effect the genotype of an individual might have in the real world.

Statistical analysis is required to demonstrate that different genes interact with one another and to determine whether they produce a significant effect on the phenotype. QTLs identify a particular region of the genome as containing a gene that is associated with the trait being assayed or measured. They are shown as intervals across a chromosome, where the probability of association is plotted for each marker used in the mapping experiment.

To begin, a set of genetic markers must be developed for the species in question. A marker is an identifiable region of variable DNA. Biologists are interested in understanding the genetic basis of phenotypes (physical traits). The aim is to find a marker that is significantly more likely to co-occur with the trait than expected by chance, that is, a marker that has a statistical association with the trait. Ideally, they would be able to find the specific gene or genes in question, but this is a long and difficult undertaking. Instead, they can more readily find regions of DNA that are very close to the genes in question. When a QTL is found, it is often not the actual gene underlying the phenotypic trait, but rather a region of DNA that is closely linked with the gene.

For organisms whose genomes are known, one might now try to exclude genes in the identified region whose function is known with some certainty not to be connected with the trait in question. If the genome is not available, it may be an option to sequence the identified region and determine the putative functions of genes by their similarity to genes with known function, usually in other genomes. This can be done using BLAST, an online tool that allows users to enter a primary sequence and search for similar sequences within the BLAST database of genes from various organisms.

Another interest of statistical geneticists using QTL mapping is to determine the complexity of the genetic architecture underlying a phenotypic trait. For example, they may be interested in knowing whether a phenotype is shaped by many independent loci, or by a few loci, and do those loci interact. This can provide information on how the phenotype may be evolving.

Molecular markers are used for the visualization of differences in nucleic acid sequences. This visualization is possible due to DNA-DNA hybridization techniques (RFLP) and/or due to techniques using the polymerase chain reaction (e.g. STS, microsatellites, AFLP). All differences between two parental genotypes will segregate in a mapping population based on the cross of these parental genotypes. The segregation of the different markers may be compared and recombination frequencies can be calculated. The recombination frequencies of molecular markers on different chromosomes are generally 50%. Between molecular markers located on the same chromosome the recombination frequency depends on the distance between the markers. A low recombination frequency corresponds to a low distance between markers on a chromosome. Comparing all recombination frequencies will result in the most logical order of the molecular markers on the chromosomes. This most logical order can be depicted in a linkage map (Paterson, 1996, Genome Mapping in Plants. R.G. Landes, Austin.). A group of adjacent or contiguous markers on the linkage map that is associated to a reduced disease incidence and/or a reduced lesion growth rate pinpoints the position of a QTL.

The nucleic acid sequence of a QTL may be determined by methods known to the skilled person. For instance, a nucleic acid sequence comprising said QTL or a resistance-conferring part thereof may be isolated from a donor plant by fragmenting the genome of said plant and selecting those fragments harboring one or more markers indicative of said QTL. Subsequently, or alternatively, the marker sequences (or parts thereof) indicative of said QTL may be used as (PCR) amplification primers, in order to amplify a nucleic acid sequence comprising said QTL from a genomic nucleic acid sample or a genome fragment obtained from said plant. The amplified sequence may then be purified in order to obtain the isolated QTL. The nucleotide sequence of the QTL, and/or of any additional markers comprised therein, may then be obtained by standard sequencing methods.

One or more such QTLs associated with a desirable trait in a donor plant can be transferred to a recipient plant to make incorporate the desirable train into progeny plants by transferring and/or breeding methods.

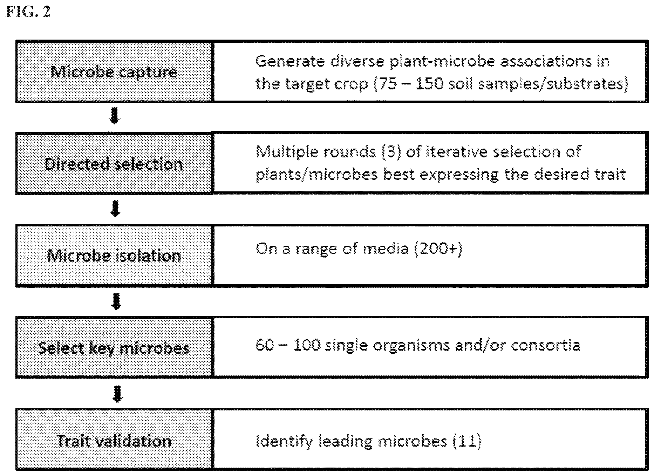

In one embodiment, an advanced backcross QTL analysis (AB-QTL) is used to discover the nucleotide sequence or the QTLs responsible for the resistance of a plant. Such method was proposed by Tanksley and Nelson in 1996 (Tanksley and Nelson, 1996, Advanced backcross QTL analysis: a method for simultaneous discovery and transfer of valuable QTL from un-adapted germplasm into elite breeding lines. Theor Appl Genet 92:191-203) as a new breeding method that integrates the process of QTL discovery with variety development, by simultaneously identifying and transferring useful QTL alleles from un-adapted (e.g., land races, wild species) to elite germplasm, thus broadening the genetic diversity available for breeding. A non-limiting exemplary scheme of AB-QTL mapping strategy is shown in Figure 2. AB-QTL strategy was initially developed and tested in tomato, and has been adapted for use in other crops including rice, maize, wheat, pepper, barley, and bean. Once favorable QTL alleles are detected, only a few additional marker-assisted generations are required to generate near isogenic lines (NILs) or introgression lines (ILs) that can be field tested in order to confirm the QTL effect and subsequently used for variety development.

Isogenic lines in which favorable QTL alleles have been fixed can be generated by systematic backcrossing and introgressing of marker-defined donor segments in the recurrent parent background. These isogenic lines are referred as near isogenic lines (NILs), introgression lines (ILs), backcross inbred lines (BILs), backcross recombinant inbred lines (BCRIL), recombinant chromosome substitution lines (RCSLs), chromosome segment substitution lines (CSSLs), and stepped aligned inbred recombinant strains (STAIRSs). An introgression line in plant molecular biology is a line of a crop species that contains genetic material derived from a similar species. ILs represent NILs with relatively large average introgression length, while BILs and BCRILs are backcross populations generally containing multiple donor introgressions per line. As used herein, the term "introgression lines or ILs" refers to plant lines containing a single marker defined homozygous donor segment, and the term "pre-ILs" refers to lines which still contain multiple homozygous and/or heterozygous donor segments.

To enhance the rate of progress of introgression breeding, a genetic infrastructure of exotic libraries can be developed. Such an exotic library comprises of a set of introgression lines, each of which has a single, possibly homozygous, marker-defined chromosomal segment that originates from a donor exotic parent, in an otherwise homogenous elite genetic background, so that the entire donor genome would be represented in a set of introgression lines. A collection of such introgression lines is referred as libraries of introgression lines or IL libraries (ILLs). The lines of an ILL cover usually the complete genome of the donor, or the part of interest. Introgression lines allow the study of quantitative trait loci, but also the creation of new varieties by introducing exotic traits. High resolution mapping of QTL using ILLs enable breeders to assess whether the effect on the phenotype is due to a single QTL or to several tightly linked QTL affecting the same trait. In addition, sub-ILs can be developed to discover molecular markers which are more tightly linked to the QTL of interest, which can be used for marker-assisted breeding (MAB). Multiple introgression lines can be developed when the introgression of a single QTL is not sufficient to result in a substantial improvement in agriculturally important traits (Gur and Zamir, Unused natural variation can lift yield barriers in plant breeding, 2004, PLoS Biol. ;2(10):e245).

### Epigenetics

In some embodiments, the breeding and selection methods of the present disclosure can be used to produce desired phenotypes through epigenetic modifications. That is, in some embodiments, the AMS and holobiomes of the present disclosure can induce or maintain desirable chromatin and/or histone level modifications leading to non-DNA sequence based changes in gene expression. Epigenetics as used herein refers to any non-DNA sequence modification of gene expression, including those due to chromatin structure changes, DNA methylation, and histone modifications (e.g., methylation and acetylation).

In some embodiments, the present disclosure teaches breeding evaluations through the tracking of epigenetic changes in progeny plants. In some embodiments, the present disclosure teaches the assessment of epigenetic changes on selected loci. For example, in some embodiments, the present disclosure teaches the use of methylation sensitive restriction enzymes and PCR analysis to determine the epigenetic status of a particular DNA region. In other embodiments, the present disclosure teaches the use of bisulfite or bisulfite pyrosequencing.

In some embodiments, the assessment of epigenetic changes is genome wide. In some embodiments the present disclosure teaches epigenetic screens through Restriction Landmark Genome Scanning, or methylation-specific digital karyotyping. In other embodiments, the present disclosure teaches the use of microarray or sequencing-based epigenetic screening. In these methods, DNA from plants is filtered through one or more antibodies recognizing various epigenetic modifications, and then sent for individual sequencing or microarray hybridization.

Persons having skill in the art will recognize that the breeding methods of the present disclosure can also utilize other techniques capable of detecting epigenetic changes in the DNA of the progeny plants. For a review of epigenetic detection techniques, *see* Shen et al., 2007 Current Opinion in Clinical Nutrition and Metabolic Care 10 pg 576-581; and Li et al., 2008 The Plant Cell 20, 259-276, each of which is hereby incorporated in its entirety for all purposes.

### EXAMPLES

### Example 1: Uniform AMS-Derived Microbial Background Useful for Conferring Growth in Nitrogen Limited Soils for Maize (Zea mays) Selective Breeding

In certain embodiments of the disclosure, the present methods aim to reduce the amount of environmental variability associated with traditional plant breeding programs.

In this prophetic example, the present methods control for the microbial diversity present in a selective maize breeding program, by utilizing the accelerated microbial selection process to define a set of microbial organisms that will be utilized in a subsequent selective maize breeding method.

### Step 1. AMS Process to Derive Microbial Consortia Beneficial to Maize Grown in Nitrogen Limiting Soils

***Microbial Capture:*** Acquisition of microorganisms may be acquired from a diverse selection of soil samples. These soil samples are not necessarily associated with areas in which maize is known to grow.

Untreated seeds of maize can be planted into each soil sample. Any number of replicates can be used, e.g. 100 replicates in 28ml containers filled with soil. Where necessary, the samples can be extended by the addition of sterile vermiculite or perlite. The maize plants utilized in the present example can be inbreds, hybrids or segregating populations. For example, the maize plants can be a group of selected maize inbreds or hybrids; or, the maize plants can be the seeds or plants of a segregating F2, F3, F4, F5, F6, F7 or later segregating generation. In one example, the maize plants are a population of segregating F2 maize genotypes obtained by selfing an F1 hybrid and planting the resultant seed. For example, the F1 is a cross between 'B73' or a B73-type inbred (e.g., 'LH132') with 'Mol7' or a Mol7-type inbred (e.g., 'LH51'). Thus, in one specific example, the F1 is the result of crossing 'LH51' X 'LH132'.

Plants can be grown with tap water as the only source of moisture, as set forth below in Table 1.

**TABLE 1**

| **Variable** | **Conditions** |
|---|---|
| Watering | Three times each week to saturation with water or synthetic fertilizer detailed in each section |
| Temperature | Constant 22-24 °C |
| Daylight period | 16 hr followed by 8 hr darkness |
| Seed sterilization | 15 min in 1-2% sodium hypochlorite followed by 30 min quenching in sodium thiosulphate |
| Volume of soil per replicate | 28ml |

After a suitable period of growth, plants can be selected on size and the roots and basal stem can be harvested by cutting away foliage 1-2 cm above the soil line.

Excess soil can be manually removed and the remaining basal stem and roots gently washed twice in tap water followed by one rinse in sterile distilled water, leaving small particles of soil attached to the root surfaces. The wet roots of replicate plants from each sample site can be combined, placed in sealable plastic bags and crushed. Sterile water (10mls) can be added and samples filtered through sterile 25µm nylon mesh to remove plant material and invertebrate pests.

The resulting microbial suspensions can be diluted to an appropriate volume and represent the initial microbial communities "captured" for subsequent use in the accelerated microbial selection process.

***Iterative Microbial Selection:*** The aforementioned microbial suspensions can be used to directly inoculate surface-sterilized seeds of maize.

Following inoculation with microbes the developing plant and microbe combinations can be watered with aqueous fertilizer solutions lacking Nitrogen. The lack of N is a selective pressure utilized to select for microbial communities able to confer increased plant growth in N limiting conditions. The plants can be grown for a sufficient period of time, such that phenotypic heterogeneity is observed, for example 30 days.

After 30 days, the maize plants exhibiting the most robust aboveground biomass vigor can be selected, e.g. largest leaf lengths, and the below ground microbial communities can be extracted from these selected plants. In some methods, only the microbes associated with the plant tissue are utilized and microbes from adjacent soil or growth medium are not used. However, in other methods, microbes associated with the plant tissue and adjoining rhizosphere are used.

The microbial communities extracted from the maize plants exhibiting the most robust aboveground biomass vigor in N limiting soils can then be utilized to inoculate a second cohort of maize seeds, as described above.

The second cohort of maize seeds (inoculated with the microbes acquired from the previous selection round) are then grown for a period of time sufficient to observe phenotypic heterogeneity in the maize plants, e.g. 30 days. Again, the maize plants exhibiting the most robust aboveground biomass vigor are selected for and the microbial communities associated with these plants are isolated.

The aforementioned iterative process of selecting the maize plants demonstrating the desired phenotypic response in N limiting conditions and subsequently capturing the associated microbial communities of the maize plants demonstrating the selected for phenotypic trait (e.g. most robust aboveground biomass) and utilizing said microbial community in subsequent inoculations can be performed any number of times.

At the end of the aforementioned accelerated microbial selection ("AMS") process, one has developed a microbial community that is adept at conferring increased biomass growth upon maize plants grown in N limiting conditions.

### Step 2. Utilize AMS-Derived Microbial Consortia in Traditional Maize Selective Breeding to Reduce Environmental (i.e. Microbial) Variability

Upon performing the AMS process, one can utilize the final microbial communities derived in said process, as the starting microbial communities in traditional plant breeding methodologies.

For example, in maize breeding methods, one would provide the above AMS-derived microbial consortia as the microbial component utilized in the maize breeding.

Sterilization of the growth medium may be required in order to ensure that the supplied AMS microbial community is not mixed with a heterogeneous and unknown microbial community. Sterilization of the soil can be accomplished in any manner known to one of skill in the art.

The maize breeding methods would then be carried out, as is standard in the art.

A benefit of this methodology is the reduced variability associated with the microbial community present during the selective maize breeding process. Normally, a breeder does not control for the microbial communities present in the breeding populations.

Further, this particular example focused upon deriving microbial consortia capable of increasing maize vigor in N limiting soils; however, any AMS-derived microbial consortia could be utilized.

For instance, the initial AMS process could be carried out in Phosphorous limited conditions, such that microbial communities are derived that increase maize vigor in P limited conditions.

Alternatively, the AMS process could be carried out without the utilization of a selective pressure, e.g. no N or P limitation.

Also envisioned are methods in which the microbial capture step of the AMS procedure is utilized on soils collected from areas specific to a particular maize hybrid, such that any resulting microbial community at the end of the AMS procedure will include microbes expected to perform well in a particular soil, within which a maize hybrid is expected to be planted.

In the above example, reference was made to "providing" the AMS-derived microbial consortia as the microbial component utilized in the maize breeding. It is envisioned that one may provide the AMS-derived microbial community in a variety of ways.

For instance, the AMS-derived microbial consortia may be supplied as a seed coating to the maize plants.

In some embodiments, the AMS-derived microbial consortia may be supplied as granules, or plugs, or soil drench, to the maize growth media.

### Example 2: Plant-Directed AMS Breeding of Cold Tolerant Soybeans (Glycine max)

In this prophetic example, plant breeding methodologies are conducted and simultaneous capture of microbial communities associated with specific plants is utilized in each breeding cycle to inoculate subsequent cohorts. Soybean breeders have continually strived to select for soybean varieties with greater cold tolerance, particularly to cold soils in addition to cooler air temperatures. The microsphere associated with colder soils is expected to be different than that of warmer soils. As a result, the microorganisms associated with soybean varieties adapted to grow in warmer environments may not be the best ones for growing new soybean varieties for growing in colder climates, especially when such new soybean varieties are developed from warmer-adapted soybeans.

According to this example, two elite soybean cultivars (e.g., two homozygous or nearly homozygous soybean genotypes with proven track records) are crossed to produce F1 hybrid plants which are then selfed to produce F2 seeds. At least one of the elite soybean cultivars used as a parental line for producing the F1 is a soybean cultivar adapted to warmer environments (e.g., a soybean cultivar from Soybean Maturity Groups VII or VIII). An example of a representative F1 is a cross between the elite soybean cultivars 'Williams 82' (Maturity Group III) X 'Howard' (Maturity Group VIII).

The resultant F2 seeds are planted in individual containers filled with soil collected from one or more soybean fields located in Soybean Maturity Group Zones 0, 00, I, and/or II (i.e., farms located within the northern United States to northern Canada). The containers planted with the soybean seeds are placed in environmentally controlled growth chambers set at 15°C/5°C day/night. Cold tolerant plants are selected and allowed to reach maturity and set F3 seed. F3 seed are harvested from the selected F2 plants and microbes are isolated from the associated soil in each selected F2 plant's container. The selected F3 seed/microbe combinations are planted in individual containers filled with autoclaved soil (or soil sterilized via any method known in the art) which is the same as the (non-autoclaved, or non-sterilized) soil used in the F2 screening.

The containers planted with the F3 soybean seeds are placed in environmentally controlled growth chambers set at 15°C/5°C day/night. Cold tolerant plants are selected and allowed to reach maturity and set F4 seed. F4 seed are harvested from the selected F3 plants and microbes are isolated from the associated soil in each selected F3 plant's container. The selected F4 seed/microbe combinations are planted in individual containers filled with autoclaved soil (or soil sterilized via any method known in the art) which is the same as the (non-autoclaved) soil used in the F2 screening.

The containers planted with the F4 soybean seeds are placed into environmentally controlled growth chambers set at 15°C/5°C day/night. Cold tolerant plants are selected and allowed to reach maturity and set F5 seed. F5 seed are harvested from the selected F4 plants and microbes are isolated from the associated soil in each selected F4 plant's container to produce selected F5 seed/microbe combinations.

As stated above, the cold tolerance selection process is repeated through the production of F5 seed during which the following number of individual soybean genotypes is screened and selected: 10,000 F2 genotypes → 1,000 F3 genotypes → 100 F4 genotypes → 10 F5 genotypes. The parental lines are used as controls through each selection cycle.

The final 10 F5 genotypes and their associated microbial consortia are planted in replicated field trials along with appropriate control varieties in one or more locations within Soybean Maturity Zones I and/or II and evaluated for early emergence, cold tolerance, lodging, yield and other agronomic traits of interest. The highest yielding F5 plants with good agronomic characteristics and their associated microbial consortia are chosen for further research and possible commercialization.

### Example 3: Microbial-Directed AMS Breeding of Aluminum Tolerance in Spring Wheat (Triticum aestivum )

In this prophetic example, the AMS process is used on parental plant material and then the AMS-derived microbial consortia are used to conduct the plant breeding. Soils in the Pacific Northwest of the United States are naturally acidic and becoming more acidic due to agronomic practices. In wheat production, soil acidity can cause aluminum (Al) toxicity that leads to severe yield reductions.

According to this example, two elite Al-tolerant spring wheat varieties are used to pre-select for a microbial consortia that can survive and flourish in high Al levels for use in a plant breeding program selecting for Al-tolerant spring wheat segregants.

The soft white spring wheat varieties 'Alpowa' and 'Babe' have been shown to have aluminum tolerance (see, e.g., Washington State University Extension Fact Sheet FS050E, Soil acidity and aluminum toxicity in the Palouse Region of the Pacific Northwest). Soil samples are collected from various, specific locations and the microbes of each are isolated. The resultant location-specific microbial consortia are cultured individually and used to inoculate the wheat varieties which are grown together in separate hydroponic containers for each different location-specific microbial consortia in a controlled growth chamber. The hydroponic solutions are all maintained at a pH of 5.5 and an Al concentration of 200 (mu)M. The microbial consortia remaining in each container when the wheat varieties mature are collected, maintained as separate consortia and increased to create location-specific, selected microbial consortia.

'Alpowa' is crossed to 'Babe' to produce F1 hybrid plants which are then selfed to produce F2 seeds. The resultant F2 seeds are planted in containers with a hydroponic solution maintained at a pH of 5.5 and an Al concentration of 200 (mu)M and placed into controlled growth chambers. Each individual container is inoculated with one of the location-specific, selected microbial consortia. Preferably, at least 10,000 F2 plants are exposed to each location-specific, selected microbial consortia.

Al-tolerant plants are selected and allowed to reach maturity and set F3 seed. F3 seed are harvested from the selected F2 plants. The selected F3 seeds are planted in containers with a hydroponic solution maintained at a pH of 5.5 and an Al concentration of 200 (mu)M and placed into controlled growth chambers. Each individual container is inoculated with one of the location-specific, selected microbial consortia. Preferably, at least 1,000 F3 plants are exposed to each location-specific, selected microbial consortia. Al-tolerant plants are selected and allowed to reach maturity and set F4 seed. The process is repeated to produce F5 seed.

As stated above, the Al-tolerance selection process is repeated through the production of F5 seed during which the following number of individual spring wheat genotypes is screened and selected: 100,000 F2 genotypes → 10,000 F3 genotypes → 1,000 F4 genotypes → 100 F5 genotypes. The parental lines are used as controls through each selection cycle.

The final 100 F5 genotypes and their associated location-specific, selected microbial consortia are planted in replicated field trials in low acidic, high Al concentration fields along with appropriate control varieties in one or more locations within the Pacific Northwest and evaluated for Al-tolerance, lodging, yield and other agronomic traits of interest. The highest yielding F5 plants with good agronomic characteristics and their associated microbial consortia are chosen for further research and possible commercialization.

### Example 4: Uniform AMS-Derived Microbial Background Useful for Conferring Drought Tolerance in Maize (Zea mays) Selective Breeding

In this example, microbial consortia derived from the AMS process were used to treat maize under a drought tolerance study. The microbial treatments influence leaf canopy temperature and chlorophyll content under drought stress and indicate improved water use efficiency and stress resilience.

Yield protection against field drought can be achieved in multiple ways. Principle among them are physiological and developmental changes that provide tolerance, avoidance, or mitigation of the complex stress. An example of a strategy to mitigate against drought stress is reduction of canopy water loss through transpiration, resulting in conservation of soil water. A more conservative use of soil water can buffer the crop against extreme soil water deficit and mitigate against the deleterious physiological effects of drought stress on yield.

Changes in stomatal response to environment that decrease conductance to water vapor are a specific physiological response that will decrease leaf and canopy transpiration and crop water use. In a crop such as wheat that uses the C3 photosynthetic pathway, decreased stomatal conductance may compromise photosynthesis during periods of more optimal soil water availability; however, integrated over the lifetime of the crop these losses would be expected to be offset by a decrease in the severity of stress experienced. For a crop such as maize, that uses the C4 photosynthetic pathway, decreased stomatal conductance may have little or no effect on photosynthetic rate.

Table 2 below, describes the results of an AMS microbial selection process for drought tolerance run on maize. Control plants received no microbe treatment. This process has resulted in the identification of multiple microbial consortia that decrease stomatal conductance relative to no-microbe controls under non-stressed growth conditions and that would be expected to conserve soil water. In this study, measurements of leaf temperature provide a surrogate measurement for leaf water loss. Because transpiration cools the leaf, warmer leaves are transpiring less.

As shown in Table 2, plants treated with 19 out of 25 consortia being tested have increased leaf temperature, the largest increase being 0.88°C.

Table 2 also provides data to support a link between decreased leaf transpiration and decreased stress. Column 3 of Table 2 provides evidence that plants treated with these consortia were less stressed after a two week soil dry down begun at the V3 developmental stage. Column 3 details the difference between the percentage decrease in leaf chlorophyll content during the dry down for each treatment and the no-microbe controls. So a more negative value signals a smaller reduction in chlorophyll content over the dry-down period and a less stressed plant. For example, in consortia BCC23, a change in leaf chlorophyll content of -7 (column 3) indicates that the decrease in leaf chlorophyll content over this period was 7% less than that for the no-microbe control treatment. In comparison, leaf chlorophyll contents of unstressed no-microbe controls were 14% and 15% less than stressed controls, in each of two experiments. In total, 17 of the 19 microbial treatments that decreased leaf transpiration also had higher leaf chlorophyll contents than control plants after the soil dry down. This is evidence that the drought stress experienced was less severe.

**TABLE 2**

| **Consortia** | **Change in Leaf Temperature (°C)** | **Change in leaf chlorophyll content (%).** |
|---|---|---|
| BCC1 | 0.82 | 1 |
| BCC23 | 0.37 | -7 |
| BCC30 | 0.7 | -2 |
| BCC34 | 0.64 | 4 |
| BCC40 | 0.35 | -6 |
| BCC65 | 0.56 | -6 |
| BCC66 | 0.87 | -3 |
| BCC67 | 0.88 | -3 |
| BCC80 | 0.56 | -3 |
| BCC81 | 0.77 | -3 |
| BCC83 | 0.25 | -7 |
| BCC84 | 0.11 | -1 |
| Unstressed | | -14 |
| BCC12 | 0.2 | -5 |
| BCC15 | -0.11 | -10 |
| BCC18 | 0.25 | -8 |
| BCC70 | -0.26 | -2 |
| BCC71 | 0.17 | -1 |
| BCC72 | -0.18 | -5 |
| BCC73 | 0.02 | -6 |
| BCC74 | 0.14 | -8 |
| BCC75 | 0.25 | -5 |
| BCC76 | 0.3 | -11 |
| BCC77 | -0.11 | -1 |
| BCC79 | -0.31 | Chlorophyll data not obtained for this sample |
| BCC82 | 0 | -1 |
| Unstressed | | -15 |

Leaf temperature shown is given as Tleaf no-microbe control - Tleaf Consortia.

The change in leaf chlorophyll content is calculated as the difference between the percentage decrease during the stress for the no-microbe control plants, and each consortia treatment, *i.e.* (Control_{before} - Control_{after}) / Control_{before} - (Consortia_{before} - Consortia_{after}) / Consortia_{before}

Consortia identified above as beneficial for drought tolerance will be used as the initial cultures for the plant breeding methods of the present disclosure.

Thus, the microbial variability will be controlled in the proposed plant breeding program by utilizing the above derived microbes. In another sense, microbes that induce a negative effect, e.g. high transpiration and high stress, could be used as the background to breed and select for plants that can overcome this stress effect (*i.e.* essentially selecting for plants that might be tolerant of negative microbial effects on plant function when under drought stress).

### INCORPORATION BY REFERENCE

All references, articles, publications, patents, patent publications, and patent applications cited herein are incorporated by reference in their entireties for all purposes.

However, mention of any reference, article, publication, patent, patent publication, and patent application cited herein is not, and should not be taken as, an acknowledgment or any form of suggestion that they constitute valid prior art or form part of the common general knowledge in any country in the world.

### REFERENCES

Aguero, C.B., Uratsu, S.L., Greve, C., Powell, A.L.T., Labavitch, J.M., Meredith, C.P., Dandekar, A.M., 2005. Evaluation of tolerance to Pierce's disease and Botrytis in transgenic plants of Vitis vinifera L.expressing the pear PGIP gene. Mol. Plant Pathol. 6, 43-51.
Akhond, M.A.Y., Machray, G.C., 2009. Biotech crops: technologies, achievements and prospects. Euphytica 166, 47-59.
Allard, Robert Wayne. 1960. Principles of Plant Breeding, John Wiley & Sons, Inc. ISBN 0-471-02309
Alonso, J.M., Ecker, J.R., 2006. Moving forward in reverse: genetic technologies to enable genome-wide phenomic screens in Arabidopsis. Nat. Rev. Genet. 7, 524-536.
Armstrong, M.R., Whisson, S.C., Pritchard, L., Bos, J.I.B., Venter, E., Avrova, A.O., Rehmany, A.P., Bohme, U., Brooks, K., Cherevach, I., Hamlin, N., White, B., Frasers, A., Lord, A., Quail, M.A., Churcher, C., Hall, N., Berriman, M., Huang, S., Kamoun, S., Beynon, J.L., Birch, P.R.J., 2005. An ancestral oomycete locus contains late blight avirulence gene Avr3a, encoding a protein that is recognized in the host cytoplasm. Proc. Natl. Acad. Sci. U. S. A. 102, 7766-7771.
Aufsatz, W., Mette, M.F., van der Winden, J., Matzke, A.J., Matzke, M., 2002a. RNA-directed DNA methylation in Arabidopsis. Proc Natl Acad Sci USA 99 Suppl 4, 16499-16506.
Aufsatz, W., Mette, M.F., van der Winden, J., Matzke, M., Matzke, A.J.M., 2002b. HDA6, a putative histone deacetylase needed to enhance DNA methylation induced by double-stranded RNA. Embo Journal 21, 6832-6841.
BAC, 2007. Advice of the Belgian Biosafety Advisory Council on the use of "Targeted Gene Repair" as a strategy to develop novel organisms (ref. WIV-ISP/BAC/2007_SC_529).
Beetham, P.R., Kipp, P.B., Sawycky, X.L., Arntzen, C.J., May, G.D., 1999. A tool for functional plant genomics: chimeric RNA/DNA oligonucleotides cause in vivo gene-specific mutations. Proc Natl Acad Sci U S A 96, 8774-8778.
Belfanti, E., Silfverberg-Dilworth, E., Tartarini, S., Patocchi, A., Barbieri, M., Zhu, J., Vinatzer, B.A., Gianfranceschi, L., Gessler, C., Sansavini, S., 2004. The HcrVf2 gene from a wild apple confers scab resistance to a transgenic cultivated variety. Proc. Natl. Acad. Sci. U.S.A. 101, 886-890.
Belzile, F.J., 2002. Transgenic, transplastomic and other genetically modified plants: a Canadian perspective. Biochimie 84, 1111-1118.
Bendahmane, A., Querci, M., Kanyuka, K., Baulcombe, D.C., 2000. Agrobacterium transient expression system as a tool for the isolation of disease resistance genes: application to the Rx2 locus in potato. Plant Journal 21, 73-81.
Benjamin, I., Kenigsbuch, D., Galperin, M., Abrameto, J., Cohen, Y., 2009. Cisgenic melons over expressing glyoxylate-aminotransferase are resistant to downy mildew. European Journal of Plant Pathology 125, 355-365.
Breyer, D., Herman, P., Brandenburger, A., Gheysen, G., Remaut, E., Soumillion, P., Van Doorsselaere, J., Custers, R., Pauwels, K., Sneyers, M., Reheul, D., 2009. Genetic modification through oligonucleotidemediated mutagenesis. A GMO regulatory challenge? Environ Biosafety Res 8, 57-64.
Britt, A.B., May, G.D., 2003. Re-engineering plant gene targeting. Trends in Plant Science 8, 90-95.
Cai, C.Q., Doyon, Y., Ainley, W.M., Miller, J.C., Dekelver, R.C., Moehle, E.A., Rock, J.M., Lee, Y.L., Garrison, R., Schulenberg, L., Blue, R., Worden, A., Baker, L., Faraji, F., Zhang, L., Holmes, M.C., Rebar, E.J., Collingwood, T.N., Rubin-Wilson, B., Gregory, P.D., Urnov, F.D., Petolino, J.F., 2009. Targeted transgene integration in plant cells using designed zinc finger nucleases. Plant Mol Biol 69, 699-709.
Campbell, C.R., Keown, W., Lowe, L., Kirschling, D., Kucherlapati, R., 1989. Homologous recombination involving small single-stranded oligonucleotides in human cells. New Biol 1, 223-227.
Chen, X.M., 2010. Small RNAs - secrets and surprises of the genome. Plant Journal 61, 941-958.
Chinnusamy, V., Zhu, J.K., 2009. RNA-directed DNA methylation and demethylation in plants. Sci. China Ser. C-Life Sci. 52, 331-343.
Cigan, A.M., Unger-Wallace, E., Haug-Collet, K., 2005. Transcriptional gene silencing as a tool for uncovering gene function in maize. Plant Journal 43, 929-940.
COGEM, 2006a. Ethical and societal aspects of cisgenesis. CGM/060706-03. The Netherlands Commission on Genetic Modification. COGEM, 2006b. New techniques in plant biotechnology. COGEM report CGM/061024-02. The Netherlands Commission on Genetic Modification (NL).
Comai L, Young K, Bradley T, Reynolds S, Greene E, Codomo C, Enns L, Johnson J, Burtner C, Odden A, Henikoff S. 2003. Efficient Discovery of DNA polymorphisms in natural populations by Ecotilling. The Plant Journal 37, 778-786.
Conner, A., Barrell, P., Baldwin, S., Lokerse, A., Cooper, P., Erasmuson, A., Nap, J.-P., Jacobs, J., 2007. Intragenic vectors for gene transfer without foreign DNA. Euphytica 154, 341-353.
Conrath, U., Linke, C., Jeblick, W., Geigenberger, P., Quick, W.P., Neuhaus, H.E., 2003. Enhanced resistance to Phytophthora infestans and Alternaria solani in leaves and tubers, respectively, of potato plants with decreased activity of the plastidic ATP/ADP transporter. Planta 217, 75-83.
Coutos-Thevenot, P., Poinssot, B., Bonomelli, A., Yean, H., Breda, C., Buffard, D., Esnault, R., Hain, R., Boulay, M., 2001. In vitro tolerance to Botrytis cinerea of grapevine 41B rootstock in transgenic plants expressing the stilbene synthase Vst1 gene under the control of a pathogen-inducible PR 10 promoter. J Exp Bot 52, 901-910.
Cruz, F.C.S., Boulton, M.I., Hull, R., Azzam, O., 1999. Agroinoculation allows the screening of rice for resistance to rice tungro bacilliform virus. Journal of Phytopathology-Phytopathologische Zeitschrift 147, 653-659.
D'Aoust, M.A., Lavoie, P.O., Couture, M.M.J., Trepanier, S., Guay, J.M., Dargis, M., Mongrand, S., Landry, N., Ward, B.J., Vezina, L.P., 2008. Influenza virus-like particles produced by transient expression in Nicotiana benthamiana induce a protective immune response against a lethal viral challenge in mice. Plant Biotechnology Journal 6, 930-940.
Dalakouras, A., Moser, M., Zwiebel, M., Krczal, G., Hell, R., Wassenegger, M., 2009. A hairpin RNA construct residing in an intron efficiently triggered RNA-directed DNA methylation in tobacco. Plant Journal 60, 840-851.
Daxinger, L., Kanno, T., Bucher, E., van der Winden, J., Naumann, U., Matzke, A.J.M., Matzke, M., 2009. A stepwise pathway for biogenesis of 24-nt secondary siRNAs and spreading of DNA methylation. Embo Journal 28, 48-57.
de Cock Buning, T., Lammerts van Bueren, E.T., Haring, M.A., de Vriend, H.C., Struik, P.C., 2006. 'Cisgenic' as a product designation. Nat Biotech 24, 1329-1331.
De Paepe, A., De Buck, S., Hoorelbeke, K., Nolf, J., Peck, I., Depicker, A., 2009. High frequency of singlecopy T-DNA transformants produced by floral dip in CRE-expressing Arabidopsis plants. Plant J 59, 517-527.
de Pater, S., Neuteboom, L.W., Pinas, J.E., Hooykaas, P.J., van der Zaal, B.J., 2009. ZFN-induced mutagenesis and gene-targeting in Arabidopsis through Agrobacterium-mediated floral dip transformation. Plant Biotechnol J 7, 821-835.
Derrick, P.M., Barker, H., 1997. Short and long distance spread of potato leafroll luteovirus: Effects of host genes and transgenes conferring resistance to virus accumulation in potato. J. Gen. Virol. 78, 243-251.
Dingle, T.C., Butler-Wu, S. MALDI-TOF Mass Spectrometry for Microorganism 2013 Indentification. Clinics in Laboratory Medicine Vol 33, Issue 3,589-609.
Dirks, R., van Dun, K., de Snoo, C.B., van den Berg, M., Lelivelt, C.L., Voermans, W., Woudenberg, L., de Wit, J.P., Reinink, K., Schut, J.W., van der Zeeuw, E., Vogelaar, A., Freymark, G., Gutteling, E.W., Keppel, M.N., van Drongelen, P., Kieny, M., Ellul, P., Touraev, A., Ma, H., de Jong, H., Wijnker, E., 2009. Reverse breeding: a novel breeding approach based on engineered meiosis. Plant Biotechnol J 7, 837-845.
Dirks, R.H.G., van Dun, C.M.P., Reinink, K., 2003. Reverse Breeding, WO/2003/017753. Rijk Zwaan Zaadteelt en Zaadhandel B.V. Dong, C., Beetham, P., Vincent, K., Sharp, P., 2006. Oligonucleotide-directed gene repair in wheat using a transient plasmid gene repair assay system. Plant Cell Rep 25, 457-465.
Durai, S., Mani, M., Kandavelou, K., Wu, J., Porteus, M.H., Chandrasegaran, S., 2005. Zinc finger nucleases: custom-designed molecular scissors for genome engineering of plant and mammalian cells. Nucleic Acids Res 33, 5978-5990.
Eamens, A., Wang, M.B., Smith, N.A., Waterhouse, P.M., 2008. RNA silencing in plants: Yesterday, today, and tomorrow. Plant Physiology 147, 456-468.
El-Hennawy M, Abdalla A, Shafey SA, Al-Ashkar IM. 2011 Production of doubled haploid wheat lines (triticum aestivum L.) using anther culture technique. Vol 56, issue 2 pg 63-72.
Erickson, F.L., Holzberg, S., Calderon-Urrea, A., Handley, V., Axtell, M., Corr, C., Baker, B., 1999. The helicase domain of the TMV replicase proteins induces the N-mediated defence response in tobacco. Plant Journal 18, 67-75.
Ferraro, G., Becher, M.L., Angel, S.O., Zelada, A., Mentaberry, A.N., Clemente, M., 2008. Efficient expression of a Toxoplasma gondii dense granule Gra4 antigen in tobacco leaves. Experimental Parasitology 120, 118-122.
Fischer, R., Vaquero-Martin, C., Sack, M., Drossard, J., Emans, N., Commandeur, U., 1999. Towards molecular farming in the future: transient protein expression in plants. Biotechnol. Appl. Biochem. 30, 113-116.
Fischer, U., Kuhlmann, M., Pecinka, A., Schmidt, R., Mette, M.F., 2008. Local DNA features affect RNA directed transcriptional gene silencing and DNA methylation. Plant Journal 53, 1-10.
Franca L, Carriho E, Kist T. 2002. A review of DNA sequencing techniques. Quarterly Reviews of Biophysics 35 pg 169-200.
Fu, X., Kohli, A., Twyman, R.M., Christou, P., 2000. Alternative silencing effects involve distinct types of non-spreading cytosine methylation at a three-gene, single-copy transgenic locus in rice. Molecular and General Genetics 263, 106-118.
Fujiki, M., Kaczmarczyk, J.F., Yusibov, V., Rabindran, S., 2008. Development of a new cucumber mosaic virus-based plant expression vector with truncated 3a movement protein. Virology 381, 136-142.
Gal-On, A., Wolf, D., Antignus, Y., Patlis, L., Ryu, K.H., Min, B.E., Pearlsman, M., Lachman, O., Gaba, V., Wang, Y., Shiboleth, Y.M., Yang, J., Zelcer, A., 2005. Transgenic cucumbers harboring the 54-kDa putative gene of Cucumber fruit mottle mosaic tobamovirus are highly resistant to viral infection and protect non-transgenic scions from soil infection. Transgenic Res 14, 81-93.
Gambino, G., Gribaudo, I., Leopold, S., Schartl, A., Laimer, M., 2005. Molecular characterization of grapevine plants transformed with GFLV resistance genes: I. Plant Cell Rep 24, 655-662.
Gamper, H.B., Parekh, H., Rice, M.C., Bruner, M., Youkey, H., Kmiec, E.B., 2000. The DNA strand of chimeric RNA/DNA oligonucleotides can direct gene repair/conversion activity in mammalian and plant cell-free extracts. Nucleic Acids Res 28, 4332-4339.
Garrido-Ramirez, E.R., Sudarshana, M.R., Gilbertson, R.L., 2000. Bean golden yellow mosaic virus from Chiapas, Mexico: Characterization, pseudorecombination with other bean-infecting geminiviruses and germ plasm screening. Phytopathology 90, 1224-1232.
Garvin M, Gharett A. 2007. DEco-TILLING: An inexpensive method for SNP discovery that reduces ascertainment bias. Molecular Ecology Notes 7, 735-746.
Geier, T., Eimert, K., Scherer, R., Nickel, C., 2008. Production and rooting behaviour of rolB-transgenic plants of grape rootstock 'Richter 110' (Vitis berlandieri X V-rupestris). Plant Cell Tissue and Organ Culture 94, 269-280.
Gilchrist E, Haughn G, Ying C, Otto S, Zhuang J, Cheung D, Hamberger B, Aboutorabi F, Kalynyak T, Johnson L, Bohlmann J, Ellis B, Douglass C, Cronk B. 2006. Use of Ecotilling as an efficient SNP discovery tool to survey genetic variation in wild populations of Populus trichocarpa. Mol. Ecol. 15, 1367-1378.
Gomez, E., Zoth, S.C., Asurmendi, S., Rovere, C.V., Berinstein, A., 2009. Expression of Hemagglutinin-Neuraminidase glycoprotein of Newcastle Disease Virus in agroinfiltrated Nicotiana benthamiana plants. J. Biotechnol. 144, 337-340.
Gur and Zamir, Unused natural variation can lift yield barriers in plant breeding, 2004, PLoS Biol.; 2 (10): pg. 245.
Hallauer Arnel, Miranda Filho, Carena, Marcelo. 1981, Quantitative Genetics in Maize Breeding, Iowa State University Press ISBN 978-1-4419-0766-0.
Han, J.S., Park, S., Shigaki, T., Hirschi, K.D., Kim, C.K., 2009. Improved watermelon quality using bottle gourd rootstock expressing a Ca2+/H+ antiporter. Mol. Breed. 24, 201-211.
Haverkort, A., Boonekamp, P., Hutten, R., Jacobsen, E., Lotz, L., Kessel, G., Visser, R., van der Vossen, E., 2008. Societal Costs of Late Blight in Potato and Prospects of Durable Resistance Through Cisgenic Modification. Potato Research 51, 47-57.
Heilersig, B., Loonen, A., Janssen, E.M., Wolters, A.M.A., Visser, R.G.F., 2006. Efficiency of transcriptional gene silencing of GBSSI in potato depends on the promoter region that is used in an inverted repeat. Mol. Genet. Genomics 275, 437-449.
Hemmer, C., Vigne, E., Goldschmidt, V., Komar, V., Marmonier, A., Valat, L., Demangeat, G., Vigneron, S., Masson, J.E., Lemaire, O., 2009. Transgenic rootstocks expressing GFLV coat protein gene in a three years field trial; resistance assessment, impact on GFLV diversity and exchanges between rootstock and scion.
Hohn, B., Puchta, H., 1999. Gene therapy in plants. Proc Natl Acad Sci U S A 96, 8321-8323.
Huang, Z., Chen, Q., Hjelm, B., Arntzen, C., Mason, H., 2009. A DNA Replicon System for Rapid High-Level Production of Virus-Like Particles in Plants. Biotechnology and Bioengineering 103, 706-714.
Huettel, B., Kanno, T., Daxinger, L., Bucher, E., van der Winden, J., Matzke, A.J.M., Matzke, M., 2007. RNAdirected DNA methylation mediated by DRD1 and Pol IVb: A versatile pathway for transcriptional gene silencing in plants. Biochim. Biophys. Acta-Gene Struct. Expression 1769, 358-374.
Hull, A.K., Yusibov, V., Mett, V., 2005. Inducible expression in plants by virus-mediated transgene activation. Transgenic Res. 14, 407-416.
Iida, S., Terada, R., 2005. Modification of endogenous natural genes by gene targeting in rice and other higher plants. Plant Mol Biol 59, 205-219.
Jacobsen, E., Nataraja, K.N., 2008. Cisgenics - Facilitating the second green revolution in India by improved traditional plant breeding. Current Science 94, 1365-1366.
Jacobsen, E., Schouten, H., 2008. Cisgenesis, a New Tool for Traditional Plant Breeding, Should be Exempted from the Regulation on Genetically Modified Organisms in a Step by Step Approach. Potato Research 51, 75-88.
Jacobsen, E., Schouten, H.J., 2007. Cisgenesis strongly improves introgression breeding and induced translocation breeding of plants. Trends Biotechnol 25, 219-223.
Jacobsen, E., Schouten, H.J., 2009. Cisgenesis: an important sub-disclosure for traditional plant breeding companies. Euphytica 170, 235-247.
Jacobsen, E., van der Vossen, E.A.G., Moselio, S., 2009. Plant Disease Resistance: Breeding and Transgenic Approaches. Encyclopedia of Microbiology. Academic Press, Oxford, pp. 597-612.
Jander, G., Barth, C., 2007. Tandem gene arrays: a challenge for functional genomics. Trends in Plant Science 12, 203-210.
Jensen NF. 1988 Plant Breeding Methodology, John Wiley & Sons, Inc. (1988).
Joh, L.D., VanderGheynst, J.S., 2006. Agroinfiltration of plant tissues for production of high-value recombinant proteins: an alternative to production in transgenic crops. Journal of the Science of Food and Agriculture 86, 2002-2004.
Joh, L.D., Wroblewski, T., Ewing, N.N., VanderGheynst, J.S., 2005. High-level transient expression of recombinant protein in lettuce. Biotechnology and Bioengineering 91, 861-871.
Kapoor, M., Baba, A., Kubo, K., Shibuya, K., Matsui, K., Tanaka, Y., Takatsuji, H., 2005. Transgene-triggered, epigenetically regulated ectopic expression of a flower homeotic gene pMADS3 in Petunia. Plant Journal 43, 649-661.
Kim, C.G., Lee, B., Kim, D.I., Park, J.E., Kim, H.J., Park, K.W., Yi, H., Jeong, S.C., Yoon, W.K., Harn, C.H., Kim, H.M., 2008. Detection of gene flow from GM to non-GM watermelon in a field trial. J. Plant Biol. 51, 74-77.
Kmiec, E.B., Johnson, C., May, G.D., 2001. Chloroplast lysates support directed mutagenesis via modified DNA and chimeric RNA/DNA oligonucleotides. Plant Journal 27, 267-274.
Kochevenko, A., Willmitzer, L., 2003. Chimeric RNA/DNA oligonucleotide-based site-specific modification of the tobacco acetolactate syntase gene. Plant Physiol 132, 174-184.
Kok, E.J., Keijer, J., Kleter, G.A., Kuiper, H.A., 2008. Comparative safety assessment of plant-derived foods. Regul Toxicol Pharmacol 50, 98-113.
Kuhl, J.C., Zarka, K., Coombs, J., Kirk, W.W., Douches, D.S., 2007. Late Blight Resistance of RB Transgenic Potato Lines. Journal of the Americal Society for Horticultural Science 132, 783-789.
Kuhn, C., Quick, W.P., Schulz, A., Riesmeier, J.W., Sonnewald, U., Frommer, W.B., 1996. Companion cell specific inhibition of the potato sucrose transporter SUT1. Plant Cell Environ. 19, 1115-1123.
Kumar, S., Allen, G.C., Thompson, W.F., 2006. Gene targeting in plants: fingers on the move. Trends Plant Sci 11, 159-161.
Kumar, S., Fladung, M., 2001. Controlling transgene integration in plants. Trends in Plant Science 6, 155-159.
Kunz, C., Narangajavana, J., Jakowitsch, J., Park, Y.D., Delon, T.R., Kovarik, A., Koukalova, B., van der Winden, J., Moscone, E., Aufsatz, W., Mette, M.F., Matzke, M., Matzke, A.J.M., 2003. Studies on the effects of a flanking repetitive sequence on the expression of single-copy transgenes in Nicotiana sylvestris and in N-sylvestris-N-tomentosiformis hybrids. Plant Mol.Biol. 52, 203-215.
Lambert, C., Tepfer, D., 1991. Use of Agrobacterium rhizogenes to create chimeric apple-trees through genetic grafting. Bio-Technology 9, 80-83.
Lammerts Van Bueren, E.T., Verhoog, H., Tiemens-Hulscher, M., Struik, P.C., Haring, M.A., 2007. Organic griculture requires process rather than product evaluation of novel breeding techniques. NJAS - Wageningen Journal of Life Sciences 54, 401-412.
Lavrov, S.A., Kibanov, M.V., 2007. Noncoding RNAs and chromatin structure. Biochemistry-Moscow 72,1422-1438.
Lee, R.W.H., Strommer, J., Hodgins, D., Shewen, P.E., Niu, Y.Q., Lo, R.Y.C., 2001. Towards development of an edible vaccine against bovine pneumonic pasteurellosis using transgenic white clover expressing a Mannheimia haemolytica A1 leukotoxin 50 fusion protein. Infect. Immun. 69, 5786-5793.
Li, H.Y., Ramalingam, S., Chye, M.L., 2006. Accumulation of recombinant SARS-CoV spike protein in plant cytosol and chloroplasts indicate potential for development of plant-derived oral vaccines. Experimental Biology and Medicine 231, 1346-1352.
Li, J., Hsia, A.P., Schnable, P.S., 2007. Recent advances in plant recombination. Curr Opin Plant Biol 10, 131-135.
Li, X., Wang, X., He, K., Ma, Y., Su, N., He, H., Stole, V., Tongprasit, W., Jin, W., Jiang, J., Terzaghi, W., Li, S., Deng, X.W. 2008 High-Resolution Mapping of Epigenetic Modification of the Rice Genome Uncovers Interplay between DNA methylation, Histone Methylation, and Gene Expression. Plant Cell 2, 259, 276
Liu L, Li Y, Li S, Hu N, He Y, Pong R, Lin D, Lu L, Law M. 2012 Comparison of Next-Generation Sequencing Systems. Journal of Biomedicine and Biotechnology Volume 2012 ID 251364.
Lloyd, A., Plaisier, C.L., Carroll, D., Drews, G.N., 2005. Targeted mutagenesis using zinc-finger nucleases in Arabidopsis. Proc Natl Acad Sci U S A 102, 2232-2237.
Lombardi, R., Circelli, P., Villani, M.E., Buriani, G., Nardi, L., Coppola, V., Bianco, L., Benvenuto, E., Donini, M., Marusic, C., 2009. High-level HIV-1 Nef transient expression in Nicotiana benthamiana using the P19 gene silencing suppressor protein of Artichoke Mottled Crinckle Virus. Bmc Biotechnology 9.
Lough, T.J., Lucas, W.J., 2006. Integrative plant biology: Role of phloem long-distance macromolecular trafficking. Annual Review of Plant Biology 57, 203-232.
Lunerova-Bedrichova, J., Bleys, A., Fojtova, M., Khaitova, L., Depicker, A., Kovarik, A., 2008. Transgeneration inheritance of methylation patterns in a tobacco transgene following a post-transcriptional silencing event. Plant Journal 54, 1049-1062.
Mackenzie, D.J., Tremaine, J.H., McPherson, J., 1991. Genetically engineered resistance to potato virus-S in potato cultivar Russet Burbank. Molecular Plant-Microbe Interactions 4, 95-102.
Maeder, M.L., Thibodeau-Beganny, S., Osiak, A., Wright, D.A., Anthony, RM., Eichtinger, M., Jiang, T., Foley, J.E., Winfrey, R.J., Townsend, J.A., Unger-Wallace, E., Sander, J.D., Muller-Lerch, F., Fu, F.L., Pearlberg, J., Gobel, C., Dassie, J.P., Pruett-Miller, S.M., Porteus, M.H., Sgroi, D.C., Iafrate, A.J., Dobbs, D., McCray, P.B., Cathomen, T., Voytas, D.F., Joung, J.K., 2008. Rapid "Open-Source" engineering of customized zinc-finger nucleases for highly efficient gene modification. Molecular Cell 31, 294-301.
Maki-Valkama, T., Pehu, T., Santala, A., Valkonen, J.P.T., Koivu, K., Lehto, K., Pehu, E., 2000. High level of resistance to potato virus Y by expressing PI sequence in antisense orientation in transgenic potato. Mol. Breed. 6, 95-104.
Maluszynki M 2003. Doubled Haploid Production in Crop Plants edited by Maluszynski ISBN 1-4020-1544-5.
Manavella, P.A., Chan, R.L., 2009. Transient transformation of sunflower leaf discs via an Agrobacterium mediated method: applications for gene expression and silencing studies. Nat Protoc 4, 1699-1707.
Mardis M. 2008. Next-Generation DNA Sequencing Methods. Genomics and Human Genetics vol 9 pg 387-402.
Makarova, K.S., Haft, D.H., Barrangou, R, Brouns, S.J., Charpentier, E., Horvath, P., Moineau, S., Mojica F.J., Wolf, Y.I., Yakunin, A.F., van der Oost, J., Koonin, E.V., 2011 Evolution and classification of the CRISPR-CAS systems, Nat. Rev. Microbiol 6, 467-477.
Martienssen, R.A., Colot, V., 2001. DNA methylation and epigenetic inheritance in plants and filamentous fungi. Science 293, 1070-1074.
Matzke, M., Aufsatz, W., Kanno, T., Daxinger, L., Papp, I., Mette, A.F., Matzke, A.J.M., 2004. Genetic analysis of RNA-mediated transcriptional gene silencing. Biochim. Biophys. Acta-Gene Struct. Expression 1677, 129-141.
Matzke, M., Matzke, A.J.M., Kooter, J.M., 2001. RNA: Guiding gene silencing. Science 293, 1080-1083.
McGurl, B., Orozcocardenas, M., Pearce, G., Ryan, C.A., 1994. Overexpression of the prosystemin gene in transgenic tomato plants generates a systemic signal that constitutively induces proteinase-inhibitor synthesis. Proc. Natl. Acad. Sci. U. S. A. 91, 9799-9802.
Mejlhede N, Kyjovska Z, Backes G, Burhenne K, Rasmussen S, Jahoor A. 2006. EcoTILLING for the identification of allelic variation in the powdery mildew resistance genes mio and Mia of barley. Plant Breeding 125, 461-467.
Meksem and Kahl, The handbook of plant genome mapping: genetic and physical mapping, 2005, Wiley-VCH, ISBN 3527311165, 9783527311163.
Mestre, P., Brigneti, G., Durrant, M.C., Baulcombe, D.C., 2003. Potato virus Y NIa protease activity is not sufficient for elicitation of Ry-mediated disease resistance in potato. Plant Journal 36, 755-761.
Mett, V., Shamloul, A.M., Hirai, H., Zhou, Z.H., Notkins, A., Yusibov, V., 2007. Engineering and expression of the intracellular domain of insulinoma-associated tyrosine phosphatase (IA-2ic), a type 1 diabetes autoantigen, in plants. Transgenic Res. 16, 77-84.
Mette, M.F., Aufsatz, W., van der Winden, J., Matzke, M.A., Matzke, A.J.M., 2000. Transcriptional silencing and promoter methylation triggered by double-stranded RNA. Embo Journal 19, 5194-5201.
Mette, M.F., van der Winden, J., Matzke, M.A., Matzke, A.J.M., 1999. Production of aberrant promoter transcripts contributes to methylation and silencing of unlinked homologous promoters in trans. Embo Journal 18, 241-248.
Meyers, A., Chakauya, E., Shephard, E., Tanzer, F.L., Maclean, J., Lynch, A., Williamson, A.L., Rybicki, E.P., 2008. Expression of HIV-1 antigens in plants as potential subunit vaccines. Bmc Biotechnology 8.
Michelmore et al., 1991, Identification of markers linked to disease-resistance genes by bulked segregant analysis: a rapid method to detect markers in specific genomic regions by using segregating populations. Proceedings of the National Academy of Sciences, USA, 99:9828-9832.
Miki, D., Shimamoto, K., 2008. De novo DNA methylation induced by siRNA targeted to endogenous transcribed sequences is gene-specific and OsMet1-independent in rice. Plant Journal 56, 539-549.
Mitani, N., Kobayashi, S., Nishizawa, Y., Takeshi, K., Matsumoto, R., 2006. Transformation of trifoliate orange with rice chitinase gene and the use of the transformed plant as a rootstock. Sci. Hortic. 108, 439-441.
Moeller, L., Wang, K., 2008. Engineering with precision: Tools for the new generation of transgenic crops. Bioscience 58,391-401.
Molinari, H.B.C., Marur, C.J., Filho, J.C.B., Kobayashi, A.K., Pileggi, M., Júnior, R.P.L., Pereira, L.F.P., Vieira, L.G.E., 2004. Osmotic adjustment in transgenic citrus rootstock Carrizo citrange (Citrus sinensis Osb. x Poncirus trifoliata L. Raf.) overproducing proline. Plant Science 167, 1375-1381.
Muskens, M.W.M., Vissers, A.P.A., Mol, J.N.M., Kooter, J.M., 2000. Role of inverted DNA repeats in transcriptional and post-transcriptional gene silencing. Plant Mol.Biol. 43, 243-260.
Myskja, B.K., 2006. The moral difference between intragenic and transgenic modification of plants. Journal of Agricultural & Environmental Ethics 19, 225-238.
Nagel, A.K., Kalariya, H., Schnabel, G., 2010. The Gastrodia Antifungal Protein (GAFP-1) and Its Transcript Are Absent from Scions of Chimeric-grafted Plum. Hortscience 45, 188-192.
Nielsen, K.M., 2003. Transgenic organisms--time for conceptual diversification? Nat Biotechnol 21, 227-228.
Nieto C, Piron F, Dalmais M, Marco C, Moriones E, Gomez L, Truniger V, Gomez P, Garcia-Mas J, Aranda M, Bendahmane A. 2007. EcoTILLING for the identification of allelic variants of melon eIF4E, a factor that controls virus susceptibility. BMC Plant Biology 7, 34-42.
Oh, T.J., May, G.D., 2001. Oligonucleotide-directed plant gene targeting - Commentary. Curr. Opin. Biotechnol. 12, 169-172.
Okano, Y., Miki, D., Shimamoto, K., 2008. Small interfering RNA (siRNA) targeting of endogenous promoters induces DNA methylation, but not necessarily gene silencing, in rice. Plant Journal 53, 65-77.
Okuzaki, A., Toriyama, K., 2004. Chimeric RNA/DNA oligonucleotide-directed gene targeting in rice. Plant Cell Rep 22, 509-512.
Park, S.M., Lee, J.S., Jegal, S., Jeon, B.Y., Jung, M., Park, Y.S., Han, S.L., Shin, Y.S., Her, N.H., Lee, J.H., Lee, M.Y., Ryu, K.H., Yang, S.G., Ham, C.H., 2005. Transgenic watermelon rootstock resistant to CGMMV (cucumber green mottle mosaic virus) infection. Plant Cell Rep 24, 350-356.
Park, T.H., Foster, S., Brigneti, G., Jones, J.D.G., 2009a. Two distinct potato late blight resistance genes from Solanum berthaultii are located on chromosome 10. Euphytica 165, 269-278.
Park, T.H., Vleeshouwers, V., Jacobsen, E., van der Vossen, E., Visser, R.G.F., 2009b. Molecular breeding for resistance to Phytophthora infestans (Mont.) de Bary in potato (Solanum tuberosum L.): a perspective of cisgenesis. Plant Breeding 128, 109-117.
Paterson AH. 1996 Mapping genes responsible for differences in Genome Mapping in Plants. R.G. Landes, Austin.
Pickford, A.S., Cogoni, C., 2003. RNA-mediated gene silencing. Cellular and Molecular Life Sciences 60, 871-882.
Pierik RLM. 1999 In vitro culture of higher plants, Springer, ISBN 079235267x, 9780792352679.
Plesha, M.A., Huang, T.K., Dandekar, A.M., Falk, B.W., McDonald, K.A., 2009. Optimization of the Bioprocessing Conditions for Scale-Up of Transient Production of a Heterologous Protein in Plants Using a Chemically Inducible Viral Amplicon Expression System. Biotechnology Progress 25, 722-734.
Pogue, G.P., Vojdani, F., Palmer, K.E., Hiatt, E., Hume, S., Phelps, J., Long, L., Bohorova, N., Kim, D., Pauly, M., Velasco, J., Whaley, K., Zeitlin, L., Garger, S.J., White, E., Bai, Y., Haydon, H., Bratcher, B., 2010. Production of pharmaceutical-grade recombinant aprotinin and a monoclonal antibody product using plant-based transient expression systems. Plant Biotechnol J 8, 638-654.
Porteus, M.H., Carroll, D., 2005. Gene targeting using zinc finger nucleases. Nat Biotechnol 23, 967-973.
Puchta, H., 2002. Gene replacement by homologous recombination in plants. Plant Mol Biol 48, 173-182.
Puchta, H., 2003. Towards the ideal GMP: Homologous recombination and marker gene excision. J. PlantPhysiol. 160, 743-754.
Puchta, H., Hohn, B., 2005. Green light for gene targeting in plants. Proc. Natl. Acad. Sci. U.S.A. 102, 11961-11962.
Quarrie S, Lazic Jancic V, Kovacevic D, Steed A, Pekic S. 1999. Bulk segregant analysis with molecular markers and its use for improving drought resistance in maize. Journal of Experimental Botany, 50(337):1299-1306.
Rance, I., Norre, F., Gruber, V., Theisen, M., 2002. Combination of viral promoter sequences to generate highly active promoters for heterologous therapeutic protein overexpression in plants. Plant Science 162, 833-842.
Reiss, B., 2003. Homologous recombination and gene targeting in plant cells. International Review of Cytology - a Survey of Cell Biology, Vol 228. Academic Press Inc, San Diego, pp. 85-139.
Rice, M.C., Czymmek, K., Kmiec, E.B., 2001. The potential of nucleic acid repair in functional genomics. Nature Biotechnology 19, 321-326.
Rice, M.C., May, G.D., Kipp, P.B., Parekh, H., Kmiec, E.B., 2000. Genetic repair of mutations in plant cell free extracts directed by specific chimeric oligonucleotides. Plant Physiology 123,427-437.
Rodriguez, M., Ramirez, N.I., Ayala, M., Freyre, F., Perez, L., Triguero, A., Mateo, C., Selman-Housein, G., Gavilondo, J.V., Pujol, M., 2005. Transient expression in tobacco leaves of an aglycosylated recombinant antibody against the epidermal growth factor receptor. Biotechnology and Bioengineering 89, 188-194.
Rommens, C.M., 2004. All-native DNA transformation: a new approach to plant genetic engineering. Trends Plant Sci 9, 457-464.
Rommens, C.M., 2007. Intragenic crop improvement: combining the benefits of traditional breeding and genetic engineering. J Agric Food Chem 55, 4281-4288.
Rommens, C.M., 2008. The need for professional guidelines in plant breeding. Trends in Plant Science 13, 261-263.
Rommens, C.M., 2010. Barriers and paths to market for genetically engineered crops. Plant Biotechnology Journal 8, 101-111.
Rommens, C.M., Bougri, O., Yan, H., Humara, J.M., Owen, J., Swords, K., Ye, J., 2005. Plant-derived transfer DNAs. Plant Physiol 139, 1338-1349.
Rommens, C.M., Haring, M.A., Swords, K., Davies, H.V., Belknap, W.R., 2007. The intragenic approach as a new extension to traditional plant breeding. Trends Plant Sci 12, 397-403.
Rommens, C.M., Humara, J.M., Ye, J., Yan, H., Richael, C., Zhang, L., Perry, R., Swords, K., 2004. Crop Improvement through Modification of the Plant's Own Genome. Plant Physiology 135, 421-431.
Rommens, C.M., Yan, H., Swords, K., Richael, C., Ye, J.S., 2008. Low-acrylamide French fries and potato chips. Plant Biotechnology Journal 6, 843-853.
Rommens, C.M., Ye, J., Richael, C., Swords, K., 2006. Improving potato storage and processing characteristics through all-native DNA transformation. J Agric Food Chem 54, 9882-9887.
Ruiter, R., Van Den Brande, I., Stals, E., Delaure, S., Cornelissen, M., D'Halluin, K., 2003. Spontaneous mutation frequency in plants obscures the effect of chimeraplasty. Plant Mol.Biol. 53, 715-729.
Saika, H., Toki, S., 2009. Towards a Highly Efficient Gene Targeting System in Higher Plants. Jarq - Jpn. Agric. Res. Q. 43, 81-85.
Sainsbury, F., Lavoie, P.O., D'Aoust, M.A., Vezina, L.P., Lomonossoff, G.P., 2008. Expression of multiple proteins using full-length and deleted versions of cowpea mosaic virus RNA-2. Plant Biotechnology Journal 6, 82-92.
Sainsbury, F., Lomonossoff, G.P., 2008. Extremely high-level and rapid transient protein production in plants without the use of viral replication. Plant Physiol 148, 1212-1218.
Schaart J.G., Visser, R.G.F., 2009. Novel Plant Breeding Techniques - Consequences of new genetic modification-based plant breeding techniques in comparison to conventional plant breeding. COGEM Research Report number 2009-02. The Netherlands Commission on Genetic Modification.
Schouten, H.J., Jacobsen, E., 2007. Are mutations in genetically modified plants dangerous? J. Biomed. Biotechnol.
Schouten, H.J., Jacobsen, E., 2008. Cisgenesis and intragenesis, sisters in innovative plant breeding. Trends Plant Sci 13, 260-261; author reply 261-263.
Schouten, H.J., Krens, F.A., Jacobsen, E., 2006a. Cisgenic plants are similar to traditionally bred plants: international regulations for genetically modified organisms should be altered to exempt cisgenesis. EMBO Rep 7, 750-753.
Schouten, H.J., Krens, F.A., Jacobsen, E., 2006b. Do cisgenic plants warrant less stringent oversight? Nat Biotechnol 24, 753.
Schouten, H.J., Soriano, J.M., Joshi, S.G., Kortstee, A.J., Krens, F.A., Schaart, J.G., van der Linden, K., Allan, A.C., Hellens, R.P., Espley, R.V., Jacobsen, E., 2009. Cisgenesis is a promising approach for fast, acceptable and safe breeding of pip fruit. Acta Hort. 814, 199-204.
Schubert, D., Williams, D., 2006. 'Cisgenic' as a product designation. Nat Biotech 24, 1327-1329.
Seymour D, Filiault D, Henry I, Monson-Miller J, Ravi M, Pang, A, Comai L, Chan S, Maloof J. 2012. Rapid creation of Arabidopsis doubled haploid lines for quantitative trait locus mapping. PNAS vol 109, pg 4227-4232.
Shen, L., Waterland, R. 2007 Methods of DNA methylation analysis, Curr. Opin Clin. Nutr Metab Care 10, 576-581
Shewry, P.R., Jones, H.D., 2005. Transgenic wheat: where do we stand after the first 12 years? Annals of Applied Biology 147, 1-14.
Shiba, H., Takayania, S., 2007. RNA silencing systems and their relevance to allele-specific DNA methylation in plants. Biosci. Biotechnol. Biochem. 71, 2632-2646.
Shibukawa, T., Yazawa, K., Kikuchi, A., Kamada, H., 2009. Possible involvement of DNA methylation on expression regulation of carrot LEC1 gene in its 5 '-upstream region. Gene 437, 22-31.
Shukla, V.K., Doyon, Y., Miller, J.C., DeKelver, R.C., Moehle, E.A., Worden, S.E., Mitchell, J.C., Arnold, N.L., Gopalan, S., Meng, X., Choi, V.M., Rock, J.M., Wu, Y.-Y., Katibah, G.E., Zhifang, G., McCaskill, D., Simpson, M.A., Blakeslee, B., Greenwalt, S.A., Butler, H.J., Hinkley, S.J., Zhang, L., Rebar, E.J., Gregory, P.D., Urnov, F.D., 2009. Precise genome modification in the crop species Zea mays usingmzinc-finger nucleases. Nature 459, 437-441.
Sijen, T., Vijn, I., Rebocho, A., van Blokland, R., Roelofs, D., Mol, J.N., Kooter, J.M., 2001. Transcriptional and posttranscriptional gene silencing are mechanistically related. Curr Biol 11, 436-440.
Silfverberg-Dilworth, E., Besse, S., Paris, R., Belfanti, E., Tartarini, S., Sansavini, S., Patocchi, A., Gessler, C., 2005. Identification of functional apple scab resistance gene promoters. Theoretical and Applied Genetics 110, 1119-1126.
Silva, G., Poirot, L., Galetto, R., Smith, J., Montoya, G., Duchateeau, P. Paques, F., 2011 Meganucleases and Other Tools for Targeted Genome Engineering: Perspectives and Challenges for Gene Therapy. Curr. Gene Ther. 1, 11-27.
Simmonds, N.W. 1979 Principles of Crop Improvement, Longman Group Limited 631.53.
Simon, P., Cannata, F., Concordet, J.-P., Giovannangeli, C., 2008. Targeting DNA with triplex-forming oligonucleotides to modify gene sequence. Biochimie 90, 1109-1116.
Steel, E., Barker, I., Danks, C., Coates, D., Boonham, N., 2010. A. tumefaciens-mediated transient expression as a tool for antigen production for cucurbit yellow stunting disorder virus. Journal of Virological Methods 163, 222-228.
Stegemann, S., Bock, R., 2009. Exchange of genetic material between cells in plant tissue grafts. Science 324, 649-651.
Stewart, C.N., Richards, H.A., Halfhill, M.D., 2000. Transgenic plants and biosafety: Science,misconceptions and public perceptions. Biotechniques 29, 832-+.
Stoddard, B. 2014 Homing endonucleases from mobile group I introns: discovery to genome engineering. Stoddard Mobile DNA 5, 7.
Storici, F., 2008. RNA-mediated DNA modifications and RNA-templated DNA repair. Curr. Opin. Mol. Ther. 10, 224-230.
Strobel G and Daisy B. 2003 Bioprospecting for Microbial Endophytes and Their Natural Products Microbiology and Molecular Biology Reviews 67 (4) pg 491-502.
Sudarshana, M.R., Plesha, M.A., Uratsu, S.L., Falk, B.W., Dandekar, A.M., Huang, T.K., McDonald, K.A., 2006. A chemically inducible cucumber mosaic virus amplicon system for expression of heterologous proteins in plant tissues. Plant Biotechnology Journal 4,551-559.
Szankowski, I., Waidmann, S., Degenhardt, J., Patocchi, A., Paris, R., Silfverberg-Dilworth, E., Broggini, G., Gessler, C., 2009. Highly scab-resistant transgenic apple lines achieved by introgression of HcrVf2 controlled by different native promoter lengths. Tree Genetics & Genomes 5, 349-358.
Tanksley and Nelson, 1996, Advanced backcross QTL analysis: a method for simultaneous discovery and transfer of valuable QTL from un-adapted germplasm into elite breeding lines. Theor Appl Genet 92:191-203.
Tovkach, A., Zeevi, V., Tzfira, T., 2009. A toolbox and procedural notes for characterizing novel zinc finger nucleases for genome editing in plant cells. Plant Journal 57, 747-757.
Townsend, J.A., Wright, D.A., Winfrey, RJ., Fu, F., Maeder, M.L., Joung, J.K., Voytas, D.F., 2009. High frequency modification of plant genes using engineered zinc-finger nucleases. Nature 459, 442-446.
Tranel, P.J., Wright, T.R., 2002. Resistance of weeds to ALS-inhibiting herbicides: what have we learned? Weed Sci. 50, 700-712.
Tripathi, S., Varma, A., 2003. Identification of sources of resistance in Lycopersicon species to Tomato leaf curl geminivirus (ToLCV) by agroinoculation. Euphytica 129, 43-52.
Triques, K., Piednoir, E., Dalmais, M., Schmidt, J., Le Signor, C., Sharkey, M., Caboche, M., Sturbois, B., Bendahmane, A., 2008. Mutation detection using ENDO1: Application to disease diagnostics in humans and TILLING and Eco-TILLING in plants. Bmc Molecular Biology 9.
Tzfira, T., White, C., 2005. Towards targeted mutagenesis and gene replacement in plants. Trends in Biotechnology 23, 567-569.
Urnov, F.D., Miller, J.C., Lee, Y.L., Beausejour, C.M., Rock, J.M., Augustus, S., Jamieson, A.C., Porteus, M.H., Gregory, P.D., Holmes, M.C., 2005. Highly efficient endogenous human gene correction using designed zinc-finger nucleases. Nature 435, 646-651.
Vahdati, K., McKenna, J.R., Dandekar, A.M., Leslie, C.A., Uratsu, S.L., Hackett, W.P., Negri, P., McGranahan, G.H., 2002. Rooting and other characteristics of a transgenic walnut hybrid (Juglans hindsii x J. regia) rootstock expressing rolABC. J. Am. Soc. Hortic. Sci. 127, 724-728.
Val Giddings, L., 2006. 'Cisgenic' as a product designation. Nat Biotech 24, 1329-1329.
Van den Boogaart, T., Maule, A.J., Davies, J.W., Lomonossoff, G.P., 2004. Sources of target specificity associated with the recovery against Pea seed-borne mosaic virus infection mediated by RNA silencing in pea. Mol. Plant Pathol. 5, 37-43.
van der Salm, T.P.M., Bouwer, R., van Dijk, A.J., Keizer, L.C.P., ten Cate, C.H.H., van der Plas, L.H.W., Dons, J.J.M., 1998. Stimulation of scion bud release by rol gene transformed rootstocks of Rosa hybrida L. Journal of Experimental Botany 49, 847-852.
van der Salm, T.P.M., TenCate, C.H.H., Dons, H.J.M., 1996. Prospects for applications of rol genes for crop improvement Plant Mol. Biol. Rep. 14, 207-228.
Vaucheret, H., Fagard, M., 2001. Transcriptional gene silencing in plants: targets, inducers and regulators. Trends in Genetics 17, 29-35.
Verdel, A., Vavasseur, A., Le Gorrec, M., Touat-Todeschini, L., 2009. Common themes in siRNA-mediated epigenetic silencing pathways. Int J Dev Biol 53, 245-257.
Vernooij, B., Friedrich, L., Morse, A., Reist, R., Kolditzjawhar, R., Ward, E., Uknes, S., Kessmann, H., Ryals, J., 1994. Salicylic-acid is not the translocated signal responsible for inducing systemic acquired resistance but is required in signal transduction. Plant Cell 6, 959-965.
Vezina, L.P., Faye, L., Lerouge, P., D'Aoust, M.A., Marquet-Blouin, E., Burel, C., Lavoie, P.O., Bardor, M., Gomord, V., 2009. Transient co-expression for fast and high-yield production of antibodies with human-like N-glycans in plants. Plant Biotechnology Journal 7, 442-455.
Vigne, E., Komar, V., Fuchs, M., 2004. Field safety assessment of recombination in transgenic grapevines expressing the coat protein gene of Grapevine fanleaf virus. Transgenic Res. 13, 165-179.
Vleeshouwers, V., Driesprong, J.D., Kamphuis, L.G., Torto-Alalibo, T., Van't Slot, K.A.E., Govers, F., Visser, R.G.F., Jacobsen, E., Kamoun, S., 2006. Agroinfection-based high-throughput screening reveals specific recognition of INF elicitins in Solanum. Mol. Plant Pathol. 7, 499-510.
Vleeshouwers, V., Rietman, H., Krenek, P., Champouret, N., Young, C., Oh, S.K., Wang, M.Q., Bouwmeester, K., Vosman, B., Visser, R.G.F., Jacobsen, E., Govers, F., Kamoun, S., Van der Vossen, E.A.G., 2008. Effector Genomics Accelerates Discovery and Functional Profiling of Potato Disease Resistance and Phytophthora Infestans Avirulence Genes. Plos One 3.
Vos P, Hogers R, Bleeker M, Reijans M, Van de Lee T, Homes M, Frijters A, Pot J, Peleman J, Kuiper M. 1995, AFLP: a new technique for DNA fingerprinting, Nucleic Acids Res. 1995 November 11; 23(21): 4407-4414.
Wang, M.B., Waterhouse, P.M., 2002. Application of gene silencing in plants. Curr. Opin. Plant Biol. 5, 146-150.
Wassenegger, M., 2000. RNA-directed DNA methylation. Plant Mol.Biol. 43, 203-220.
Welander, M., Pawlicki, N., Holefors, A., Wilson, F., 1998. Genetic transformation of the apple rootstock M26 with the RolB gene and its influence on rooting. J. Plant Physiol. 153, 371-380.
Wijnker, E., de Jong, H., 2008. Managing meiotic recombination in plant breeding. Trends Plant Sci 13,640-646.
Woo, P.C.Y., Lau, S.K.P., Teng, J.L.L, Tse, H., Yuen, K.Y., 2008 Then and now: use of 16s rDNA gene sequencing for bacterial identification and discovery of novel bacteria in clinical microbiology laboratories. Clin Microbiol Infect 14, 908-934.
Wright, D.A., Thibodeau-Beganny, S., Sander, J.D., Winfrey, R.J., Hirsh, A.S., Eichtinger, M., Fu, F., Porteus, M.H., Dobbs, D., Voytas, D.F., Joung, J.K., 2006. Standardized reagents and protocols for engineering zinc finger nucleases by modular assembly. Nat. Protoc. 1, 1637-1652.
Wright, D.A., Townsend, J.A., Winfrey, R.J., Jr., Irwin, P.A., Rajagopal, J., Lonosky, P.M., Hall, B.D., Jondle, M.D., Voytas, D.F., 2005. High-frequency homologous recombination in plants mediated by zinc finger nucleases. Plant J 44, 693-705.
Wu, J., Kandavelou, K., Chandrasegaran, S., 2007. Custom-designed zinc finger nucleases: What is next? Cellular and Molecular Life Sciences 64, 2933-2944.
Xu, J., Wang, Y.Z., Yin, H.X., Liu, X.J., 2009. Efficient Agrobacterium tumefaciens-mediated transformation of Malus zumi (Matsumura) Rehd using leaf explant regeneration system. Electron. J. Biotechnol. 12.
Yeates, C., Gillings, M.R., Davidson, A.D., Altavilla, N., and Veal, D.A., 1998 Methods for microbial DNA extraction from soil for PCR amplification. Biol Preced Online 1,40-47.
Youk, E.S., Pack, I.S., Kim, Y., Yoon, W.K., Kim, C., Ryu, S.B., Harn, C.H., Jeong, S., Kim, H.M., 2009. A framework for molecular genetic assessment of a transgenic watermelon rootstock line. Plant Science 176, 805-811.
Zeevi, V., Tovkach, A., Tzfira, T., 2008. Increasing cloning possibilities using artificial zinc finger nucleases. Proc. Natl. Acad. Sci. U. S. A. 105, 12785-12790.
Zelada, A.M., Calarnante, G., Santangelo, M.D., Bigi, F., Verna, F., Mentaberry, A., Cataldi, A., 2006. Expression of tuberculosis antigen ESAT-6 in Nicotiana tabacum using a potato virus X-based vector. Tuberculosis 86, 263-267.
Zenna, N.S., Cruz, F.C.S., Javier, E.L., Duka, I.A., Barrion, A.A., Azzam, O., 2006. Genetic analysis of tolerance to rice tungro bacilliform virus in rice (Oryza sativa L.) through agroinoculation. Journal of Phytopathology 154, 197-203.
Zhang Z, Qiu F, Liu Y, Ma K, Li Z, Xu S. 2008. Chromosome elimination and in vivo haploid production induced by Stock 6 derived inducer line in maize (Zea mays L.). Plant Cell Rep. Dec 27(12) 1851-60.
Zhu, L., Holefors, A., Ahlman, A., Xue, Z., Welander, M., 2001. Transformation of the apple rootstock M.9/29 with the rolB gene and its influence on rooting and growth. Plant Sci 160, 433-439.
Zhu, L.H., Welander, M., 1999. Growth characteristics of apple cultivar Gravenstein plants grafted onto the transformed rootstock M26 with rolA and rolB genes under nonlimiting nutrient conditions. Plant Science 147, 75-80.
Zhu, T., Mettenburg, K., Peterson, D.J., Tagliani, L., Baszczynski, C.L., 2000. Engineering herbicide-resistant maize using chimeric RNA/DNA oligonucleotides. Nat Biotechnol 18, 555-558.
Zhu, T., Peterson, D.J., Tagliani, L., St Clair, G., Baszczynski, C.L., Bowen, B., 1999. Targeted manipulation of maize genes in vivo using chimeric RNA/DNA oligonucleotides. Proc Natl Acad Sci U S A 96, 8768-8773.
Zinniel DK, Lambrecht N, Harris B, Zhengyu F, Kuczmarski D, Higley P, Ishimaru C. 2002 Isolation and Characterization of Endophytic Colonizing Bacteria from Agronomic Crops and Prairie Plants. Applied and Environmental Microbiology 68 (5): 2198-2208.

The invention further includes the subject matter of the claims of PCT/US2015/032278 from which this application is derived, the content of which is reproduced below as numbered paragraphs (paras).
1. A method for controlling the microbial variability associated with selective plant breeding, comprising:
   a) subjecting one or more plants to a growth medium in the presence of a first set of one or more microorganisms;
   b) selecting one or more plants and/or growth medium following step a);
   c) acquiring a second set of one or more microorganisms from said one or more plants and/or growth medium selected in step b);
   d) repeating steps a) to c) one or more times, wherein the second set of one or more microorganisms acquired in step c) is used as the first set of microorganisms in step a) of any successive repeat;
   e) selecting one or more microorganisms that is associated with imparting a beneficial property to a plant; and
   f) providing the selected one or more microorganisms to a plant undergoing a selective plant breeding program or a growth medium used to grow said plant during the selective plant breeding program.
2. The method of paragraph 1, wherein the selected one or more microorganisms is provided as a seed coating to said plant undergoing a selective plant breeding program.
3. The method of paragraph 1, wherein the selected one or more microorganisms is provided in the form of a granule, plug, liquid drench, topical formulation, or foliar application.
4. The method of paragraph 1, wherein the one or more microorganisms is provided to the growth medium used to grow said plant undergoing a selective plant breeding program and wherein said provided one or more microorganisms account for approximately at least 1%, or at least 10%, or at least 25%, or at least 50%, or at least 75%, or at least 90% of the total microbial diversity present in said growth medium.
5. The method of paragraph 1, wherein the one or more microorganisms is provided to the growth medium used to grow said plant undergoing a selective plant breeding program and wherein said provided one or more microorganisms account for approximately at least 1%, or at least 10%, or at least 25%, or at least 50%, or at least 75%, or at least 90% of the total microbial diversity present in said growth medium and wherein said microbial diversity present in the growth medium is maintained from an F1 generation through each successive selective generation.
6. The method of paragraph 1, wherein the one or more microorganisms is provided to the growth medium used to grow said plant undergoing a selective plant breeding program and wherein said provided one or more microorganisms account for approximately at least 1%, or at least 10%, or at least 25%, or at least 50%, or at least 75%, or at least 90% of the total microbial diversity present in said growth medium and wherein said microbial diversity present in the growth medium is maintained from an F1 generation through each successive selective generation, such that upon reaching at least an F4 generation the microbial diversity in said plant growth medium is at least 90% similar to the microbial diversity found in the growth medium of the F1 generation.
7. The method of paragraph 1, further comprising:
   g) selecting a plant based upon a desired phenotypic or genotypic trait during the course of the selective plant breeding program and simultaneously collecting the microorganisms associated with said plant or plant growth medium.
8. The method of paragraph 1, further comprising:
   g) selecting a plant based upon a desired phenotypic or genotypic trait during the course of the selective plant breeding program and simultaneously collecting the microorganisms associated with said plant or plant growth medium; and
   h) providing the microorganisms collected from step g) to a plant or plant growth medium utilized in the next subsequent generation of the selective plant breeding program.
9. The method of paragraph 1, wherein a selective pressure is applied in step a).
10. The method of paragraph 1, wherein a selective pressure is applied in step a) and wherein the selective pressure is biotic and includes exposing the one or more plants to an organism selected from the group consisting of: fungi, bacteria, viruses, insects, mites, nematodes, and combinations thereof.
11. The method of paragraph 1, wherein a selective pressure is applied in step a) and wherein the selective pressure is abiotic and includes exposing the one or more plants to an abiotic pressure selected from the group consisting of: salt concentration, temperature, pH, water, minerals, organic nutrients, inorganic nutrients, organic toxins, inorganic toxins, metals, and combinations thereof.
12. The method of paragraph 1, wherein the selective plant breeding program is conducted in a soil-free or hydroponic system.
13. A method for conducting holobiome plant breeding, comprising:
   a) subjecting one or more plants to a growth medium in the presence of a first set of one or more microorganisms;
   b) selecting one or more plants and/or growth medium following step a);
   c) acquiring a second set of one or more microorganisms from said one or more plants and/or growth medium selected in step b);
   d) repeating steps a) to c) one or more times, wherein the second set of one or more microorganisms acquired in step c) is used as the first set of microorganisms in step a) of any successive repeat;
   e) selecting one or more microorganisms that is associated with imparting a beneficial property to a plant;
   f) providing the selected one or more microorganisms to a plant undergoing a selective plant breeding program or a growth medium used to grow said plant during the selective plant breeding program;
   g) selecting a plant based upon a desired phenotypic or genotypic trait during the course of the selective plant breeding program and simultaneously collecting the microorganisms associated with said plant or plant growth medium; and
   h) providing the microorganisms collected from step g) to a plant or plant growth medium utilized in the next subsequent generation of the selective plant breeding program.
14. A method for conducting holobiome plant breeding, comprising:
   a) crossing two plant cultivars to produce F1 hybrid plants;
   b) selfing the F1 hybrid plants to produce F2 seed;
   c) planting the F2 seed in soil collected from a region exhibiting a desired environmental property,
      wherein said desired environmental property represents an environmental property for which the successive cohort plants of the selective plant breeding process are selected to tolerate;
   d) growing the F2 seed under environmental conditions that approximate the desired environmental property;
   e) selecting F2 plants that exhibit the best phenotypic response to said environmental property and allowing said selected F2 plants to reach maturity and set F3 seed;
   f) harvesting F3 seed from the selected F2 plants and simultaneously harvesting a microbial community associated with the F2 plants and/or the soil utilized to grow said F2 plants;
   g) planting the F3 seed in soil from step c) that has been inoculated with the microbial community collected in step f); and
   h) repeating steps d) to g) one or more times.
15. The method of paragraph 14, wherein the soil utilized in step g), and any successive repeats of the plant selection process, is autoclaved before being inoculated with the microbial community collected in the preceding step.
16. The method of paragraph 15, wherein the desired environmental property is selected from the group consisting of: cold temperature, high temperature, high humidity, drought, salinity, low nitrogen, low phosphorous, low photosynthetically active radiation, high elemental metal concentrations, high soil acidity, and combinations thereof.
17. The method of paragraph 15, wherein the soil from step c) is inoculated by applying to said soil a granule, plug, or liquid drench, comprising the harvested microbial community.
18. The method of paragraph 15, wherein the plant selection process is repeated through the production of F5 seed.
19. The method of paragraph 15, further comprising: maintaining parental lines as controls through each successive plant selection cycle and said parental lines are grown in the soil from step c), but said soil is not inoculated with a harvested microbial community during successive plant selection cycles.
20. The method of paragraph 15, further comprising:
   maintaining parental lines as controls through each successive plant selection cycle and said parental lines are grown in the soil from step c), but said soil is not inoculated with a harvested microbial community during successive plant selection cycles; and
   wherein the plant selection process is repeated through the production of F5 seed; and
   wherein the selected F4 plants that produced the F5 seed demonstrate an increased desired phenotypic response to said environmental property, as compared to the parental line plants.
21. The method of paragraph 15, further comprising:
   h) repeating steps d) to g) through the production of F5 seed;
   i) planting the harvested F5 seed, and the microbial community harvested in association with the F4 plants and/or the soil utilized to grow said F4 plants that produced the F5 seed, in a replicated field trial; and
   j) selecting the best performing F5 plants.

## Claims

1. A method of producing an improved hybrid plant, the method comprising:
(a) placing a first set of one or more plants in a growth medium in the presence of a first set of microorganisms;
(b) growing the first set of one or more plants and selecting a second set of one or more plants from the first set, based on a desired characteristic;
(c) acquiring a second set of microorganisms from the second set of plants;
(d) repeating steps (a) through (c) at least once, wherein the set of microorganisms obtained in step (c) is used as the set of microorganisms in a successive iteration of (a);
(e) acquiring a final set of microorganisms from the last repeat, to create a curated consortium;
(f) sexually crossing a new plant with another plant in the presence of the curated consortium;
(g) select at least one hybrid offspring from the cross performed in (f);
(h) obtaining a set of one or more microorganisms from the hybrid offspring of (g);
(i) sexually crossing the hybrid offspring of (f) with another plant in the presence of the set of one or more microorganisms of (h), to produce an improved hybrid plant.

2. The method of claim 1, wherein the growth medium is soil.

3. The method of claim 1, wherein the growth medium is hydroponic.

4. The method of claim 1, wherein the set of one or more microorganisms of step (i) is present as a seed treatment.

5. The method of claim 1, wherein the set of one or more microorganisms of step (i) is provided in the form of a granule, plug, liquid drench, topical formulation, or foliar application.

6. The method of claim 1, wherein the set of one or more microorganisms of step (i) is provided to the growth medium used to grow said plant undergoing a selective plant breeding program and wherein said provided one or more microorganisms account for approximately at least 1%, or at least 10%, or at least 25%, or at least 50%, or at least 75%, or at least 90% of the total microbial diversity present in said growth medium.

7. The method of claim 1, wherein the set of one or more microorganisms of step (i) is provided to the growth medium used to grow said plant undergoing a selective plant breeding program and wherein said provided one or more microorganisms account for approximately at least 1%, or at least 10%, or at least 25%, or at least 50%, or at least 75%, or at least 90% of the total microbial diversity present in said growth medium and wherein said microbial diversity present in the growth medium is maintained from an F1 generation through each successive selective generation.

8. The method of claim 1, wherein set of one or more microorganisms of step (i) is provided to the growth medium used to grow said plant undergoing a selective plant breeding program and wherein said provided one or more microorganisms account for approximately at least 1%, or at least 10%, or at least 25%, or at least 50%, or at least 75%, or at least 90% of the total microbial diversity present in said growth medium and wherein said microbial diversity present in the growth medium is maintained from an F1 generation through each successive selective generation, such that upon reaching at least an F4 generation the microbial diversity in said plant growth medium is at least 90% similar to the microbial diversity found in the growth medium of the F1 generation.

9. The method of claim 1, wherein a selective pressure is applied in step (b).

10. The method of claim 9, wherein a selective pressure is biotic and includes exposing the one or more plants to an organism selected from the group consisting of: fungi, bacteria, viruses, insects, mites, nematodes, and combinations thereof.

11. The method of claim 9, wherein the selective pressure is abiotic and includes exposing the one or more plants to an abiotic pressure selected from the group consisting of: salt concentration, temperature, pH, water, minerals, organic nutrients, inorganic nutrients, organic toxins, inorganic toxins, metals, and combinations thereof.

12. A plant obtained by the method of any one of claims 1 to 11.

13. A plant population obtained by the method of any one of claims 1 to 11.
